# DEMANDE DE BREVET EUROPEEN

(11) **EP 4 563 595 A1**
(43) Date de publication de la demande: **04.06.2025**
(21) Numéro de dépôt: 23307075.4
(22) Date de dépôt: 28.11.2023
(51) Int. Cl.: C07K 16/26, A61P 5/06

(54) **ANTICORPS VARIANTS HUMANISES OU FRAGMENTS DE CEUX-CI POTENTIALISANT LA BIOACTIVITE DES GONADOTROPINES**

(71) Demandeur: IGYXOS, 37380 Nouzilly (FR)
(72) Inventeur: MAUREL, Marie-Christine, 37000 Tours (FR); KARA, Elodie, 37250 Veigne (FR); CASTERET, Sophie, 37190 Valleres (FR); DUPUY, Laurence, 37110 Villedomer (FR); DECOURTYE, Jérémy, 37000 Tours (FR)
(74) Mandataire: Novagraaf Technologies

(57) **Abrégé**

La présente invention concerne de nouveaux anticorps variants humanisés ou fragments de ceux-ci capables de potentialiser la bioactivité des gonadotropines.

## Description

### Domaine technique

La présente invention concerne de nouveaux anticorps variants humanisés ou fragments de ceux-ci, dérivés de l'anticorps monoclonal murin CF12 dirigé contre l'hormone folliculo-stimulante (anti-FSH) humaine, et capables de potentialiser la bioactivité des gonadotropines.

La présente invention trouve ses applications principalement en médecine de la reproduction, notamment pour induire l'ovulation ou la polyovulation chez un mammifère femelle, restaurer et/ou stimuler la spermatogénèse chez un mammifère mâle, ou encore pour augmenter la stéroïdogenèse chez un mammifère mâle ou femelle.

Dans la description ci-dessous, les références entre crochets ([ ]) renvoient à la liste des références présentées à la fin du texte.

### Etat de la technique

Les gonadotrophines (ou gonadotropines) sont des hormones glycoprotéiques complexes jouant un rôle central dans la régulation de la reproduction chez les vertébrés en agissant sur les fonctions des gonades (ovaires et testicules). Deux de ces hormones sont sécrétées chez tous les vertébrés : l'hormone lutéinisante (LH) et l'hormone folliculo-stimulante (FSH). Chez deux groupes de mammifères, équidés et primates, il existe en outre une gonadotrophine chorionique (CG) sécrétée par le placenta : la choriogonadotropine humaine (hCG) et la choriogonadotropine équine (eCG) qui agissent toutes deux via des récepteurs LH.

L'hormone lutéinisante (LH) est produite par les cellules gonadotropes du lobe antérieur de l'hypophyse sous stimulation de la GnRH (*Gonadotropin Releasing Hormone*), elle-même produite par l'hypothalamus. La LH stimule la production de testostérone chez les mâles, tandis qu'elle intervient dans les modifications du cycle ovarien chez les femelles où elle est responsable de la croissance folliculaire terminale et de l'ovulation puis de la transformation du follicule ovulatoire rompu en corps jaune. Pendant la phase lutéale du cycle menstruel, la LH stimule la sécrétion de progestérone par le corps jaune, indispensable au développement précoce et à l'implantation de l'embryon.

L'hormone folliculo-stimulante (ou FSH) est produite par les cellules gonadotropes du lobe antérieur de l'hypophyse sous stimulation de la GnRH produite par l'hypothalamus. Chez les mâles, elle stimule les cellules de Sertoli, cellules nourricières indispensables à la spermatogénèse. Chez les femelles, elle est responsable du recrutement des follicules primordiaux, immatures, de leur croissance et de leur différentiation en follicules pré-ovulatoires en stimulant les récepteurs FSH des cellules de la granulosa.

La FSH comme la LH est constituée d'une sous-unité α commune à toutes les hormones glycoprotéiques d'une même espèce (LH, FSH, la Chorio Gonadotropine (CG) et l'hormone thyréostimulante (TSH)) et d'une sous-unité β spécifique responsable de la spécificité d'activité de l'hormone ; activité qui n'existe que si les deux sous-unités sont associées de manière non covalente sous-forme d'un dimère. Seule la forme dimérique est capable d'activer le récepteur dont elle est spécifique et de stimuler les cellules cibles exprimant ce récepteur. La FSH a une structure semblable à celle de la LH. Chez les femelles, les taux de LH et de FSH sont cycliques : très faibles en dehors de la période ovulatoire, avec un pic de sécrétion en période préovulatoire.

Les gonadotrophines sont utilisées en médecine vétérinaire et humaine, pour induire l'ovulation chez les mammifères femelles. Dans le domaine vétérinaire, bien qu'efficaces, ces traitements présentent un risque sanitaire du fait de l'utilisation d'hormones extraites à partir de fluides biologiques (sang, urine) ou de tissus (hypophyses). C'est le cas de la gonadotropine chorionique équine (eCG) extraite à partir de sang de juments gravides, et de la LH et de la FSH porcines extraites à partir d'hypophyses de porc. Dans le domaine vétérinaire, on utilise également une hCG extraite à partir d'urine de femmes enceintes, le Chorulon^{®} (Laboratoire MSD).

Dans le domaine de la clinique humaine, et particulièrement de la Procréation Médicalement Assistée (ou PMA), on utilise des hormones extraites à partir d'urine de femmes ménopausées telles que Fostimon^{®} (Laboratoire Genévrier) qui est une FSH purifiée et Menopur^{®} (Laboratoire Ferring Pharmaceuticals) qui est une hMG (human menoposal gonadotropin), mélange de FSH et de LH. On utilise également des FSH humaines recombinantes, telles que Gonal-FO (Laboratoire Merck Serono), Puregon^{®} (Laboratoire Merck Schering-Plough), Rekovelle^{®} (Laboratoire Ferring Pharmaceuticals) et des FSH biosimilaires recombinantes, Ovaleap^{®} (Teva) et Bemfola^{®} (Gedeon Richter); des hCG et LH recombinantes telles que Ovitrelle^{®} et Luveris^{®} (Laboratoire Merck Serono). Cependant l'usage répété de ces hormones peut induire une réaction immunitaire qui peut neutraliser l'effet des hormones conduisant ainsi à une baisse d'efficacité thérapeutique lors des traitements suivants.

En revanche, il a également été mis en évidence chez les animaux de rente traités avec la eCG appelée également PMSG (*Prégnant Mare Serum Gonadotropin*) que la réaction immunitaire pouvait, dans certains cas, produire des anticorps polyclonaux anti-eCG capables de potentialiser les bioactivités FSH et LH de la eCG injectée lors des traitements [1]. Des travaux ont montré également que la fraction purifiée de ces anticorps, co-administrée avec de la eCG, *in vivo* chez le rat femelle immature, entraînait une stimulation significativement plus importante des ovaires [2]. Depuis, il a également été mis en évidence trois anticorps monoclonaux anti-LH capables de potentialiser son action ainsi que celle de la FSH pour deux d'entre eux [3], et, plus récemment, l'anticorps monoclonal anti-FSH murin CF12 capable de potentialiser la bioactivité de la FSH, de la LH et de la CG [4].

### Description de l'invention

Les inventeurs ont maintenant produit des variants humanisés dérivés de l'anticorps monoclonal anti-FSH murin CF12 [4] dans lesquels (i) les séquences des CDRs (*Complementary Determining Region*) (régions hypervariables des régions variables des chaînes lourdes et légères d'un anticorps qui constituent les éléments du paratope et permettent de déterminer la complémentarité de l'anticorps avec l'épitope de l'antigène) sont identiques à celles de l'anticorps monoclonal CF12, et (ii) les séquences des Framework (FRs) (régions encadrant les CDRs constituant la « charpente » et donnant une configuration stable au domaine variable) de l'anticorps monoclonal CF12 dont au moins un FR porte au moins une mutation, sont insérées dans une charpente d'immunoglobuline humaine (*e*.*g*. région Fc de la chaîne d'immunoglobuline gamma-4 (IgG4)). Ces anticorps variants humanisés présentent de manière inattendue la capacité de potentialiser *in vitro* et *in vivo* la bioactivité de la FSH avec un effet égal ou supérieur à celui de l'anticorps monoclonal anti-FSH murin CF12. Ils peuvent également potentialiser *in vivo* la bioactivité de la LH et de la hCG avec un effet inférieur à celui de l'anticorps monoclonal anti-FSH murin CF12 ; ce qui présente l'avantage supplémentaire et nouveau de privilégier et mieux cibler la potentialisation de la FSH dans une indication thérapeutique.

Les séquences en acides aminés des régions variables des chaînes lourdes et légères des variants humanisés de l'anticorps monoclonal anti-FSH murin CF12 ont été synthétisées. Elles sont présentées dans le tableau ci-dessous.

**Tableau 1**

| **Variants humanisés et modifiés de l'anticorps monoclonal anti-FSH murin CF12** | |
|---|---|
| **Domaine variable de la chaine lourde (VH)** | |
| CF12-VH13 consensus (SEQ ID NO : 1) | QX²QLVQSGAEVKKPGASVKVSCKX²⁴SGFTF SSSYIX³⁵WX³⁷RQAPGQRLEWX⁴⁸X⁴⁹WIYAGT GGTSYX⁶¹QKFX⁶⁵GX⁶⁷X⁶⁸X⁶⁹X⁷⁰TX⁷²DTSASTA YMEX⁸³SSLRSEDTAVYYCARHGSYFDYWGQ GTLVTVSS où X² est V ou G, X²⁴ est A ou T, X³⁵ est H ou S, X³⁷ est V ou L, X⁴⁸ est I ou M, X⁴⁹ est A ou G, X⁶¹ est S ou N, X⁶⁵ est T ou Q, X⁶⁷ est R ou K, X⁶⁸ est A ou V, X⁶⁹ est T ou Q, X⁷⁰ est I ou L, X⁷² est V ou R, X⁸³ est L ou F |
| CF12-VH366 consensus (SEQ ID NO : 2) | EVQLVESGGGLVQPGGSLRLSCAX²⁴SGFTFS SSYISWLRQAPGKGLEWX⁴⁸AWIYAGTGGTSY AQX⁶³VKGRFX⁶⁹X⁷⁰SVDTSKNTAYLQMNSLRA EDTAVYYCARHGSYFDYWGQGTLVTVSS où X²⁴ est A ou T, X⁴⁸ est I ou V, X⁶³ est S ou K, X⁶⁹ est Q ou T, X⁷⁰ est L ou I |
| CF12-VH158 consensus (SEQ ID NO : 3) | QMQLVQSGPEVKKPGTSVKVSCKX24SGFTFS SSYISWLRQARGQRLEWIAWIYAGTGGTSYA QKFQERVX69X70TVDMSTSTAYMEFSSLRSED TAVYYCARHGSYFDYWGQGTLVTVSS où X²⁴ est T ou A, X⁶⁹ est Q ou T, X⁷⁰ est L ou I |
| CF12-13-VH0 (SEQ ID NO : 5) | QVQLVQSGAEVKKPGASVKVSCKASGFTFSS SYIHWVRQAPGQRLEWMGWIYAGTGGTSYS QKFQGRVTITRDTSASTAYMELSSLRSEDTAV YYCARHGSYFDYWGQGTLVTVSS |
| CF12-13-VH1 (SEQ ID NO : 6) | QVQLVQSGAEVKKPGASVKVSCKASGFTFSS SYISWVRQAPGQRLEWMAWIYAGTGGTSYN QKFTGKVTITRDTSASTAYMELSSLRSEDTAV YYCARHGSYFDYWGQGTLVTVSS |
| CF12-13-VH2 (SEQ ID NO : 7) | QGQLVQSGAEVKKPGASVKVSCKASGFTFSS SYISWLRQAPGQRLEWIAWIYAGTGGTSYNQ KFTGKVTITRDTSASTAYMELSSLRSEDTAVY YCARHGSYFDYWGQGTLVTVSS |
| CF12-13-VH3 (SEQ ID NO : 8) | QGQLVQSGAEVKKPGASVKVSCKASGFTFSS SYISWLRQAPGQRLEWIAWIYAGTGGTSYNQ KFTGKATLTVDTSASTAYMELSSLRSEDTAVY YCARHGSYFDYWGQGTLVTVSS |
| CF12-13-VHB (SEQ ID NO : 9) | QVQLVQSGAEVKKPGASVKVSCKTSGFTFSS SYISWLRQAPGQRLEWIAWIYAGTGGTSYSQ |
| | KFQGRVQLTVDTSASTAYMEFSSLRSEDTAV YYCARHGSYFDYWGQGTLVTVSS |
| CF12-13-VHC (SEQ ID NO : 10) | QVQLVQSGAEVKKPGASVKVSCKASGFTFSS SYISWLRQAPGQRLEWMAWIYAGTGGTSYS QKFQGRVTITVDTSASTAYMEFSSLRSEDTAV YYCARHGSYFDYWGQGTLVTVSS |
| CF12-366-VHB (SEQ ID NO : 11) | EVQLVESGGGLVQPGGSLRLSCATSGFTFSS SYISWLRQAPGKGLEWIAWIYAGTGGTSYAQ KVKGRFQLSVDTSKNTAYLQMNSLRAEDTAV YYCARHGSYFDYWGQGTLVTVSS |
| CF12-366-VHC (SEQ ID NO : 12) | EVQLVESGGGLVQPGGSLRLSCAASGFTFSS SYISWLRQAPGKGLEWVAWIYAGTGGTSYAQ SVKGRFTISVDTSKNTAYLQMNSLRAEDTAVY YCARHGSYFDYWGQGTLVTVSS |
| CF12-158-VHB (SEQ ID NO : 13) | QMQLVQSGPEVKKPGTSVKVSCKTSGFTFSS SYISWLRQARGQRLEWIAWIYAGTGGTSYAQ KFQERVQLTVDMSTSTAYMEFSSLRSEDTAV YYCARHGSYFDYWGQGTLVTVSS |
| CF12-158-VHC (SEQ ID NO : 14) | QMQLVQSGPEVKKPGTSVKVSCKASGFTFSS SYISWLRQARGQRLEWIAWIYAGTGGTSYAQ KFQERVTITVDMSTSTAYMEFSSLRSEDTAVY YCARHGSYFDYWGQGTLVTVSS |

| Domaine variable de la chaine légère (VL) | |
|---|---|
| CF12-VL0/VL139 consensus (SEQ ID NO : 4) | DIX3X4TQSPX9SLX12X13SX15GX17RX19TIX22CX2 4X25SQSVDYDGDSYMX38WYQQKPGX46X47PK LLIYAASX57X58ESGVPX64RFSGSGSGTDFTLT ISSLQX84EDX87AX89YYCQQSNEDPYTFGQGT KLEIK où X³ est Q ou V, X⁴ est M ou L, X⁹ est D ou S, X¹² est A ou S, X¹³ est A ou V, X¹⁵ est L ou V, X¹⁷ est E ou D, X¹⁹ est A ou V, X²² est N ou T, X²⁴ est R ou K, X²⁵ est A ou S, X³⁸ est A ou N, X⁴⁶ est Q ou K, X⁴⁷ est P ou A, X⁵⁷ est N ou S, X⁵⁸ est R ou L, X⁶⁴ est D ou S, X⁸⁴ est A ou P, X⁸⁷ est V ou F, X⁸⁹ est V ou T |
| CF12-VL0 (SEQ ID NO : 15) | DIVMTQSPDSLAVSLGERATINCKSSQSVDYD GDSYMAWYQQKPGQPPKLLIYAASNRESGV PDRFSGSGSGTDFTLTISSLQAEDVAVYYCQ QSNEDPYTFGQGTKLEIK |
| CF12-VL139 (SEQ ID NO : 16) | DIQLTQSPSSLSASVGDRVTITCRASQSVDYD GDSYMNWYQQKPGKAPKLLIYAASSLESGVP |
| | SRFSGSGSGTDFTLTISSLQPEDFATYYCQQS NEDPYTFGQGTKLEIK |

Dans le tableau 1, les séquences correspondant aux CDRs sont soulignées.

La présente invention a pour objet de nouveaux anticorps variants humanisés issus d'un anticorps monoclonal murin anti-hormone folliculo-stimulante humaine (anti-FSH) qui potentialise la bioactivité de l'hormone folliculo-stimulante (FSH), de l'hormone lutéinisante (LH) et de l'hormone chorionique gonadotrope humaine (hCG) (e.g. l'anticorps anti-FSH murin CF12 de séquence VH « QGQMQQSGAELVKPGASVKLSCKTSGFTFSSSYISWLKQKPGQSLEWIAWIYAG TGGTSYNQKFTGKAQLTVDTSSSTAYMQFSSLTTEDSAIYYCARHGSYFDYWGQG TTLTVSS » (SEQ ID NO : 18) et de séquence VL « DIVLTQSPASLAVSLGQRATISCKASQSVDYDGDSYMNWYQQKPGQPPKLLIYAA SNLESGIPARFSGSGSGTDFTLNIHPVEEEDAATYYCQQSNEDPYTFGGGTKLEIK » (SEQ ID NO : 19)) ou fragments de ceux-ci, tous liant la FSH et potentialisant la bioactivité de la FSH, de la LH et de l'hCG, et caractérisés en ce que :
le domaine variable de la chaine lourde dudit anticorps variant humanisé ou fragment de celui-ci contient la séquence en acides animés QX²QLVQSGAEVKKPGASVKVSCKX²⁴SGFTFSSSYIX³⁵WX³⁷RQAPGQRLEWX⁴⁸X⁴⁹ WIYAGTGGTSYX⁶¹QKFX⁶⁵GX⁶⁷X⁶⁸X⁶⁹X⁷⁰TX⁷²DTSASTAYMEX⁸³SSLRSEDTAVYYC ARHGSYFDYWGQGTLVTVSS où X² est V ou G, X²⁴ est A ou T, X³⁵ est H ou S, X³⁷ est V ou L, X⁴⁸ est I ou M, X⁴⁹ est A ou G, X⁶¹ est S ou N, X⁶⁵ est T ou Q, X⁶⁷ est R ou K, X⁶⁸ est A ou V, X⁶⁹ est T ou Q, X⁷⁰ est I ou L, X⁷² est V ou R, X⁸³ est L ou F (SEQ ID NO : 1), ou la séquence en acides aminés EVQLVESGGGLVQPGGSLRLSCAX²⁴SGFTFSSSYISWLRQAPGKGLEWX⁴⁸AWIYA GTGGTSYAQX⁶³VKGRFX⁶⁹X⁷⁰SVDTSKNTAYLQMNSLRAEDTAVYYCARHGSYFDY WGQGTLVTVSS où X²⁴ est A ou T, X⁴⁸ est I ou V, X⁶³ est S ou K, X⁶⁹ est Q ou T, X⁷⁰ est L ou I (SEQ ID NO : 2), ou la séquence en acides aminés QMQLVQSGPEVKKPGTSVKVSCKX²⁴SGFTFSSSYISWLRQARGQRLEWIAWIYAG TGGTSYAQKFQERVX⁶⁹X⁷⁰TVDMSTSTAYMEFSSLRSEDTAVYYCARHGSYFDYW GQGTLVTVSS où X²⁴ est T ou A, X⁶⁹ est Q ou T, X⁷⁰ est L ou I (SEQ ID NO : 3) ;
   et
le domaine variable de la chaine légère dudit anticorps variant humanisé ou fragment de celui-ci contient la séquence en acides aminés DIX³X⁴TQSPX⁹SLX¹²X¹³SX¹⁵GX¹⁷RX¹⁹TIX²²CX²⁴X²⁵SQSVDYDGDSYMX³⁸WYQQKP GX⁴⁶X⁴⁷PKLLIYAASX⁵⁷X⁵⁸ESGVPX⁶⁴RFSGSGSGTDFTLTISSLQX⁸⁴EDX⁸⁷AX⁸⁹YYC QQSNEDPYTFGQGTKLEIK où X³ est Q ou V, X⁴ est M ou L, X⁹ est D ou S X¹² est A ou S, X¹³ est A ou V, X¹⁵ est L ou V, X¹⁷ est E ou D, X¹⁹ est A ou V, X²² est N ou T, X²⁴ est R ou K, X²⁵ est A ou S, X³⁸ est A ou N, X⁴⁶ est Q ou K, X⁴⁷ est P ou A, X⁵⁷ est N ou S, X⁵⁸ est R ou L, X⁶⁴ est D ou S, X⁸⁴ est A ou P, X⁸⁷ est V ou F, X⁸⁹ est V ou T (SEQ ID NO : 4).

On entend par « anticorps variant humanisé » au sens de la présente invention, un anticorps résultant de l'insertion des régions hypervariables (CDRs) parentales de l'anticorps monoclonal anti-FSH murin CF12 dans une charpente d'immunoglobuline humaine, et dont au moins une région framework (FR) parentale a été mutée.

On entend par « fragment de celui-ci » au sens de la présente invention, par exemple, un fragment d'un anticorps variant humanisé selon l'invention : Fab, Fab', F(ab')2, Fv, dsFv, scFv, ou scFv-Fc avec un Fc humain, nanocorps. De préférence, il s'agit d'un fragment scFv (fragment variable simple chaîne pour «*single chain Fragment variable*»). Par exemple, un fragment scFv selon la présente invention a la séquence SEQ ID NO : 17.

En particulier, un anticorps variant humanisé ou fragment de celui-ci selon la présente invention est caractérisé en ce que :
le domaine variable de la chaine lourde dudit anticorps variant humanisé ou fragment de celui-ci contient la séquence en acides aminés QVQLVQSGAEVKKPGASVKVSCKASGFTFSSSYIHWVRQAPGQRLEWMGWIYAG TGGTSYSQKFQGRVTITRDTSASTAYMELSSLRSEDTAVYYCARHGSYFDYWGQG TLVTVSS (SEQ ID NO: 5), ou la séquence en acides aminés QVQLVQSGAEVKKPGASVKVSCKASGFTFSSSYIS\NVRQAPGQRLEVVMAWIYAGT GGTSYNQKFTGKVTITRDTSASTAYMELSSLRSEDTAVYYCARHGSYFDYWGQGT LVTVSS (SEQ ID NO : 6), ou la séquence en acides aminés QGQLVQSGAEVKKPGASVKVSCKASGFTFSSSYISWLRQAPGQRLEWIAWIYAGT GGTSYNQKFTGKVTITRDTSASTAYMELSSLRSEDTAVYYCARHGSYFDYWGQGT LVTVSS (SEQ ID NO : 7), ou la séquence en acides aminés QGQLVQSGAEVKKPGASVKVSCKASGFTFSSSYISWLRQAPGQRLEWIAWIYAGT GGTSYNQKFTGKATLTVDTSASTAYMELSSLRSEDTAVYYCARHGSYFDYWGQGT LVTVSS (SEQ ID NO : 8), ou la séquence en acides aminés QVQLVQSGAEVKKPGASVKVSCKTSGFTFSSSYISWLRQAPGQRLEWIAWIYAGTG GTSYSQKFQGRVQLTVDTSASTAYMEFSSLRSEDTAVYYCARHGSYFDYWGQGTL VTVSS (SEQ ID NO : 9), ou la séquence en acides aminés QVQLVQSGAEVKKPGASVKVSCKASGFTFSSSYISWLRQAPGQRLEWMAWIYAGT GGTSYSQKFQGRVTITVDTSASTAYMEFSSLRSEDTAVYYCARHGSYFDYWGQGT LVTVSS (SEQ ID NO: 10), ou la séquence en acides aminés EVQLVESGGGLVQPGGSLRLSCATSGFTFSSSYISWLRQAPGKGLEWIAWIYAGTG GTSYAQKVKGRFQLSVDTSKNTAYLQMNSLRAEDTAVYYCARHGSYFDYWGQGT LVTVSS (SEQ ID NO: 11), ou la séquence en acides aminés EVQLVESGGGLVQPGGSLRLSCAASGFTFSSSYISWLRQAPGKGLEWVAWIYAGT GGTSYAQSVKGRFTISVDTSKNTAYLQMNSLRAEDTAVYYCARHGSYFDYWGQGT LVTVSS (SEQ ID NO: 12), ou la séquence en acides aminés QMQLVQSGPEVKKPGTSVKVSCKTSGFTFSSSYISWLRQARGQRLEWIAWIYAGT GGTSYAQKFQERVQLTVDMSTSTAYMEFSSLRSEDTAVYYCARHGSYFDYWGQG TLVTVSS (SEQ ID NO: 13), ou la séquence en acides aminés QMQLVQSGPEVKKPGTSVKVSCKASGFTFSSSYISWLRQARGQRLEWIAWIYAGT GGTSYAQKFQERVTITVDMSTSTAYMEFSSLRSEDTAVYYCARHGSYFDYWGQGT LVTVSS (SEQ ID NO : 14) ;
   et
le domaine variable de la chaine légère dudit anticorps variant humanisé ou fragment de celui-ci contient la séquence en acides aminés DIVMTQSPDSLAVSLGERATINCKSSQSVDYDGDSYMAWYQQKPGQPPKLLIYAAS NRESGVPDRFSGSGSGTDFTLTISSLQAEDVAVYYCQQSNEDPYTFGQGTKLEIK (SEQ ID NO: 15), ou la séquence en acides aminés DIQLTQSPSSLSASVGDRVTITCRASQSVDYDGDSYMNWYQQKPGKAPKLLIYAAS SLESGVPSRFSGSGSGTDFTLTISSLQPEDFATYYCQQSNEDPYTFGQGTKLEIK (SEQ ID NO : 16).

Par exemple, un anticorps variant humanisé ou fragment de celui-ci selon la présente invention est caractérisé en ce que :
le domaine variable de la chaine lourde dudit anticorps variant humanisé ou fragment de celui-ci contient la séquence SEQ ID NO : 5, ou SEQ ID NO : 6, ou SEQ ID NO : 7 ou SEQ ID NO : 8, et le domaine variable de la chaine légère dudit anticorps variant humanisé ou fragment de celui-ci contient la séquence SEQ ID NO : 15 ;
   ou
le domaine variable de la chaine lourde dudit anticorps variant humanisé ou fragment de celui-ci contient la séquence SEQ ID NO : 9, ou SEQ ID NO : 10, et le domaine variable de la chaine légère dudit anticorps variant humanisé ou fragment de celui-ci contient la séquence SEQ ID NO : 16 ;
   ou
le domaine variable de la chaine lourde dudit anticorps variant humanisé ou fragment de celui-ci contient la séquence SEQ ID NO : 11 ou SEQ ID NO : 12, et le domaine variable de la chaine légère dudit anticorps variant humanisé ou fragment de celui-ci contient la séquence SEQ ID NO : 16 ;
   ou
le domaine variable de la chaine lourde dudit anticorps variant humanisé ou fragment de celui-ci contient la séquence SEQ ID NO : 13 ou SEQ ID NO : 14, et le domaine variable de la chaine légère dudit anticorps variant humanisé ou fragment de celui-ci contient la séquence SEQ ID NO : 16.

Par exemple, un anticorps variant humanisé ou fragment de celui-ci selon la présente invention est caractérisé en ce qu'il comprend le domaine variable de la chaine lourde et le domaine variable de la chaine légère de séquences respectives suivantes : SEQ ID NO : 9 et SEQ ID NO : 16 (CF12-13(VHB)-VL139) ; SEQ ID NO : 10 et SEQ ID NO : 16 (CF12-13(VHC)-VL139) ; SEQ ID NO : 11 et SEQ ID NO : 16 (CF12-366(VHB)-VL139) ; SEQ ID NO : 12 et SEQ ID NO : 16 (CF12-366(VHC)-VL139) ; SEQ ID NO : 5 et SEQ ID NO : 15 (CF12-13(H0)-VL0) ; SEQ ID NO : 6 et SEQ ID NO : 15 (CF12-13(H1)-VL0) ; SEQ ID NO : 7 et SEQ ID NO : 15 (CF12-13(H2)-VL0) ; SEQ ID NO : 8 et SEQ ID NO : 15 (CF12-13(H3)-VL0) ; SEQ ID NO : 13 et SEQ ID NO : 16 (CF12-158(VHB)-VL139) ; SEQ ID NO : 14 et SEQ ID NO : 16 (CF12-158(VHC)-VL139).

Par exemple, un anticorps variant humanisé ou fragment de celui-ci selon la présente invention est caractérisé en ce qu'il comprend le domaine variable de la chaine lourde et le domaine variable de la chaine légère de séquences respectives suivantes : SEQ ID NO : 6 et SEQ ID NO : 15 (CF12-13(H1)-VL0) ; SEQ ID NO : 8 et SEQ ID NO : 15 (CF12-13(H3)-VL0) ; SEQ ID NO : 12 et SEQ ID NO : 16 (CF12-366(VHC)-VL139) ; SEQ ID NO : 9 et SEQ ID NO : 16 (CF12-13(VHB)-VL139) ; SEQ ID NO : 11 et SEQ ID NO : 16 (CF12-366(VHB)-VL139) ; SEQ ID NO : 14 et SEQ ID NO : 16 (CF12-158VHC-VL139).

De préférence, un anticorps variant humanisé ou fragment de celui-ci selon la présente invention est caractérisé en ce qu'il comprend le domaine variable de la chaine lourde contenant la séquence SEQ ID NO : 8 ou SEQ ID NO : 9, et le domaine variable de la chaine légère contenant la séquence SEQ ID NO : 15 ou SEQ ID NO : 16, respectivement. En particulier, il s'agit de l'anticorps variant humanisé ou fragment de celui-ci selon la présente invention caractérisé en ce qu'il comprend le domaine variable de la chaine lourde contenant la séquence SEQ ID NO : 8 et le domaine variable de la chaine légère contenant la séquence SEQ ID NO : 15 ; ou le domaine variable de la chaine lourde contenant la séquence SEQ ID NO : 9 et le domaine variable de la chaine légère contenant la séquence SEQ ID NO : 16.

Préférentiellement, un anticorps variant humanisé ou fragment de celui-ci selon la présente invention est caractérisé en ce qu'il comprend le domaine variable de la chaine lourde contenant la séquence SEQ ID NO : 9, et le domaine variable de la chaine légère contenant la séquence SEQ ID NO : 16.

| Séquence complète de la chaîne lourde du variant 13VHB-139VL (CF12-13VHB) | |
|---|---|
| Séquence peptidique SEQ ID NO : 20 | QVQLVQSGAEVKKPGASVKVSCKTSGFTFSSSYISWLRQAPGQRLEWIAWI YAGTGGTSYSQKFQGRVQLTVDTSASTAYMEFSSLRSEDTAVYYCARHGS YFDYWGQGTLVTVSSASTKGPSVFPLAPCSRSTSESTAALGCLVKDYFPEP VTVSWNSGALTSGVHTFPAVLQSSGLYSLSSVVTVPSSSLGTKTYTCNVDH KPSNTKVDKRVESKYGPPCPPCPAPEFLGGPSVFLFPPKPKDTLMISRTPE VTCVVVDVSQEDPEVQFNWYVDGVEVHNAKTKPREEQFNSTYRVVSVLTV LHQDWLNGKEYKCKVSNKGLPSSIEKTISKAKGQPREPQVYTLPPSQEEMT KNQVSLTCLVKGFYPSDIAVEWESNGQPENNYKTTPPVLDSDGSFFLYSKL TVDKSRWQEGNVFSCSVMHEALHNHYTQKSLSLSLG |

| Séquence complète de la chaîne légère du variant 13VHB-139VL (CF12-VL139) | |
|---|---|
| Séquence peptidique SEQ ID NO : 21 | DIQLTQSPSSLSASVGDRVTITCRASQSVDYDGDSYMNWYQQKPGKAPKL LIYAASSLESGVPSRFSGSGSGTDFTLTISSLQPEDFATYYCQQSNEDPYTF GQGTKLEIKRTVAAPSVFIFPPSDEQLKSGTASVVCLLNNFYPREAKVQWK VDNALQSGNSQESVTEQDSKDSTYSLSSTLTLSKADYEKHKVYACEVTHQ GLSSPVTKSFNRGEC |

La présente invention a également pour objet une composition pharmaceutique caractérisée en ce qu'elle comprend un anticorps variant humanisé ou fragment de celui-ci selon la présente invention, et un véhicule pharmaceutiquement acceptable.

On entend par « véhicule pharmaceutiquement acceptable », par exemple, une solution isotonique stérile, ou un tampon phosphate salin stérile (ex PBS), ou un tampon de formulation approprié.

Selon un mode particulier de réalisation de la présente invention, la composition pharmaceutique de la présente invention peut comprendre en outre de la FSH, ou de la LH, ou un mélange FSH et LH, ou de la hMG, ou un mélange FSH et hMG, ou un mélange LH et hMG, ou de la hCG, ou un mélange hCG et FSH, ou un mélange hCG et hMG. Ces hormones pouvant être extraites ou recombinantes.

La présente invention a également pour objet un anticorps variant humanisé ou fragment de celui-ci selon la présente invention ou une composition pharmaceutique selon la présente invention, pour une utilisation comme médicament, en particulier pour potentialiser la bioactivité de la FSH. Par exemple ledit médicament est destiné à induire l'ovulation ou la polyovulation chez un mammifère femelle, et/ou à stimuler la stéroïdogenèse chez un mammifère mâle ou femelle (par exemple en augmentant le taux d'oestradiol et/ou de progestérone endogènes circulants chez un mammifère femelle). Par exemple ledit médicament est destiné à restaurer et/ou stimuler la spermatogénèse chez un mammifère mâle afin d'augmenter le niveau de production des spermatozoïdes et leur qualité (mobilité et morphologie) et augmenter ainsi leur nombre et leur concentration dans le sperme.

La présente invention a également pour objet un anticorps variant humanisé ou fragment de celui-ci selon la présente invention ou une composition pharmaceutique selon la présente invention, pour une utilisation dans la prévention ou le traitement de l'infertilité ou de l'hypofertilité chez un mammifère mâle ou femelle. Par exemple, il s'agit de la prévention ou le traitement de l'infertilité dans le cas d'un hypogonadisme hypogonadotrope, pour restaurer et stimuler une spermatogénèse chez un mammifère mâle ou dans le cas d'une azoospermie non obstructive idiopathique ou non-idiopathique; ou dans le cas d'un hypogonadisme hypogonadotrope, pour restaurer et stimuler une ovogenèse chez un mammifère femelle. Par exemple, il s'agit de la prévention ou du traitement de l'hypofertilité dans le cas d'une oligozoospermie, oligoasthénospermie, oligotératospermie, oligoasthénotératospermie, asthénospermie, tératospermie ou asthénotératospermie chez un mammifère mâle ; ou dans le cas d'une anovulation, d'un dysfonctionnement de l'ovaire ou d'une faible réserve ovarienne (faible nombre de follicules à antrum) chez un mammifère femelle.

Dans le cas d'un mammifère femelle souffrant d'infertilité ou d'hypofertilité, l'administration de l'anticorps variant humanisé ou fragment de celui-ci selon la présente invention ou de la composition pharmaceutique selon la présente invention, va permettre une stimulation ovarienne en vue d'une procréation naturelle avec ou sans insémination artificielle, ou d'une procréation médicalement assistée (PMA) par Insémination Artificielle (IA) ou par Fécondation In Vitro (FIV) avec ou sans ICSI (Intra Cytoplasmic Sperm Injection) ou d'une Maturation In Vitro (MIV) des ovocytes.

Il est à noter que l'administration de l'anticorps variant humanisé ou fragment de celui-ci selon la présente invention ou de la composition pharmaceutique selon la présente invention, à un mammifère femelle sain, va également permettre de stimuler l'ovaire et déclencher l'ovulation dans le cadre d'une procréation naturelle avec ou sans insémination artificielle. L'administration de l'anticorps variant humanisé ou fragment de celui-ci selon la présente invention ou de la composition pharmaceutique selon la présente invention, à un mammifère femelle sain, va permettre de stimuler le recrutement et la croissance des follicules ovariens ainsi que la maturation des ovocytes qui seront ponctionnés dans le cas d'une Fécondation In Vitro (FIV).

Dans le cas d'un mammifère mâle souffrant d'infertilité, comme l'hypogonadisme hypogonadotrope, ou d'hypofertilité, comme l'oligozoospermie, l'oligoasthénotératospermie, l'oligoasthénospermie, l'oligotératospermie, l'oligoasthénotératospermie, l'asthénospermie, la tératospermie ou l'asthénotératospermie, l'administration de l'anticorps variant humanisé ou fragment de celui-ci selon la présente invention ou de la composition pharmaceutique selon la présente invention, va permettre de stimuler ou restaurer la spermatogénèse augmentant ainsi la production et la qualité des spermatozoïdes à un niveau suffisant pour permettre une fécondation naturelle avec ou sans IA, ou une fécondation artificielle dans le cas d'une FIV classique ou avec ICSI.

On entend par « infertilité/hypofertilité hormono dépendante » au sens de la présente invention, une infertilité/hypofertilité pouvant être due à une insuffisance hormonale par exemple à de faibles concentrations circulantes de FSH ou absence de cette hormone résultant d'une cause externe (e.g. les pesticides, les perturbateurs endocriniens) ou interne (*e*.*g*. insuffisance hypophysaire ou hypothalamique congénitale ou traumatique, cancer, intervention chirurgicale, ou problème de réceptivité des gonades à la FSH due à une anomalie des récepteurs ou de la FSH par exemple une mutation ou un polymorphisme des récepteurs FSH).

Au sens de la présente invention, l'hypofertilité peut aussi concerner des mammifères mâles ou femelles ayant des niveaux circulants de FSH normaux. Dans ce cas l'administration de l'anticorps variant humanisé ou fragment de celui-ci selon la présente invention ou de la composition pharmaceutique selon la présente invention, seul sans hormones exogènes, va permettre de potentialiser l'activité de la FSH endogène et de stimuler la folliculogenèse et la maturation ovocytaire chez un mammifère femelle et la spermatogénèse chez un mammifère mâle.

Au sens de la présente invention, l'hypofertilité peut aussi concerner des mammifères femelles ayant une faible réserve ovarienne et qualifiées de « faibles répondeuses » car ne répondant pas aux traitements hormonaux de stimulation ovarienne. Dans ce cas l'administration de l'anticorps variant humanisé ou fragment de celui-ci selon la présente invention, seul ou en association avec un traitement de FSH exogène et/ou hMG exogène, va permettre d'amplifier la réponse des cellules cibles à la FSH et augmenter ainsi i) le nombre de follicules ovariens stimulés de taille suffisante pour réaliser une ponction et ii) le nombre d'ovocytes matures ponctionnés, condition indispensable pour envisager une FIV.

La présente invention a également pour objet un anticorps variant humanisé ou fragment de celui-ci selon la présente invention ou une composition pharmaceutique selon la présente invention, pour une utilisation pour stimuler la procréation chez un mammifère femelle.

L'anticorps variant humanisé ou fragment de celui-ci selon la présente invention ou la composition pharmaceutique selon la présente invention, peut être utilisé(e) chez l'homme ou l'animal, en particulier les rongeurs, et le primate non-humain.

L'anticorps variant humanisé ou fragment de celui-ci selon la présente invention ou la composition pharmaceutique selon la présente invention, peut être administré(e) par injection, par exemple, intramusculaire, intraveineuse, intrapéritonéale, sous-cutanée, transcutanée, intradermique, intraorbitaire, intraoculaire, ophtalmique, ou par voie transoculaire, sans altérer leur effet potentialisant de la bioactivité de la FSH, de la LH et/ou de la hCG.

Même si dans la présente description il est fait référence à une composition pharmaceutique, on comprend bien que chacun des composés de la composition peut être administré concomitamment aux autres composés (par exemple dans une seule composition ou dans deux compositions, chacune de ces compositions comprenant un ou plusieurs des composants précités, le mode d'administration de chacun des composés ou composition(s) pouvant être identique ou différent), ou indépendamment les uns des autres, par exemple successivement, par exemple administrations indépendantes d'un anticorps variant humanisé ou fragment de celui-ci selon la présente invention et d'une hormone ou mélange d'hormones précitées, ces administrations étant réalisées sur un même mammifère. Ces différentes administrations peuvent être réalisées, indépendamment l'une de l'autre ou de manière liée (composition ou co-administration), par un mode d'administration identique ou différent (injection, ingestion, application topique, etc.). L'anticorps variant humanisé, ou fragment de celui-ci, peut être administré une seule fois chez la femelle et toutes les deux semaines pendant plusieurs mois chez le mâle. L'anticorps variant humanisé, ou fragment de celui-ci, peut être administré seul ou en association avec une administration d'hormone ou d'un mélange d'hormones précitées, cette dernière se faisant une ou plusieurs fois par semaine, pendant deux à plusieurs semaines.

### BREVE DESCRIPTION DES FIGURES

La Figure 1 représente la liaison d'anticorps variants humanisés selon l'invention sur FSH (A) et hCG (B).
La Figure 2 représente l'effet potentialisant *in vitro* sur la FSH humaine d'anticorps variants humanisés selon l'invention produits en lignée CHO transitoire : (A) variants HOLO, H1L0, H2L0 et H3L0 (B) variants 13VHB- et 13VHC-139VL (C) variants 158VHB-, 158VHC-, 366VHB- et 366VHC-139VL.
La Figure 3 représente l'effet potentialisant *in vitro* sur la FSH humaine d'anticorps variants humanisés selon l'invention produits en lignée CHO stable : (A) variants H3L0, H1L0 (B) variants 13VHB-, 158VHC-, 366VHB-, 366VHC-139VL (C) variant 13VHB-139VL.
La Figure 4 représente l'effet potentialisant *in vitro* sur différentes préparations pharmaceutiques de FSH humaine et sur la hMG, de l'anticorps variant humanisé 13VHB-139VL (SEQ ID NO : 9 et SEQ ID NO : 16) : (A) sur Fostimon^{®} et Menopur^{®} (B) sur Bemfola^{®} et Ovaleap^{®} (C) sur Puregon^{®} et Rekovelle^{®}.

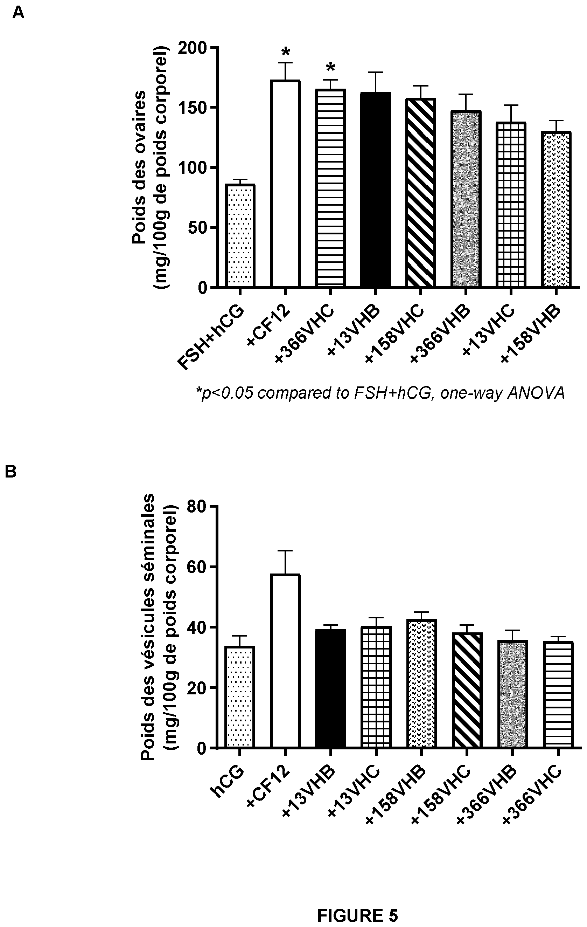

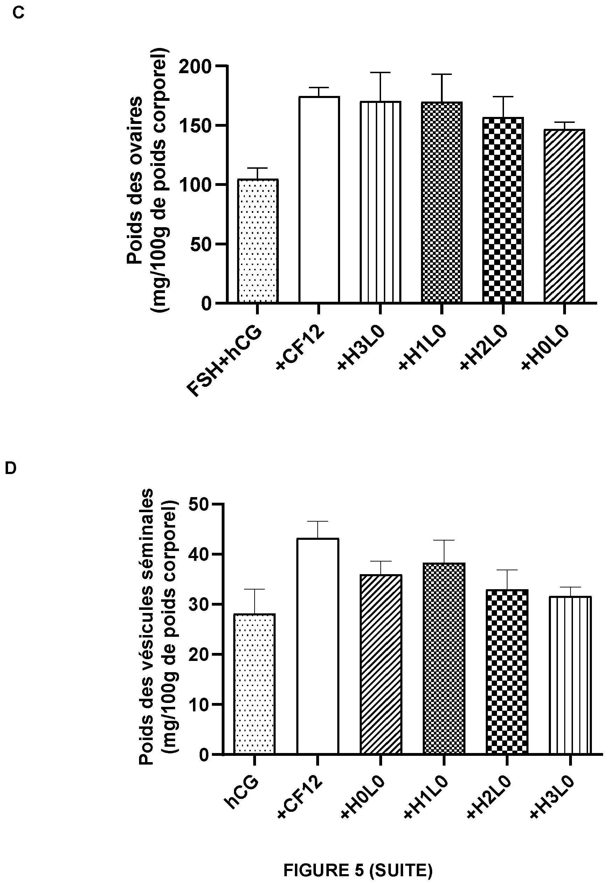

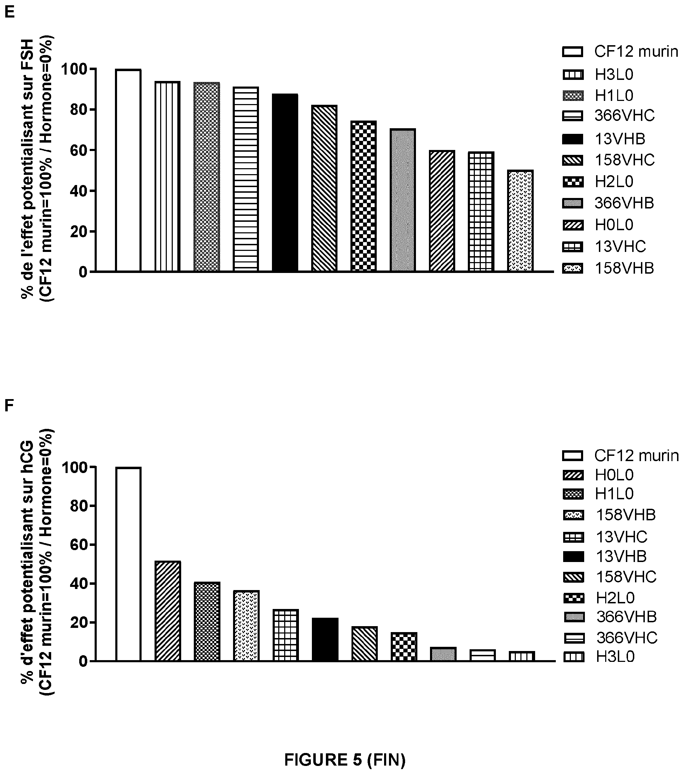

La Figure 5 représente l'effet potentialisant *in vivo* sur les FSH et LH/CG humaines, chez la rate et le rat immatures, d'anticorps variants humanisés selon l'invention produits en lignée transitoire: (A) effet des variants 366VHC-, 13VHB-, 158VHC-, 366VHB-, 13VHC- et 158VHB-139VL sur la FSH chez la rate ; (B) effet des variants 366VHC-, 13VHB-, 158VHC-, 366VHB-, 13VHC- et 158VHB-139VL sur la LH/hCG chez le rat ; (C) effet des variants H0L0, H1L0, H2L0 et H3L0 sur la FSH chez la rate (D) ; effet des variants H0L0, H1L0, H2L0 et H3L0 sur la hCG chez le rat ; (E) classement des meilleurs variants selon leur effet potentialisant sur la FSH (F) ; classement des meilleurs variants selon leur effet potentialisant sur la hCG.
La Figure 6 représente l'effet potentialisant *in vivo* sur les FSH (A) et LH/CG (B) humaines chez la rate et le rat immatures, respectivement, d'anticorps variants humanisés (H1L0, H3L0, 13VHB-139VL, 366VHB-139VL, 366VHC-139VL, 158VHC-139VL) selon l'invention, produits en lignée CHO stable.
La Figure 7 représente l'effet potentialisant *in vivo* sur les FSH (A, C, D, E) et LH/CG (B) humaines chez le Rat immature mâle et femelle, respectivement, de l'anticorps variant humanisé 13VHB-139VL (SEQ ID NO : 9 et SEQ ID NO : 16).
La Figure 8 représente les différents stades de maturation d'ovocytes (stades vésicule germinative (VG), métaphase 1 (MI) et métaphase 2 de la méïose (MII)) de singe Cynomolgus femelle, observés en microscopie optique et confocale.
La Figure 9 représente l'effet potentialisant des variants humanisés H1L0 et 366VHC-139VL sur la sécrétion d'oestradiol (A) et de progestérone (B) chez le singe cynomolgus femelle ayant reçu une seule injection du variant au premier jour du traitement et une injection par jour de 37,5 UI de FSH humaine pendant 12 jours.
La Figure 10 représente l'effet potentialisant des variants humanisés H3L0, 366VHB-139VL et 13VHB-139VL sur le nombre et la taille des follicules ovariens observés au treizième jour du traitement chez le singe cynomolgus femelle ayant reçu une seule injection du variant au premier jour du traitement et une injection de 37,5 UI de FSH humaine pendant 12 jours.
La Figure 11 représente l'effet potentialisant des variants humanisés H3L0, 366VHB-139VL et 13VHB-139VL, sur la sécrétion d'oestradiol (A) et de progestérone (B) chez le singe cynomolgus femelle ayant reçu une seule injection du variant au premier jour du traitement et une injection par jour de 37,5 UI de FSH humaine pendant 12 jours.
La Figure 12 représente l'effet potentialisant de l'anticorps variant humanisé 13VHB-139VL (SEQ ID NO : 9 et SEQ ID NO : 16) sur la reprise et la stimulation de la spermatogénèse chez le rat mâle adulte azoospermique traité avec hCG + hMG en combinaison avec 13VHB-139VL, par l'augmentation du poids des testicules (A), de la réserve testiculaire (B) et du nombre de spermatozoïdes épididymaires (C et D).
La Figure 13 représente les coupes histologiques de testicules de rat mâle adultes azoospermiques, non traités (A), traités avec hCG + hMG (B), et traités avec hCG + hMG en combinaison avec l'anticorps variant humanisé 13VHB-139VL (SEQ ID NO : 9 et SEQ ID NO : 16) (C).
La Figure 14 représente l'effet potentialisant de l'anticorps variant humanisé 13VHB-139VL (SEQ ID NO : 9 et SEQ ID NO : 16) sur la reprise et la stimulation de la spermatogénèse chez la souris hypogonadique *hpg -*/*-* mâle adulte traité avec FSH + hCG seules ou en combinaison avec 13VHB-139VL.
La Figure 15 représente l'effet potentialisant de l'anticorps variant humanisé 13VHB-139VL (SEQ ID NO : 9 et SEQ ID NO : 16) sur la reprise et la stimulation de la spermatogénèse chez la souris hypogonadique *hpg -*/*-* mâle adulte traité avec hMG seule ou en combinaison avec 13VHB-139VL.
La Figure 16 représente l'effet potentialisant de l'anticorps variant humanisé 13VHB-139VL (SEQ ID NO : 9 et SEQ ID NO : 16) sur la reprise et la stimulation de la spermatogénèse chez la souris hypogonadique *hpg -*/*-* mâle adulte traité avec FSH puis avec hMG seules ou en combinaison avec 13VHB-139VL : effet observé sur le poids des testicules (A), la réserve testiculaire (B) et le nombre de spermatozoïdes épididymaires (C et D).
La Figure 17 représente l'analyse des populations cellulaires du testicule par cytométrie en flux, chez la souris hypogonadique *hpg* -/- mâle adulte non traité (A), traité avec FSH puis hMG (B), ou traité avec FSH puis hMG en combinaison avec l'anticorps variant humanisé 13VHB-139VL (SEQ ID NO : 9 et SEQ ID NO : 16) (C).
La Figure 18 représente la liaison sur la FSH humaine, la hMG et la hCG du fragment scFv dont la séquence peptidique (SEQ ID NO : 18) est dérivée de l'anticorps variant humanisé 13VHB-139VL (SEQ ID NO : 9 et SEQ ID NO : 16) selon l'invention.
La Figure 19 représente l'effet potentialisant *in vitro,* sur la FSH humaine (A) et sur la hMG (B) de l'anticorps variant humanisé 13VHB-139VL (SEQ ID NO : 9 et SEQ ID NO : 16) et de son scFv (SEQ ID NO : 18) comparativement.
La Figure 20 représente l'effet potentialisant *in vitro* du scFv 13VHB (SEQ ID NO : 18) sur la bioactivité LH de la hCG.

### EXEMPLES

### EXEMPLE 1 : OBTENTION DES VARIANTS HUMANISES DE L'ANTICORPS CF12.

Dans ces variants, les séquences des CDRs (*Complementary Determining Regions,* régions hypervariables des régions variables des chaînes lourdes et légères de CF12 qui constituent les éléments du paratope et permettent de déterminer la complémentarité de l'anticorps avec l'épitope de l'antigène) sont strictement identiques à celles de l'anticorps monoclonal CF12. A l'inverse, les séquences des Framework (FRs) (régions encadrant les CDRs constituant la « charpente » et donnant une configuration stable au domaine variable) portent au moins une mutation sur au moins un FR de l'anticorps monoclonal CF12. Ces structures ont été insérées dans une charpente d'immunoglobuline humaine (région Fc) d'isotype IgG4.

L'humanisation de l'anticorps monoclonal murin CF12 a été réalisée par « CDR-grafting ». Les trois régions parentales hypervariables CDRs de chaque chaîne lourde (VH) et légère (VL) de CF12 ont été greffées sur la charpente d'un domaine variable d'immunoglobuline humaine acceptrice FR (*Framework*) [5, 6]. Les séquences charpentes humaines les plus homologues aux séquences murines ont été sélectionnées à partir de bases de données de structures germinales humaines comme IgBlast (https://www.ncbi.nlm.nih.gov/igblast/) ou IMGT (http://www.imgt.org).

La méthode d'humanisation a aussi pris en considération l'introduction dans les frameworks de rétro-mutations de certains acides aminés humains en murins pour prévenir la perte d'affinité ou de stabilité de l'anticorps dues à des incompatibilités structurelles entre les frameworks humains et les CDRs murins greffés [7, 8]. Afin d'identifier les acides aminés qu'il serait nécessaire de rétro-muter, un modèle 3-D de l'anticorps parental a été construit *in silico* selon des protocoles établis [9]. Pour cela, les séquences des chaînes variables lourdes (VH) et légères (VL) ont été numérotées/annotées selon les nomenclatures IMGT (Lefranc et al., 2015 ; Kabat et al., 1991 ; Chothia et al., 1987, 1989,1998) [10-14] afin d'identifier les frameworks et les CDRs. Les résidus des frameworks et des CDRs des VL et VH ont alors été utilisés pour rechercher les séquences similaires de structures d'anticorps murins résolues via BLAST protéine. Les modèles de structure retenus pour les CDRs ont été greffés sur ceux sélectionnés pour les frameworks. Enfin, les modèles partiels de VL et VH reconstitués ont été soumis manuellement à des mutagénèses et optimisés (The PyMOL Molecular Graphics System, Version 1.8, Schrödinger, LLC)

La meilleure structure 3-D de l'anticorps parental a alors été construite à l'aide de logiciels comme MOE et PAPS (Packing Angle Prédiction Server: http://www.bioinf.org.uk/abs/paps/) [15] afin de trouver le meilleur arrangement tertiaire prédit puis optimisée [16, 17]. La comparaison entre ce modèle et ceux des anticorps humanisés avec les séquences germinales sélectionnées a conduit à la construction par rétro-mutations des variants d'humanisation de CF12.

Les séquences des germlines humaines sélectionnées pour l'humanisation de la chaîne VH sont : IGHV1-3*01, IGHV1-58*01, IGHV3-66*01.

Les séquences des germlines humaines sélectionnées pour l'humanisation de la chaîne VL sont : IGKV1-39*01 et IGKV4-1*01.

Les séquences des germlines humaines pour les jonctions VH et VL sont IGHJ4*01 et IGKJ2*01 respectivement.

Dix variants humanisés de CF12 ont ainsi été développés en insérant les chaînes variables VH et VL dans une charpente d'immunoglobuline humaine d'isotype IgG4 portant la mutation N228P et l'allotype R409K.

Les variants humanisés sont décrits dans le tableau 1 ci-dessous.

**Tableau 1**

| Nom du variant | VH | | VL | |
|---|---|---|---|---|
| | Germline | SEQ ID NO | Germline | SEQ ID N0 |
| H0L0 | IGHV1-3*01 | 5 | IGKV4-1*01 | 15 |
| H1L0 | IGHV1-3*01 | 6 | IGKV4-1*01 | 15 |
| H2L0 | IGHV1-3*01 | 7 | IGKV4-1*01 | 15 |
| H3L0 | IGHV1-3*01 | 8 | IGKV4-1*01 | 15 |
| 13VHB-139VL | IGHV1-3*01 | 9 | IGKV1-39*01 | 16 |
| 13VHC-139VL | IGHV1-3*01 | 10 | IGKV1-39*01 | 16 |
| 158VHB-139VL | IGHV1-58*01 | 13 | IGKV1-39*01 | 16 |
| 158VHC-139VL | IGHV1-58*01 | 14 | IGKV1-39*01 | 16 |
| 366 VHB-139VL | IGHV3-66*01 | 11 | IGKV1-39*01 | 16 |
| 366VHC-139VL | IGHV3-66*01 | 12 | IGKV1-39*01 | 16 |

Ces variants humanisés ont été caractérisés par rapport à leurs propriétés de liaison et par rapport à leur effet pharmacologique *in vitro* et *in vivo.*

Dans un premier temps, les variants ont été produits par expression transitoire en cellules eucaryotes. Ainsi, les variants 13VHB-139VL, 13VHC-139VL, 366VHB-139VL, 366VHC-139VL, 158VHB-139VL, 158VHC-139VL ont été produits par transfection chimique transitoire de cellules CHOEBNALT-85-1E9 par des plasmides pQMCF-1.2 comportant les inserts des séquences ADN à exprimer. Les variants H0L0, H1L0, H2L0 et H3L0 ont été produits par transfection chimique transitoire de cellules HEK et CHO par des plasmides MI-mAbs' IgGP and IgK.

Dans un deuxième temps, les six meilleurs candidats 13VHB-139VL, 366VHB-139VL, 366VHC-139VL, 158VHC-139VL, H1L0 et H3L0 ont été produits dans des lignées stables. Les lignées cellulaires ont été générées en transfectant le gène d'intérêt dans la lignée cellulaire hôte CHO-M (dérivée de CHO-K1) à l'aide de la microporation de plasmides SLXplasmid 220_Puro_BT+, suivie d'un cycle de clonage et d'une seconde transfection avec des plasmides porteurs d'un autre marqueur de sélection, SLXplasmid_221_Hygro_BT+ (SuperTransfection). Deux cycles supplémentaires de dilution d'une seule cellule ont été effectués pour obtenir des lignées cellulaires clonales super transfectées hautement productives.

Dans tous les cas, les transferts de plasmides dans les cellules ont été effectués par co-transfection d'un vecteur porteur du gène d'intérêt du VH et d'un vecteur porteur du gène d'intérêt du VL à des ratios variables entre les deux vecteurs. Les variants produits ont été purifiés par chromatographie d'affinité sur protéine A.

### EXEMPLE 2 : MESURE DE L'ACTIVITE DE LIAISON DES VARIANTS HUMANISES SUR LA FSH ET SUR LA LH/hCG

L'activité de liaison des anticorps humanisés sur les hormones recombinantes humaines FSH (GonalF^{®} 1050 UI/1,75 ml, Merck) et hCG (Ovitrelle^{®} 250 microgrammes, Merck) a été étudiée par technique ELISA. Chaque hormone a été préparée à la concentration de 10 µg/ml dans du tampon carbonate de sodium 0,1 M pH 9,6 et distribuée à raison de 100 µl par puits sur une plaque ELISA (MaxiSorp^{™}, Nunc). Le temps d'adsorption des hormones a été de 1 heure à 37°C suivi de 18 heures à +4°C. Après cinq lavages, les puits ont été traités avec 100 µl de PBS additionné de Tween 0,1% et de BSA 1% pendant 1 heure à 37°C, puis chaque anticorps a été distribué à raison de 100 µl/puits et incubé 1 heure à 37°C. Sur chaque hormone évaluée, les anticorps ont été distribués à différentes concentrations selon une gamme de 6,25 à 100 µg/ml.

Après cinq lavages, un anticorps secondaire couplé à la peroxydase (HRP) (anti-human IgG4 Fc, Abcam 99817) dilué au 1/2500^{e} a été distribué à raison de 100 µl/puits et incubé 1 heure à 37°C. Après cinq lavages, l'activité enzymatique a été révélée avec du TMB distribué à raison de 100 µl/puits. Le temps de révélation a été de 1 heure à température ambiante. Après arrêt de la réaction avec H₂SO₄ 1 M (50 µl/puits) l'intensité de la réaction colorée (Densité Optique - DO) a été mesurée à l'aide d'un spectrophotomètre pour plaques ELISA.

Le tableau 2 ci-dessous indique les valeurs de densité optique obtenues avec les dix variants à la concentration de 100µg/ml sur la FSH et la hCG.

**Tableau 2**

| Variant | Liaison sur FSH (DO) | Liaison sur hCG (DO) |
|---|---|---|
| H0L0 | 2,34 | 1,5 |
| H1L0 | 1,02 | 0,65 |
| H2L0 | 1,57 | 1 |
| H3L0 | 2,28 | 1,55 |
| 13VHB | 0,3 | 0,075 |
| 13VHC | 0,24 | 0,05 |
| 158VHB | 1,94 | 0,62 |
| 158VHC | 1,03 | 0,28 |
| 366VHB | 1,73 | 0,95 |
| 366VHC | 0,3 | 0,09 |

Les résultats obtenus par technique ELISA démontrent que les variants ont une meilleure liaison sur la FSH que sur la hCG. Ils révèlent une forte variabilité de l'activité de liaison entre les variants, la valeur de DO allant de 2,28 à 0,24.

La liaison croisée obtenue sur la hCG est faible et peut s'expliquer par le fait que la FSH humaine et la hCG ont la même sous-unité alpha contrairement à la sous-unité béta qui diffère d'une hormone à l'autre.

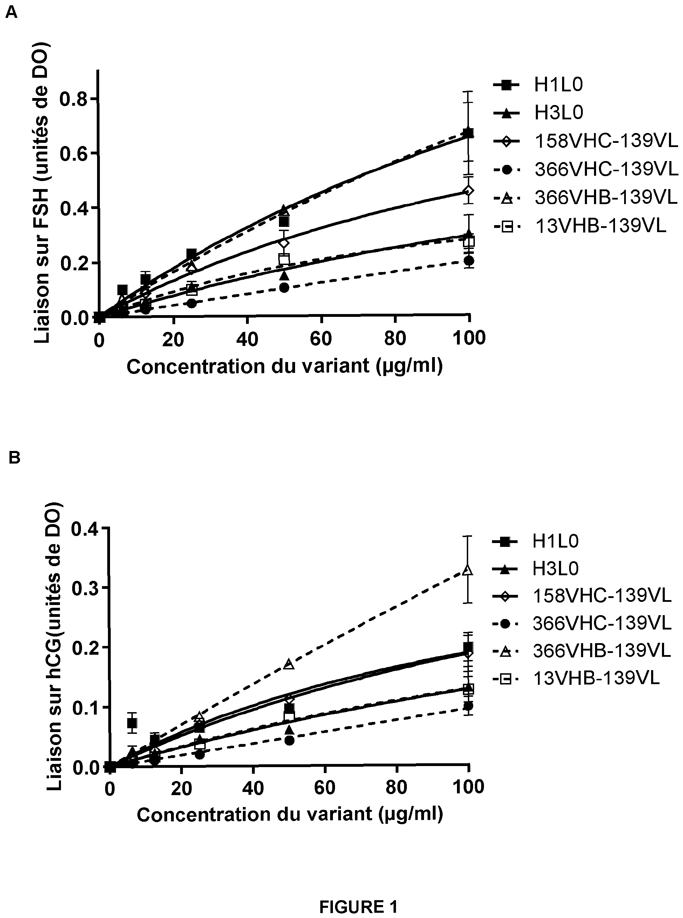

La même analyse a été conduite avec les six variants sélectionnés, produits en lignée CHO stable. Les résultats obtenus avec une gamme d'anticorps allant de 5 à 100 µg/ml sur la FSH et la hCG adsorbées sont illustrés sur la Figure 1. Chaque variant se lie préférentiellement sur la FSH humaine (Figure 1A) et présente une plus faible liaison sur la hCG (Figure 1B).

Les niveaux de liaison obtenus sur la FSH sont variables selon les anticorps avec un ordre d'intensité de liaison globalement identique entre les deux types de production (expression en lignée transitoire et en lignée stable).

### EXEMPLE 3 : MESURE IN VITRO DE L'EFFET POTENTIALISANT DES VARIANTS HUMANISES SUR LA BIOACTIVITE DE LA FSH ET DE LA LH/hCG

L'effet potentialisant des variants humanisés sur la bioactivité de la FSH humaine a été mesurée *in vitro* sur des cellules HEK 293 exprimant le récepteur FSH humain. Les cellules ont été stimulées avec une gamme de FSH humaine (Gonal-f ^{®}, Merck, France) seule ou en combinaison avec un variant humanisé.

La comparaison de la courbe dose-réponse obtenue avec la FSH ou avec le complexe FSH/variant a permis de quantifier l'effet potentialisant de chacun des dix variants sur l'activité de la FSH et de sélectionner les meilleurs candidats en fonction de cette propriété.

### Sur cellules HEK 293 hFSHR GloSensor^{®}

L'effet potentialisant des variants humanisés sur l'activité de la FSH a été évalué *in vitro* sur des cellules HEK 293 exprimant de façon stable d'une part le récepteur humain de la FSH, et d'autre part un senseur de l'adénosine monophosphate cyclique (AMPc), le GloSensor^{®} (Promega référence E2401, Charbonnières-les-Bains, France). Les cellules ont été distribuées à raison de 80 000 cellules par puits, dans des boites 96 puits à contours et fond blancs (Greiner Bio One référence 655083, Dominique Dutscher, Brumath, France), dans du milieu EMEM (Lonza référence BE12-611F, Ozyme, Saint Cyr l'Ecole, France) supplémenté avec 10% de sérum de veau foetal (Eurobio CVFSVF00 01, Les Ulys, France) décomplémenté au préalable, 1% pénicilline/streptomycine (Lonza référence DE17-602E) et incubées toute la nuit dans un incubateur à 37°C 5% CO₂ sous atmosphère humide. Le lendemain, le milieu des cellules a été aspiré et remplacé par du milieu de culture EMEM contenant 1% d'HEPES (Lonza référence BE17-737E, Ozyme, Saint Cyr l'Ecole, France) et du substrat GloSensor^{®} (Promega référence E1291, Charbonnières-les-Bains, France) à raison de 4 µl de substrat dans 100 µl de milieu par puits. Après 2 heures d'incubation à 21°C sous atmosphère humide et à l'abri de la lumière, 10 µl d'une préparation de FSH ou de FSH + variant a été ajoutée sur les cellules et la luminescence émise a été quantifiée à l'aide d'un lecteur de plaques 96 puits LUMIstar Oméga (BMG Labtech, Champigny sur Marne, France). La luminescence a été mesurée pendant environ 60 minutes.

L'effet potentialisant a été quantifié en comparant les courbes dose-réponse obtenues avec une gamme de concentrations de FSH (Gonal-f ^{®}) allant de 10⁻¹¹ M à 10⁻⁸ M, préparée seule ou en combinaison avec un variant préparé à une concentration fixe de 10 µg/ml (M). Le mélange FSH+variant a été préincubé 20 minutes à 37°C avant le dépôt sur les cellules. Ces courbes dose-réponse représentent le signal maximal de luminescence obtenu lors de la stimulation des cellules à chacune des concentrations de FSH seule ou avec le variant.

Les résultats obtenus ont été analysés avec le logiciel Prism (GraphPad Prism Software Inc., San Diego, CA, USA, version 10.0.1). La fonction non linéaire « log (agoniste) versus response » a été utilisée pour tracer la réponse en fonction de la concentration de FSH et caractériser l'EC50 de la FSH seule, l'EC50 de la FSH complexée au variant testé (puissance) et le signal maximal de la réponse (efficacité) dans chacun des cas.

Afin de pouvoir comparer les courbes entre elles, le signal luminescent est exprimé en pourcentage par rapport à la réponse maximale obtenue avec la FSH seule qui est fixée à 100%. Le niveau basal de la réponse est fixé à 0%.

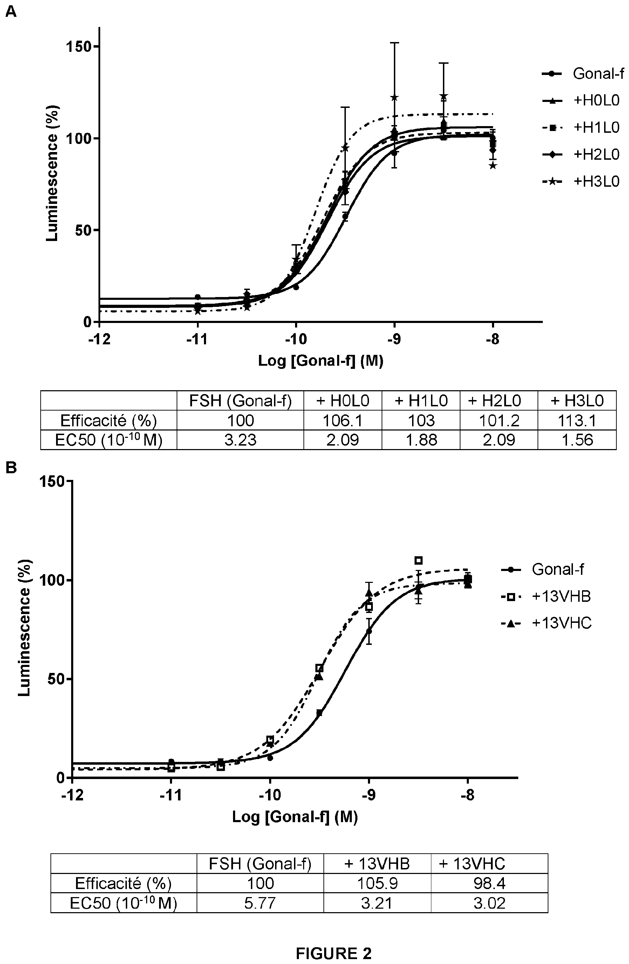

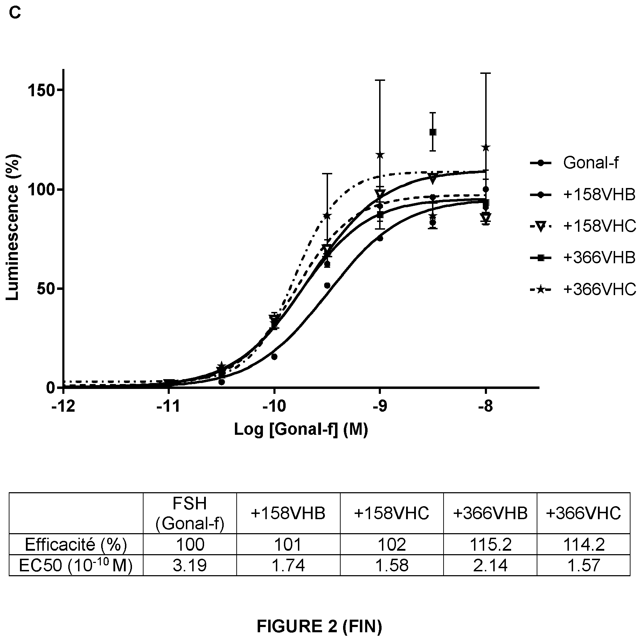

Cette étude a tout d'abord été réalisée avec les dix variants humanisés produits en cellules CHO transfectées de façon transitoire dans le but de les sélectionner par rapport à leur effet potentialisant sur l'activité de la FSH. Les résultats obtenus sont illustrés Figure 2A pour les variants H0L0, H1L0, H2L0 et H3L0, Figure 2B pour les variants 13VHB- et 13VHC-139VL et Figure 2C pour les variants 158VHB-, 158VHC-, 366VHB- et 366VHC-139VL. La réponse cellulaire obtenue avec chaque variant a été caractérisée par son niveau maximal (efficacité) et par son EC50 (puissance). Pour tous les variants, on observe que le complexe FSH+variant induit une augmentation de la puissance de la réponse en AMPc, avec une valeur de l'EC50 1,5 à 2 fois supérieure à celle obtenue avec la FSH seule. Pour certains variants, on observe également une augmentation de la réponse maximale avec le complexe FSH+variant allant jusqu'à 120% par rapport à la FSH seule (366 VHB et 366 VHC, H3L0, H0L0, 13VHB). Ceci traduit une meilleure efficacité de la réponse en AMPc lors d'une stimulation avec FSH+variant. La potentialisation de l'activité de la FSH atteint dans tous les cas un plateau ce qui suggère que l'effet potentialisant n'induit pas un phénomène non contrôlé d'hyperstimulation des cellules.

Les six variants ayant le plus fort effet potentialisant sur l'activité FSH ont ensuite été sectionnés et produits en lignée cellulaire CHO stable. Il s'agit des variants H3L0, H1L0 et 13VHB-, 158VHC-, 366VHB-, 366VHC-139VL.

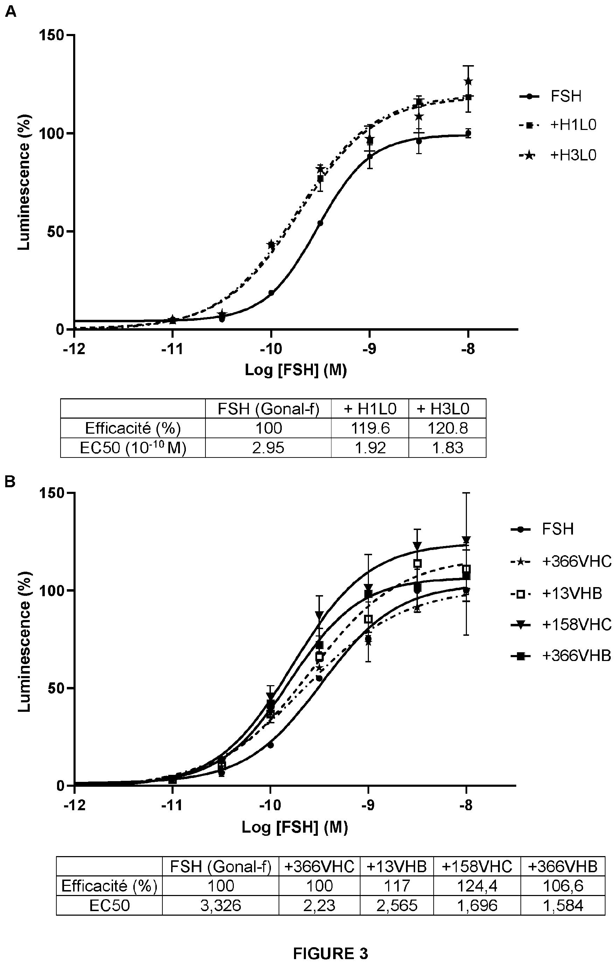

Leur effet potentialisant sur l'activité FSH a ensuite été caractérisé *in vitro,* selon le même protocole. Les résultats obtenus sont illustrés Figure 3A pour les variants H3L0, H1L0 et Figure 3B pour les variants 13VHB-, 158VHC-, 366VHB- et 366VHC-139VL. Là encore, pour tous les variants, les courbes dose réponse obtenues avec les complexes FSH+variant ont une valeur d'EC50 de 1,3 à 2,1 fois supérieure à celle obtenue avec la FSH seule, traduisant une augmentation de la puissance de la réponse en AMPc. A l'exception du variant 366VHC-139VL, on observe également une augmentation de la réponse maximale avec le complexe FSH+variant pouvant aller jusqu'à 125% par rapport à la FSH seule (par ordre croissant d'efficacité: 366VHC-, 366VHB-, 13VHB-139VL, H1L0, H3L0, 158VHC-139VL).

En conclusion, l'effet potentialisant sur la FSH est d'amplitude variable selon les variants. Il se traduit par une augmentation de la puissance de la réponse AMPc obtenu avec le complexe variant+FSH et par une augmentation de l'efficacité de la réponse avec un niveau maximal d'AMPc plus élevé que celui obtenu avec la FSH seule.

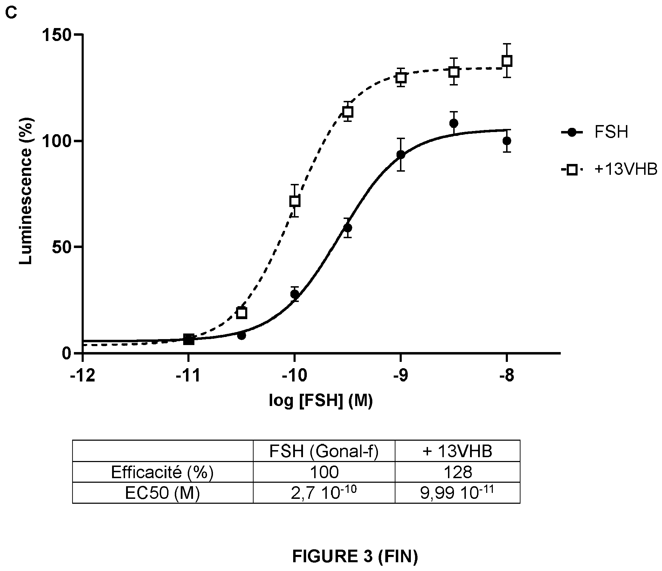

Enfin, les caractéristiques pharmacologiques du variant 13VHB-139VL, choisi après de nombreux tests *in vivo,* et produit par sa lignée clonale finale, ont été mesurées et sont illustrées Figure 3C. Les courbes représentent la moyenne de trois essais indépendants réalisés avec Gonal-f et montrent une valeur d'EC50 doublée par rapport à la FSH seule (1,1.10⁻¹⁰ M versus 2,08.10⁻¹⁰ M respectivement) et une efficacité augmentée de 130% en présence du variant, confirmant son effet potentialisant sur l'activité de la FSH.

Les propriétés potentialisantes du variant 13VHB ont également été évaluées sur d'autres préparations de FSH humaine et sur une hMG, disponibles sur le marché et utilisées en médecine de la reproduction humaine. Ces préparations sont utilisées en médecine humaine chez la femme, par exemple, pour les traitements de stimulation ovarienne dans le cadre de la PMA. Les mêmes conditions expérimentales ont été utilisées que celles décrites ci-dessus pour Gonal-f.

Il s'agit des hormones extraites purifiées :
- Fostimon ^{®} (FSH) (IBSA, Italie) ;
- Menopur ^{®} (hMG purifiée contenant 50% de FSH et 50% de LH) (Ferring, France) ;
et des FSH recombinantes :
- Biosimilaires de la Follitropine alpha : Ovaleap ^{®} (Teva, Allemagne) et Bemfola ^{®} (Gedeon Richter, France) ;
- Follitropine béta : Puregon ^{®} (MSD, USA) ;
- Follitropine delta : Rekovelle ^{®} (Ferring, France).

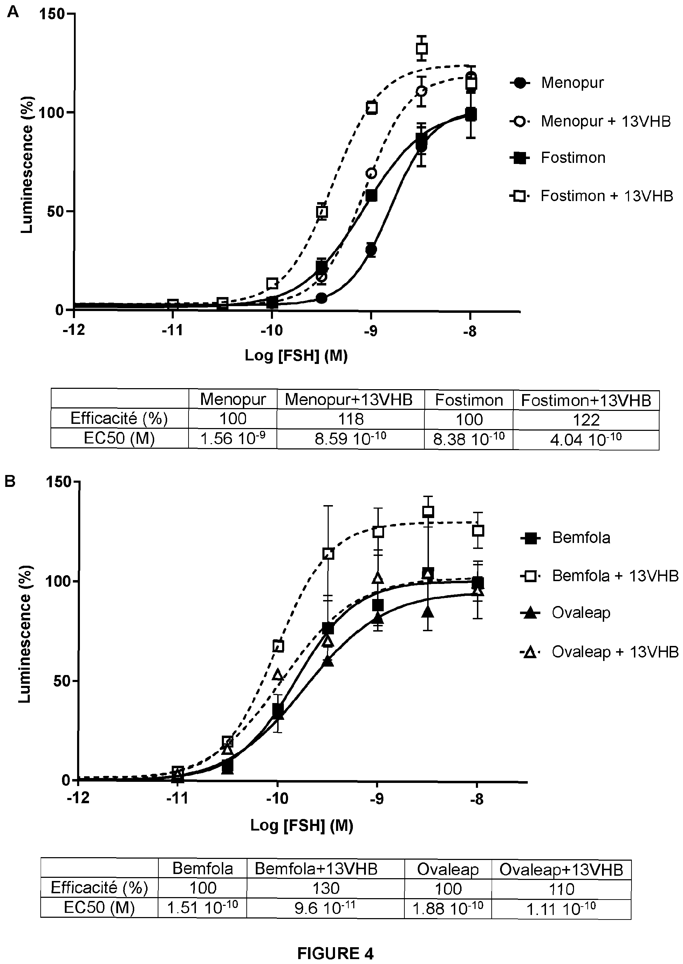

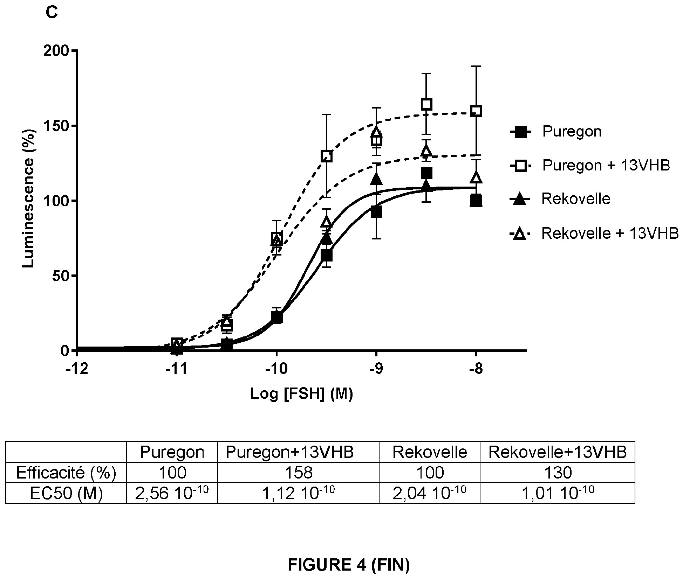

Les résultats sont illustrés Figure 4. Pour chaque hormone, trois essais indépendants ont été réalisés à chaque fois et un exemple représentatif est illustré. La Figure 4A, illustre les résultats obtenus avec les deux hormones extraites Fostimon^{®} et Menopur^{®} et montre une valeur d'EC50 doublée en présence de 13VHB et une réponse maximale de 120% par rapport à l'hormone seule indiquant une meilleure puissance et une meilleure efficacité de la réponse.

La Figure 4B illustre l'effet potentialisant de 13VHB sur l'activité des deux FSH recombinantes biosimilaires, Bemfola^{®} et Ovaleap^{®}. La valeur de l'EC50 a été augmentée de 1,57 à 1,7 fois et la réponse maximale est de 130% par rapport à l'hormone seule dans le cas de Bemfola^{®} et plus faiblement dans le cas de Ovaleap^{®} (110%).

L'effet potentialisant de 13VHB sur l'activité des follitropines béta (Puregon^{®}) et delta (Rekovelle^{®}) est illustré Figure 4C. En présence de 13VHB, l'efficacité a été augmentée de 130% dans le cas de Rekovelle^{®} et de 158% dans le cas de Puregon^{®}. Parallèlement, la puissance (EC50) est supérieure de 2 à 2,3 fois à celle de l'hormone seule dans le cas de Puregon et de Rekovelle respectivement.

En conclusion, ces résultats ont démontré que l'effet potentialisant du variant 13VHB s'exerce sur toutes les préparations de FSH et hMG disponibles sur le marché et utilisées en médecine de la reproduction humaine. La combinaison du variant 13VHB avec ces différents types de FSH et hMG permet dans tous les cas une meilleure efficacité avec une augmentation du niveau maximal de la réponse de 20 à 60% par rapport à l'hormone seule et une meilleure puissance avec augmentation de l'EC50 d'un facteur 1,6 à 2,3.

### EXEMPLE 4 : MESURE IN VIVO DE L'EFFET POTENTIALISANT DES VARIANTS HUMANISES SUR LA BIOACTIVITE DE LA FSH ET DE LA LH/hCG

L'effet potentialisant des variants humanisés sur la bioactivité de la FSH et de la hCG/LH a ensuite été étudié et caractérisé *in vivo* chez le rat immature, femelle et mâle, afin d'évaluer l'impact de l'humanisation de la séquence sur l'effet potentialisant *in vivo.* Pour cela, l'effet de chaque variant injecté en combinaison avec le traitement hormonal a été systématiquement comparé à celui de l'anticorps murin CF12 et du traitement hormonal seul.

### Mesure de l'activité FSH sur des rates immatures par le Bioassay de Steelman et Pohley

L'effet potentialisant des variants humanisés sur l'activité de la FSH a été évalué *in vivo* sur des rates Wistar Han immatures âgées de 25 jours (Charles River, L'Arbresle, France). Le protocole utilisé a été celui de Steelman et Pohley [18]. Les animaux ont reçu une injection sous-cutanée de 100 µl de [hCG 3,5 UI + hFSH 0,5 UI] ou de 100 µl du mélange [hCG 3,5 UI + hFSH 0,5 UI + variant 2 µg] 2 fois par jour, pendant 3 jours. La dose de 2 µg correspond à une concentration de l'ordre de 0,3 µg/ml de sang si l'on admet un volume de sang de 65 ml/kg chez le rat (University of Pittsburgh Policy for Regulating the Volume of Experimental Blood Sample Withdrawals in Laboratory Animals). Les variants ont été formulés dans du PBS stérile et les hormones dans le milieu fourni par le fabricant pour leur préparation.

Le 4^{ème} jour, les rates ont été euthanasiées, leurs ovaires ont été disséqués et pesés par paire. L'augmentation du poids des ovaires est proportionnelle à l'activité de la FSH injectée. Chaque lot expérimental comprenait cinq femelles.

### Mesure de l'activité la hCG/LH sur des rats immatures par le Bioassay de Scobey

L'effet des variants sur l'activité de la LH/hCG a été évalué *in vivo* sur des rats mâles immatures selon le protocole de Scobey et collaborateurs [19]. Pour cela, des rats Wistar Han âgés de 3 semaines ont reçu une injection sous-cutanée de 100 µl de hCG 1,5 UI ou de 100 µl du mélange (hCG 1,5 UI + un variant 2 µg), une fois par jour pendant quatre jours. La dose de 2 µg correspond à une concentration de l'ordre de 0.3 µg/ml de sang si l'on admet un volume de sang de 65 ml/kg chez le rat (University of Pittsburgh Policy for Regulating the Volume of Experimental Blood Sample Withdrawals in Laboratory Animals). Les variants ont été formulés dans du PBS stérile et les hormones dans le milieu fourni par le fabricant pour leur préparation.

Le 5^{ème} jour, les animaux ont été sacrifiés, et les vésicules séminales disséquées et pesées. Le poids des vésicules séminales est proportionnel à l'activité de la hCG injectée. Chaque lot expérimental comprenait cinq femelles.

Dans un premier temps les dix variants exprimés en lignée transitoire ont été caractérisés par rapport à leur effet potentialisant *in vivo* sur l'activité de la FSH et de la LH/CG et comparés à celui de l'anticorps murin CF12. L'objectif étant de sélectionner des variants sur leur effet potentialisant FSH.

### Effet potentialisant sur l'activité FSH et LH des dix variants humanisés produits en lignée transitoire

### 1/ Effet des variants humanisés comprenant la chaine légère 139VL

Une première série de six variants caractérisés par la séquence 139VL (SEQ ID NO : 16) a été évaluée dans les deux bioassays. Il s'agit des variants 13VHB, 13VHC, 158VHB, 158VHC, 366VHB et 366VHC.

Leur effet potentialisant sur l'activité de la FSH a été quantifié en comparant le poids moyen des ovaires des femelles du lot variant+FSH par rapport à celui des femelles traitées avec hormones seules. Les résultats sont illustrés Figure 5A et ont montré que les variants 366VHC, 158VHC et 13VHB ont un effet potentialisant proche de celui de l'anticorps murin CF12. Le poids moyen des ovaires des femelles traitées avec le mélange hCG/FSH+variant est de 1,91 à 1,82 fois plus élevé qu'avec le traitement hormonal seul contre 2 fois dans le lot traité avec hCG/FSH+CF12. Les trois autres variants 366VHB, 13VHC et 158VHB ont un effet potentialisant qui tend à être plus faible avec une augmentation du poids moyen des ovaires allant de 1,71 à 1,5 fois plus importante par rapport au traitement hormonal seul.

Les résultats obtenus dans le bioassay de Scobey sont illustrés Figure 5B. Ils ont montré de façon surprenante et inattendue que les variants présentent une baisse plus ou moins importante de leur effet potentialisant sur l'activité LH/CG par rapport à l'anticorps murin CF12. En effet, le poids moyen des vésicules séminales des mâles traités avec le mélange hCG+variant est de 1,26 à 1,05 fois plus élevé qu'avec la hCG seule contre 1,71 fois pour les animaux traités avec hCG+CF12. La baisse de l'effet des variants sur l'activité LH/CG représente donc une perte allant de 45% à 67% par rapport à l'anticorps murin CF12.

### 2/ Effet des variants humanisés comprenant la chaine légère L0

De la même façon, l'effet des quatre variants comprenant la chaine légère VL0 (SEQ ID NO : 15) ont été caractérisés dans les deux bioassays. Il s'agit des variants H0L0, H1L0, H2L0 et H3L0.

Leur effet potentialisant sur l'activité de la FSH est illustré Figure 5C. Les variants H1L0 et H3L0 ont montré un effet potentialisant égal à celui de l'anticorps CF12 murin avec dans les trois cas un poids moyen des ovaires 1,67 fois plus élevé que dans le lot traité avec hormones seules. Les deux autres variants, H2L0 et particulièrement H0L0, ont un effet plus faible : le poids moyen des ovaires des femelles traitées avec hCG/FSH+variant est de 1,5 et 1,4 fois plus élevé que chez les femelles ayant reçu le traitement hormonal seul contre 1,67 fois dans le lot traité avec hCG/FSH+CF12.

Les résultats obtenus dans le bioassay de Scobey sont illustrés Figure 5D et ont montré de façon surprenante et inattendue, un effet potentialisant sur l'activité LH/hCG plus faible que celui de l'anticorps murin CF12. Cette diminution est variable selon les variants mais en aucun cas l'effet potentialisant sur la LH est annulé. Le poids moyen des vésicules séminales des mâles traités avec le mélange hCG+variant est de 1,28 à 1,05 fois plus élevé qu'avec la hCG seule contre 1,54 fois pour le lot traité avec hCG+CF12. La baisse de l'effet des variants sur LH représente donc une perte allant de 26% à 49% par rapport à l'effet de l'anticorps murin CF12.

### 3/ Classement de l'ensemble des variants humanisés selon leur effet potentialisant sur FSH et LH/hCG et sélection des six meilleurs

Afin d'intégrer l'ensemble des résultats obtenus et comparer les dix variants entre eux, une formule de calcul a été utilisée permettant de quantifier le % d'effet potentialisant sur FSH et sur LH de chaque variant par rapport à CF12.

Dans ce calcul, l'effet de CF12 représente le 100% et l'effet du traitement hormonal seul est fixé à 0%.

L'effet de chaque variant est exprimé en % par rapport à CF12 selon la formule suivante : (A-C) / (B-C) *100, où
- A est la valeur obtenue avec le traitement (variant + hormone) ;
- B est la valeur obtenue avec le traitement (CF12 + hormone) ;
- C est la valeur obtenue avec le traitement hormonal seul ;
- le facteur 100 permet d'exprimer le rapport en %.

Le classement obtenu par rapport à l'effet potentialisant de l'activité de la FSH est illustré Figure 5E. Leur effet potentialisant varie de 95% à 50% par rapport à celui de CF12 (100%). Cinq variants ont un fort effet potentialisant allant de 100 à 80% : H3L0 (94%), H1L0 (93,5%), 366VHC (91,5%), 13VHB (88%) et 158VHC (82,5%). Les cinq autres ont un effet moins important compris entre 75 et 50% par rapport à CF12 (100%) : H2L0 (74,5%), 366VHB (71%), H0L0 (60%), 13VHC (59,5%) et 158VHB (50,5%).

Le classement obtenu par rapport à l'effet potentialisant sur l'activité de la LH/hCG est illustré Figure 5F et a mis en évidence un effet potentialisant plus faible des variants, allant de 52% à 5%, comparativement à celui de CF12 (100 %). Cinq variants ont un effet potentialisant allant de 52% à 20% : H0L0 (51,84%), H1L0 (40,8%), 158VHB (36,6%), 13VHC (26,8%) et 13VHB (22,3%). Les cinq autres variants ont un effet plus faible allant de 19% et 5% : 158VHC (17,9%), H2L0 (15%), 366VHB (7,4%), 366VHC (6,2%) et H3L0 (5%).

L'ensemble de ces résultats indique que l'humanisation de la séquence de l'anticorps murin CF12 a permis de conserver l'effet potentialisant des variants sur l'activité de la FSH : 7 variants sur 10 ont entre 95 et 70% de l'effet de l'anticorps murin d'origine. A l'inverse, et de façon inattendue et surprenante, l'humanisation a conduit à une diminution plus ou moins importante de l'effet potentialisant des variants sur l'activité de la LH/hCG, cinq d'entre eux ayant un effet en dessous de 20% de celui de l'anticorps murin d'origine. Il est important de souligner qu'en aucun cas cette diminution n'a conduit à une disparition totale de l'effet potentialisant sur LH/hCG. Tous les variants ont gardé à des degrés variables un effet potentialisant sur la LH/hCG.

De façon surprenante également, l'impact de l'humanisation sur l'effet potentialisant FSH n'est pas corrélé à celui observé sur l'effet potentialisant LH : un variant présentant une baisse importante de l'effet sur la LH peut avoir conservé un fort effet potentialisant sur l'activité FSH, c'est le cas de H3L0. A l'inverse, le variant ayant l'effet le plus élevé sur l'activité LH (par exemple H0L0) a un effet potentialisant sur l'activité FSH diminué, inférieur à 60%. Enfin, certains variants ont un classement homogène entre l'effet sur FSH et sur LH, c'est le cas de 158VHC par exemple.

Une étude de l'impact des séquences des germlines humaines sélectionnées pour l'humanisation de la chaîne VH ou VL sur une éventuelle modification de l'effet potentialisant n'a montré aucune corrélation entre un impact négatif sur l'effet FSH ou LH et une ou des séquences particulières de germlines.

### Effet potentialisant sur l'activité FSH et LH des six variants humanisés sélectionnés et produits en lignée stable.

Le classement des variants produits en lignée transitoire par rapport à leur effet potentialisant sur l'activité de la FSH a permis de sélectionner les six meilleurs candidats. Ces variants ont été produits en lignée CHO stable et leur effet potentialisant a été contrôlé et mesuré *in vivo* par rapport à l'effet de l'anticorps monoclonal murin CF12. La même formule de calcul que précédemment a été utilisée afin de quantifier en % l'effet potentialisant des variants humanisés sur l'activité de la FSH et de la LH/CG par rapport à CF12 dont l'effet est fixé à 100%. L'effet du traitement hormonal seul est fixé à 0%.

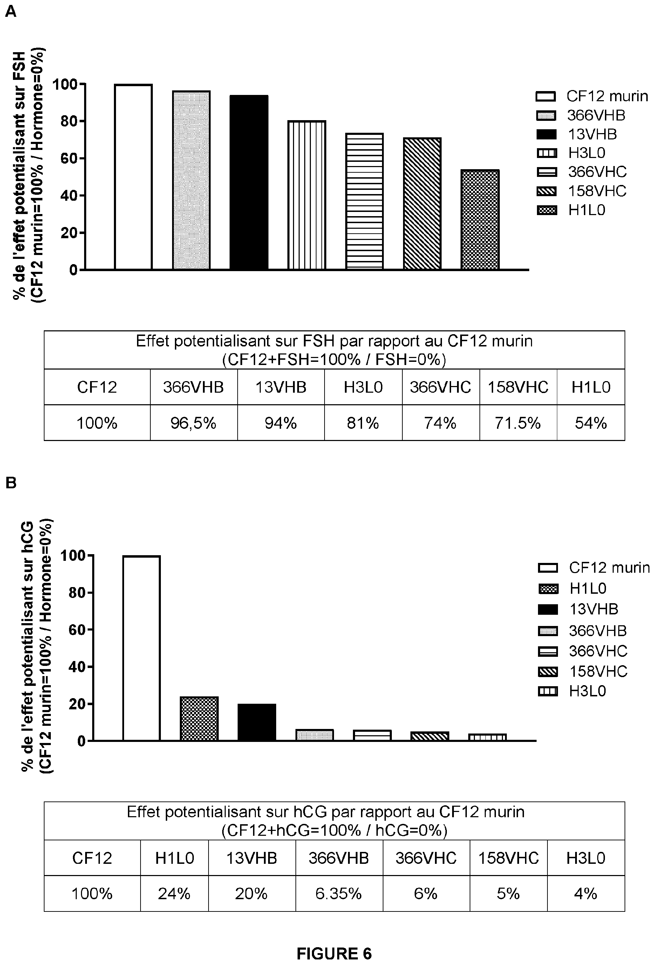

Les résultats illustrés Figure 6A montrent que l'effet potentialisant sur l'activité de la FSH est resté très élevé et stable. Les variants 366VHB, 13VHB et H3L0 sont les plus potentialisants avec respectivement un effet égal à 96,5, 94% et 81% par rapport à celui de CF12. Les variants 366VHC et 158VHB ont un effet égal à 74 et 71% par rapport à celui de CF12 respectivement. Le variant H1L0 est le seul variant à présenter une baisse de son effet potentialisant sur la FSH (54%).

Les résultats de la Figure 6B illustrent pour tous les variants, une baisse répétée de l'effet potentialisant sur l'activité LH/CG par rapport à l'anticorps murin CF12. Leur classement est globalement identique à celui des dix variants produits en expression transitoire et marque une chute surprenante de l'amplitude de l'effet potentialisant sur LH/CG représentant 24% à 3% de l'effet de CF12. Les variants H1L0 et 13VHB ont un effet potentialisant sur l'activité LH/CG représentant 24 et 20% respectivement de l'effet de CF12 et sont les plus efficients. Les quatre autres variants ont un effet très réduit allant de 6,35% à 4% de celui de CF12.

Globalement, les propriétés pharmacodynamiques des variants produits en lignée stable n'ont pas été modifiées à l'exception de H1L0 dont l'effet potentialisant sur l'activité FSH a baissé et dans une moindre mesure celui sur l'activité LH/CG.

En conclusion, l'humanisation de CF12 a conduit de façon surprenante à des variants fortement potentialisants de l'activité de la FSH et plus faiblement potentialisants de l'activité de la LH/CG, conduisant à une nouvelle molécule plus spécifique dans son action potentialisante sur la FSH. Ces nouvelles propriétés permettront de mieux cibler les cellules exprimant les récepteurs FSH (e.g. cellules de la granulosa dans l'ovaire, cellules de Sertoli dans le testicule) et par conséquent de mieux cibler et de mieux définir les indications thérapeutiques potentielles du futur médicament.

### Effet potentialisant sur l'activité FSH et LH du variant 13VHB-139VL produit dans sa lignée stable définitive.

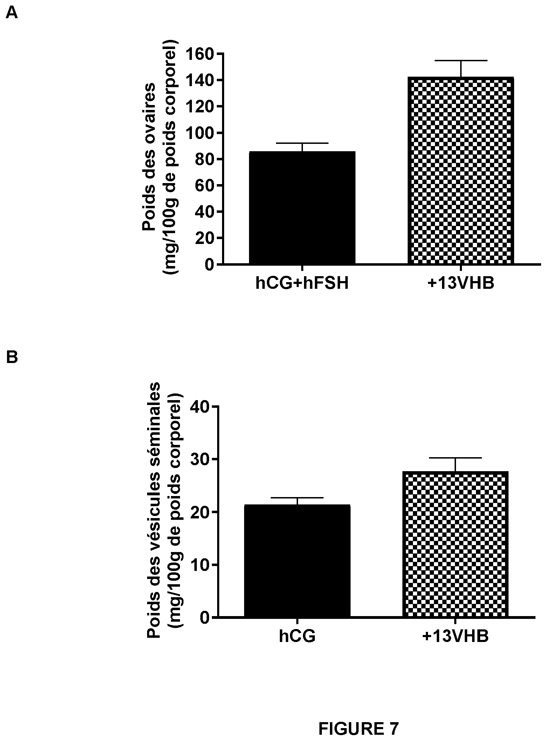

L'effet potentialisant du variant sélectionné comme candidat de premier rang pour le développement d'un médicament, 13VHB, produit dans sa lignée CHO monoclonale finale, a été vérifié et caractérisé sur l'activité de la FSH (Figure 7A) et de la LH/CG (Figure 7B). Les résultats ont indiqué que le produit final du variant 13VHB a un effet potentialisant sur la FSH du même ordre que CF12 avec un poids moyen des ovaires augmenté de 1,7 fois chez les rates traitées avec 13VHB + FSH/hCG par rapport aux rates traitées avec FSH/hCG seules (Figure 7A). Comparativement, l'effet potentialisant sur l'activité de la hCG est resté faible avec un poids moyen des vésicules séminales augmenté de 1,3 fois chez les rats traités avec 13VHB + hCG par rapport aux rats traités avec hCG seule (Figure 7B). Ces résultats ont confirmé que 13VHB est un variant à fort effet potentialisant de l'activité FSH et à plus faible effet potentialisant de l'activité LH/CG.

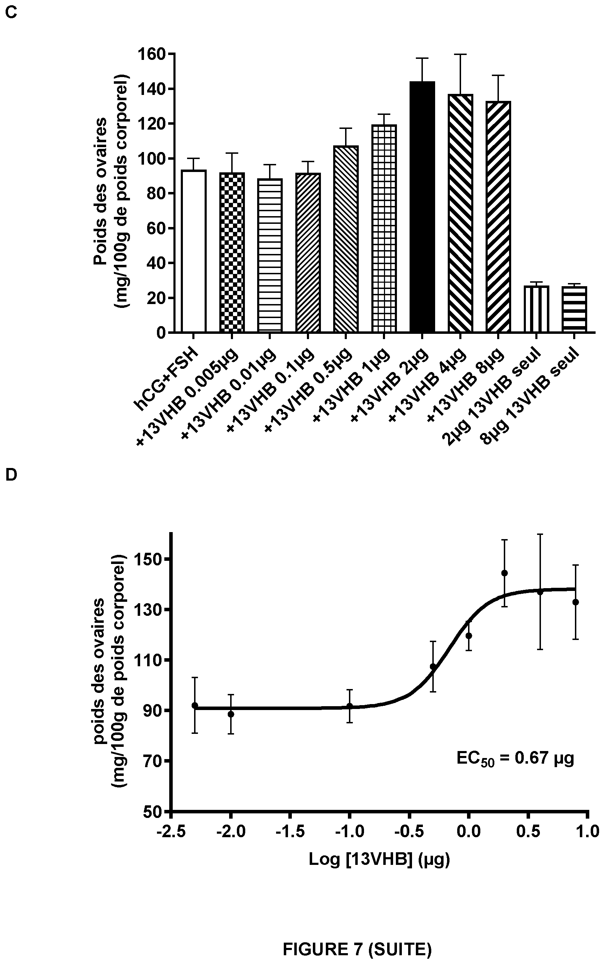

Afin de caractériser plus finement l'effet potentialisant sur FSH, une étude dose-réponse a été réalisée avec le même protocole de Steelman et Pohley [18] chez des rats femelles immatures traitées avec des doses croissantes du variant 13VHB, allant de 0,005 µg à 8 µg/injection/100 g de poids corporel, combinées à une dose fixe de FSH (Gonal-f) de 0.5 UI. Les résultats sont exprimés sous forme de données brutes (poids des ovaires en mg par 100 g de poids corporel) dans la Figure 7C et sous forme de courbe dose-réponse dans la Figure 7D. Ils démontrent clairement un effet dose du variant 13VHB dans le test biologique FSH avec une dose optimale de 2 µg/injection/100 g de poids corporel. La dose de 13VHB correspondant à 20% de l'activité (EC20) est de 0.486 µg/100 g soit 4.86 µg/injection/kg de poids corporel. Celle de l'EC50 est de 0.73 µg/100 g soit 7.3 µg/injection/kg de poids corporel.

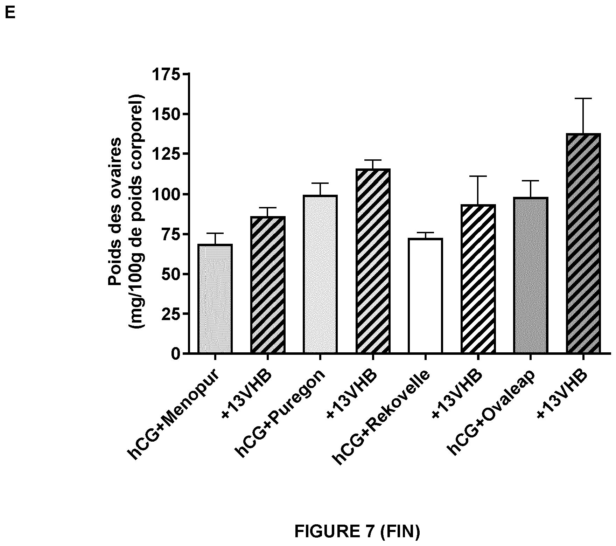

L'effet potentialisant de 13VH a été évalué sur d'autres préparations de FSH humaine utilisées en clinique humaine dans le cadre des techniques de PMA, comme Gonal-f, pour stimuler la croissance folliculaire et la maturation ovocytaire. Il s'agit des follitropines recombinantes alpha, Bemfola (Gedeon Richter, France) et Ovaleap (Theramex, France) de la Follitropine Béta Puregon et de la Follitropine Delta Rekovelle (Ferring, France). La hMG extraite Menopur (Ferring, France) composée de 50% de hFSH et 50% de hLH a également été testée. Chaque préparation hormonale a été injectée à la dose unique de 0,5 UI et 13VHB a été injecté à 2 µg/ injection/100 g de poids corporel. Les résultats sont illustrés Figure 7E et représentent les données brutes obtenues à l'issue des différents traitements avec ou sans 13VHB. Ils ont montré que le variant 13VHB exerce un effet potentialisant sur toutes les préparations de FSH testées avec une amplitude variable. Une augmentation respectivement de 146% et 140% du poids moyen des ovaires a été obtenue chez les femelles traitées avec 13VHB en association avec les follitropines alpha, Benfola^{®} et Ovaleap^{®}, par rapport aux femelles traitées avec la même hormone sans 13VHB. L'augmentation est de 130% avec la follitropine delta et la hMG (Menopur^{®}) par rapport aux femelles traitées sans 13VHB et de 120% avec la follitropine béta, (Puregon^{®}). Dans ces trois derniers cas, l'effet est moins élevé en raison probablement d'une différence d'activité spécifique propre à chacune de ces préparations de FSH.

### EXEMPLE 5 : EFFET POTENTIALISANT IN VIVO DES VARIANTS HUMANISES SELECTIONNES SUR LA FOLLICULOGENESE ET LA MATURATION OVOCYTAIRE CHEZ LE PRIMATE NON HUMAIN FEMELLE

L'effet potentialisant des cinq meilleurs variants humanisés sélectionnés *in vitro,* et, *in vivo* chez le rat immature femelle, a ensuite été évalué chez le singe Cynomolgus femelle (*Macaca fascicularis*) dans le cadre de traitements de stimulation ovarienne identiques à ceux utilisés chez la femme en vue d'une Fécondation In Vitro.

Trois protocoles différents utilisés en médecine de la reproduction chez la femme, ont ainsi été adaptés et mis au point pour une utilisation chez la guenon Macaca *fascicularis*:
- un protocole de type agoniste long, basé sur un prétraitement de deux semaines avec un agoniste du GnRH avant le traitement de stimulation ovarienne avec la FSH. Dans ce cas, le variant est utilisé en association avec un traitement FSH exogène.
- un protocole de type antagoniste court comprenant une stimulation de l'ovaire soit avec une injection quotidienne de FSH pendant 12 jours, soit avec une seule injection du variant à J1 du cycle. Dans ce cas, le variant a été utilisé seul sans aucune injection de FSH exogène et a été comparé au traitement classique avec FSH. Dans les deux cas, une injection quotidienne d'antagoniste du GnRH a été réalisée lorsque des follicules de taille préovulatoire ont été observés lors du suivi échographie.
- un protocole de type agoniste court (ou protocole agoniste « flare-up ») comprenant une injection d'agoniste du GnRH à J1 du cycle et une stimulation de l'ovaire soit avec une injection quotidienne de FSH pendant 9 jours à partir de J6, soit avec une seule injection du variant à J1 du cycle. Dans ce cas, le variant a été utilisé seul sans aucune injection de FSH exogène et comparé au traitement classique avec FSH exogène.

### Effet potentialisant des variants humanisés en association avec de la FSH exogène dans le cadre d'un Traitement Agoniste Long

Des femelles matures âgées d'au moins 36 mois ont été traitées avec un agoniste du GnRH à action prolongée, le Décapeptyl^{®} LP (1,5 mg par femelle) le premier jour des règles afin d'inhiber la sécrétion des gonadotrophines endogènes. Une deuxième injection de cet agoniste a été réalisée 15 à 21 jours après la première afin de couvrir tout le cycle de stimulation. Le traitement de stimulation ovarienne a débuté 15 à 21 jours après la première injection de Décapeptyl LP. Ce traitement comprenait :
- soit 1 injection de FSH humaine (Gonal-f^{®}, Merck KGaA, Allemagne) 37,5 UI par jour pendant 12 jours ;
- soit 1 injection de FSH humaine (Gonal-f^{®}, Merck KGaA, Allemagne) 37,5 UI par jour pendant 12 jours et une injection unique d'anticorps potentialisant à une dose de 20 µg/kg, le premier jour des règles, 20 minutes après l'injection de la Gonal-f. La dose de 20µg/kg correspond à une concentration de l'ordre de 0,3 µg/ml de sang si l'on admet un volume de sang de 65 ml/kg (University of Pittsburgh Policy for Regulating the Volume of Experimental Blood Sample Withdrawals in Laboratory Animals).

Des échographies trans-abdominales des ovaires et des prises de sang ont été effectuées au cours du traitement de stimulation ovarienne afin de suivre respectivement la croissance des follicules ovariens et la sécrétion des hormones de la stéroïdogenèse telles que l'œstradiol et la progestérone.

Les femelles ont ensuite reçu une injection de 2600 UI de hCG (Ovitrelle^{®}, Merck KGaA, Allemagne) 36 heures après la dernière injection de Gonal-f. La ponction folliculaire par laparotomie a été réalisée 36 heures après l'injection de hCG.

Après ouverture de l'abdomen, les ovaires ont été délicatement ramenés à la surface et les follicules ont été ponctionnés à l'aide d'une aiguille 25 G et d'une seringue 5 ml, et de milieu de culture G-MOPS PLUS (Vitrolife Référence 10130, Paris, France). Le liquide folliculaire ponctionné a été recueilli dans des tubes Falcon 14 ml à fond rond (Dutscher référence 352001, Brumath, France) contenant 200 µl d'héparine (Héparine Choay 25000 UI/5 ml, Cheplapharm Arzneimittel GmbH, Greifswald, Allemagne),

Les ovocytes ont ensuite été recherchés dans le liquide folliculaire sous loupe binoculaire puis décoronisés par traitement à la hyaluronidase (BioCare Europe référence 90101-5X1ML, Rome, Italie). Ils ont ensuite été observés au microscope pour déterminer leur stade de maturation évalué selon la présence ou non du premier globule polaire. Afin de confirmer leur stade de maturation et de contrôler leur qualité, les ovocytes ont ensuite été fixés dans une solution de paraformaldéhyde (PAF) 4% contenant 0,5% de Triton et 1% de BSA pendant 30 min à 37°C, à l'abri de la lumière. Les ovocytes ont ensuite été transférés dans une solution de PBS/BSA 1% et conservés à 4°C jusqu'à leur analyse par microscopie confocale après marquage de l'ADN et de la tubuline par immunocytochimie.

### 1/ Effet des variants 366VHC-139VL et H1L0

Une première série de neuf femelles réparties en 3 groupes de 3 animaux a permis d'évaluer l'effet des variants 366VHC-139VL et H1L0 en combinaison avec la FSH comparativement à un traitement avec FSH uniquement. Ces variants ont été produits en lignée CHO transitoire et formulés dans du PBS.

Afin d'évaluer la croissance folliculaire au cours des trois traitements, un suivi échographique des ovaires a été réalisé du premier jour de traitement jusqu'à la ponction folliculaire (J15) afin de mesurer la taille (en millimètres) et le nombre des follicules. Les résultats illustrés dans le tableau 3 représentent le nombre de follicules totaux et le nombre de gros follicules de taille supérieure à 3 mm observés à J13, jour de l'injection d'hCG. La taille supérieure à 3 mm définit un follicule préovulatoire. Cette taille est suffisante pour permettre une ponction.

**Tableau 3 : Nombre de follicules par ovaire, chez les guenons traitées avec FSH, FSH+H1L0 et FSH+366VHC (moyenne ± SEM). * p<0.05 Effet supérieur de FSH+CF12-H1L0 versus FSH (two-way ANOVA).**

| | Follicules totaux | Follicules > 3 mm | % follicules > 3 mm |
|---|---|---|---|
| FSH seule | 17,3 ± 3,7 | 7 ± 2,4 | 40% |
| + H1L0 | 25 ± 3,9 | 18,5 ± 5,2 (*) | 74% |
| + 366VHC | 24 ± 5,1 | 12,8 ± 3,3 | 53% |

Les traitements associant une injection d'anticorps avec le traitement FSH ont permis le recrutement d'un plus grand nombre de follicules totaux, détectables à l'échographie, avec plus de follicules de grosse taille. Dans les groupes traités avec FSH + H1L0 ou FSH + 366VHC, le nombre de follicules totaux a été augmenté de 1,5 fois. Le nombre de follicules de diamètre supérieur à 3 mm est 2,64 fois plus élevé (*, p<0.05 versus FSH) avec le traitement FSH+H1L0 et 1,8 fois avec le traitement FSH+366VHC (non-significatif). Ces résultats démontrent qu'un traitement FSH associant un variant humanisé a induit une meilleure stimulation ovarienne, un meilleur recrutement et une meilleure croissance folliculaire particulièrement avec H1L0 dont l'effet est significativement supérieur à celui de la FSH seule (p<0.05).

L'analyse des ponctions folliculaires a permis de compter le nombre d'ovocytes totaux, de trier les ovocytes immatures au stade de Vésicule Germinale (stade VG) et de Métaphase 1 (stade MI) et les ovocytes matures au stade Métaphase 2 de la méïose (stade MII) caractérisés par la présence d'un premier globule polaire. Les résultats présentés dans le tableau 4 illustrent le nombre moyen d'ovocytes totaux et d'ovocytes matures (MII) obtenus avec chacun des trois traitements.

**Tableau 4 : Variation du nombre moyen d'ovocytes totaux et d'ovocytes matures ponctionnés chez les guenons traitées avec FSH, FSH+H1L0 et FSH+366VHC-(moyenne ± SEM)**

| | Ovocytes totaux | Ovocytes matures | % ovocytes matures |
|---|---|---|---|
| FSH seule | 12,3 ± 2,2 | 6,7 ± 4,3 | 54% |
| + H1L0 | 28,3 ± 2,9 | 21 ± 3,1 | 74% |
| +366VHC | 29,3 ± 17,9 | 17,3 ± 10,2 | 59% |

Les guenons traitées avec la combinaison variant + FSH ont donné à la fois plus d'ovocytes totaux et plus d'ovocytes matures que le groupe traité avec de la FSH seule : 2,6 fois plus d'ovocytes matures avec le variant 366VHC et 3,15 fois plus avec le variant H1L0. Ces résultats démontrent que le traitement FSH en combinaison avec un variant potentialisant a induit une meilleure qualité ovocytaire et une meilleure maturation de l'ovocyte, particulièrement dans le cas du variant H1L0 où 74% des ovocytes ponctionnés étaient des ovocytes matures contre 54% avec la FSH seule.

Ce résultat est majeur dans la mesure où le taux de succès d'une Fécondation In Vitro est d'autant plus élevé que le nombre d'ovocytes matures (MII) collectés est important.

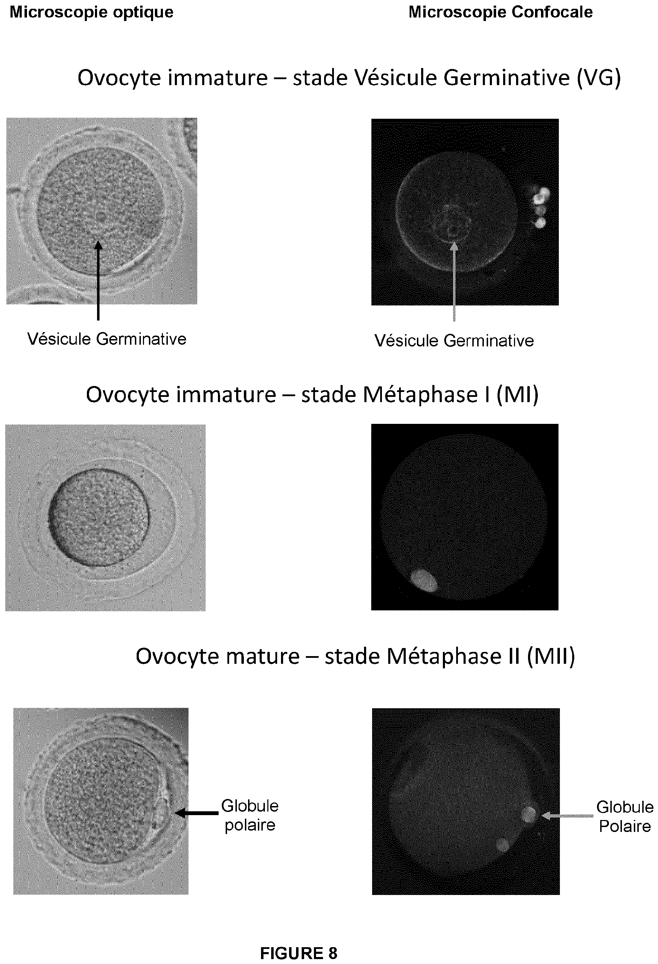

L'observation des ovocytes en microscopie confocale après marquage de l'ADN et de la Tubuline, a montré que la structure et la qualité des ovocytes issus d'un traitement associant un variant humanisé étaient tout à fait normales et identiques aux ovocytes issus d'un traitement standard avec FSH seule. La Figure 8 illustre les différents stades de maturation des ovocytes observés respectivement en microscopie optique et confocale : ovocytes immatures au stade Vésicule Germinative (VG) et au stade Métaphase 1 (MI) et ovocyte mature au stade Métaphase 2 de la méïose (MII) caractérisé par la présence du premier Globule Polaire (GP).

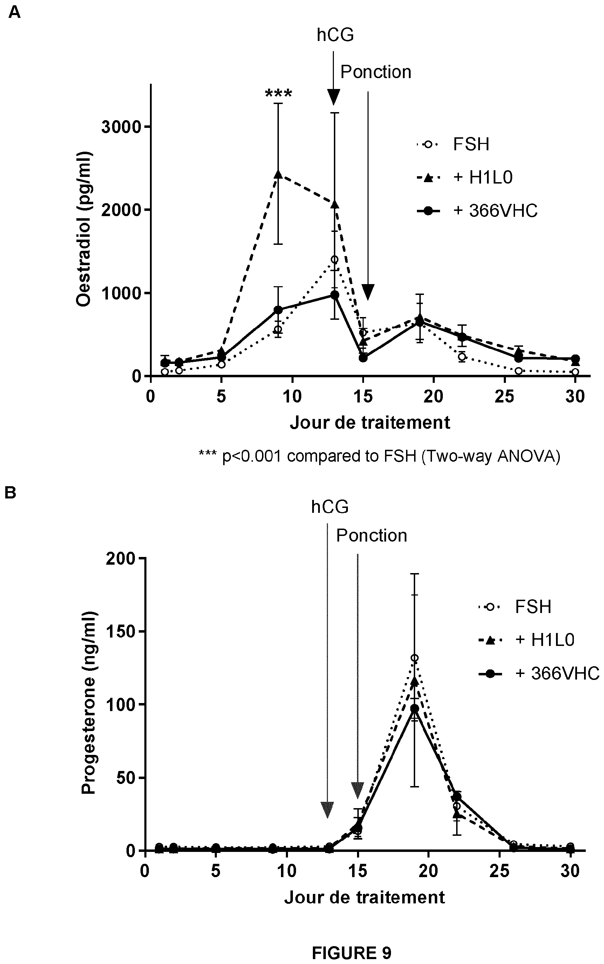

Afin de caractériser l'effet des variants sur la réponse stéroïdogène de l'ovaire, un suivi de la sécrétion d'oestradiol et de progestérone a été réalisé tout au long du cycle du début de la stimulation (J1) à la fin de la phase lutéale (J30). Les résultats sont présentés Figure 9. Le groupe de femelles traitées avec FSH+H1L0 a présenté une sécrétion d'oestradiol plus importante comparativement aux deux autres lots tout au long de la phase folliculaire (Figure 9A). A J9, la concentration en oestradiol est significativement supérieure chez les guenons traitées avec FSH+H1L0 comparativement aux femelles traitées avec FSH seule d'un facteur 4 (p<0.001) ou avec FSH+366VHC d'un facteur 3 (p<0.01) (Two way-ANOVA). Cette sécrétion d'oestradiol plus élevée est corrélée à la meilleure croissance folliculaire et à la meilleure maturation ovocytaire observée chez les femelles traitées avec FSH+H1L0. Il n'y a pas de différence significative entre le groupe FSH et FSH+366VHC.

Aucune différence dans la sécrétion de progestérone n'a été observée entre les trois traitements (Figure 9B).

### 2/ Effet des variants H3L0, 366VHB-139VL et 13VHB-139VL

Une deuxième série de traitements a été réalisée dans ce même protocole sur treize guenons afin d'évaluer l'effet potentialisant de trois autres variants humanisés : H3VH-L0VL (H3L0), 366VHB-139VL (366VHB) et 13VHB-139VL (13VHB). Ces variants ont été produits en lignée CHO stable. Ils étaient formulés et dilués dans du PBS.

Les guenons ont été réparties en 4 lots : 3 lots de 3 guenons traitées avec FSH seule, FSH+H3L0 ou FSH+366VHB et un lot de 4 guenons traitées avec FSH+13VHB.

L'effet potentialisant de ces 3 variants a été évalué dans un traitement de superovulation identique au précédent en utilisant les mêmes paramètres physiologiques pour suivre la stimulation ovarienne. L'effet des variants a été comparé à celui d'un traitement standard avec de la FSH seule.

Le recrutement et la croissance folliculaire a été suivie par échographie des ovaires du premier jour de traitement jusqu'à la ponction folliculaire (J15). Le tableau 5 illustre la taille et le nombre des follicules observés dans les 4 groupes.

**Tableau 5 : taille et nombre des follicules par ovaire, chez les guenons traitées avec FSH, FSH+H3L0, FSH+366VHB, FSH+13VHB (moyenne ± SEM).**

| | Follicules totaux | Follicules > 4mm | % follicules > 4mm |
|---|---|---|---|
| FSH seule | 25,33 ± 3,1 | 6 ± 3,1 | 23,7% |
| + H3L0 | 39 ± 12,4 | 12 ± 14,7 | 30,8% |
| + 366VHB | 45 ± 10,4 | 25,33 ± 3,8 | 56,3% |
| + 13VHB | 79,25 ± 35,4 | 43,25 ± 21,11 | 54,6% |

Les résultats indiquent que la stimulation ovarienne a été plus importante avec les traitements associant FSH+variants par rapport à la FSH seule, particulièrement avec le traitement FSH+366VHB et FSH+13VHB. Le nombre de follicules de diamètre supérieur à 4 mm est 7,2 fois plus élevé dans le lot FSH+13VHB par rapport au lot FSH et 4,2 fois plus élevé que dans le lot FSH+366VHB.

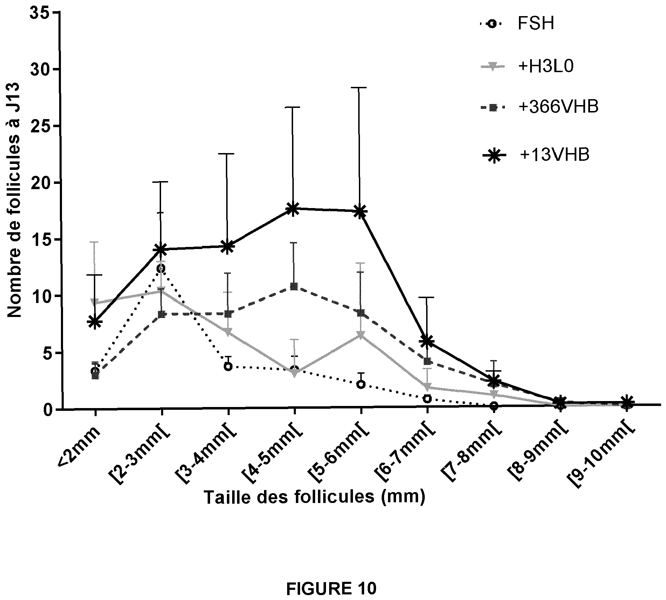

Le nombre de follicules mesurés à J13 et classés par catégorie de taille est illustré Figure 10, pour chacun des lots de femelles. Le lot traité avec FSH+13VHB a donné le plus grand nombre de follicules en croissance et les plus grandes tailles de follicules. Ainsi, 18 follicules de 4-5mm ont été observés en moyenne dans le groupe FSH+13VHB contre 10 dans le groupe FSH+366VHB et 3 dans les groupes FSH seule et FSH+H3L0. Ceci traduit une meilleure croissance folliculaire et une meilleure différenciation.

La ponction des follicules a été réalisée à J15 du traitement et a montré des différences importantes entre les traitements.

Le nombre d'ovocytes totaux ponctionnés et parmi eux le nombre d'ovocytes matures observés (Stade Métaphase II) pour chaque traitement (FSH seule ou en combinaison avec une injection d'anticorps à J1) sont résumés dans le tableau 6.

**Tableau 6 : Nombre d'ovocytes totaux et d'ovocytes matures (MII) obtenus dans chaque groupe expérimental. Les résultats sont exprimés en moyenne ± SEM.**

| | Ovocytes totaux | Ovocytes matures | % de maturation |
|---|---|---|---|
| FSH seule | 17,33 ± 7,68 | 8,33 ± 2,66 | 48% |
| + 366VHB | 16 ± 1,52 | 11,33 ± 3,7 | 70,8% |
| + H3L0 | 35,33 ± 6,56 | 20,33 ± 5,8 | 52,5% |
| + 13VHB | 40,5 ± 12,91 | 25,5 ± 8,37 | 65,3% |

Les résultats montrent que les femelles traitées avec FSH en association avec un variant humanisé ont eu un nombre d'ovocytes total et un nombre d'ovocytes matures plus élevés. Les femelles traitées avec le variant 13VHB ont donné les meilleurs résultats avec 3 fois plus d'ovocytes matures au stade MII que les femelles traitées avec FSH seule : 25,5 MII contre 8,33 respectivement. Le traitement avec le variant H3L0 a donné 2,4 fois plus d'ovocytes matures qu'avec le traitement avec la FSH seule : 20,33 MII contre 8,33 respectivement. Le traitement avec 13VHB a donc permis d'obtenir la meilleure maturation ovocytaire aboutissant au taux le plus élevé d'ovocytes matures, développés au stade Métaphase II de la méiose. Ce résultat est cliniquement très important car le taux de réussite d'une FIV dépend du nombre d'ovocytes matures collectés, seuls les ovocytes au stade MII étant aptes à être fécondés.

Une observation des ovocytes en microscopie confocale après marquage de l'ADN et de la Tubuline, a montré là encore que la structure et la qualité des ovocytes issus d'un traitement associant les variants 366VHB, H3L0 ou 13VHB sont tout à fait normales et identiques aux ovocytes issus d'un traitement standard avec FSH seule.

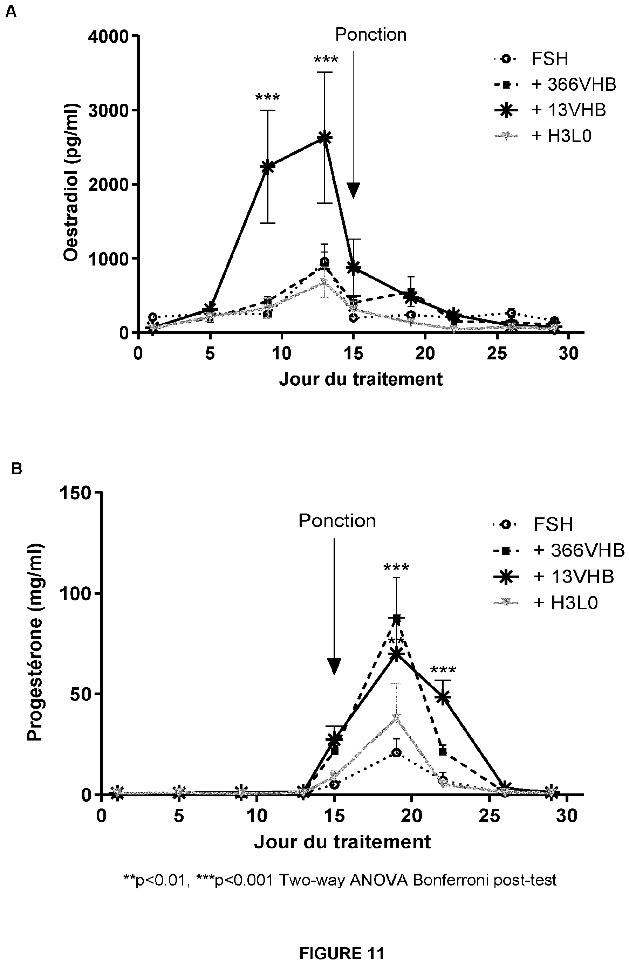

En complément, l'effet des trois variants en association avec le traitement FSH a été caractérisé également sur la réponse stéroïdogène de l'ovaire au cours du cycle de traitement. Un suivi de la sécrétion d'oestradiol et de progestérone a été mesuré et comparé dans les quatre groupes de femelles traitées avec FSH, FSH+366VHB, FSH+H3L0, FSH+13VHB (Figure 11). Les résultats illustrés sur la Figure 11A montrent de façon très significative que les femelles traitées avec le variant 13VHB ont eu une sécrétion d'oestradiol trois fois plus importante qu'avec les autres variants ou la FSH seule (p<0.001, Two-way ANOVA). Cette plus forte sécrétion d'oestradiol est corrélée à la plus forte croissance folliculaire observée en échographie et à la meilleure maturation des ovocytes ayant conduit à un nombre d'ovocytes matures 3 fois plus important avec le variant 13VHB. Ces résultats indiquent clairement que le variant 13VHB a permis une meilleure croissance folliculaire et une meilleure maturation des ovocytes.

Les résultats illustrés sur la Figure 11B, montrent de façon très significative que la sécrétion de progestérone est également plus importante chez les guenons ayant reçu le traitement FSH associant les variants 13VHB (p<0.01) et 366VHB (p<0.001, Two-way ANOVA). Cette meilleure sécrétion de progestérone traduit une meilleure qualité des corps jaunes, hormone indispensable au bon développement embryonnaire précoce et à la prévention d'une perte embryonnaire précoce.

En conclusion, l'ensemble des résultats obtenus avec les cinq meilleurs variants humanisés dans ce premier protocole chez le primate non humain, a permis de sélectionner le variant 13VHB comme candidat de premier rang pour le développement du médicament. Le variant H3L0 a été retenu comme candidat de deuxième rang au vu du nombre plus élevé d'ovocytes matures obtenus par rapport au traitement FSH seule ou FSH en association avec le variant 366VHB.

### Effet du variant 13VHB administré seul, sans FSH exogène, dans le cadre d'un protocole de Traitement Antagoniste Court.

Le protocole Antagoniste Court est très utilisé chez la femme en vue d'une FIV. Il a été adapté et utilisé chez le singe Cynomolgus pour étudier l'effet du variant 13VHB injecté seul, sur l'activité de la FSH endogène.

Dans ce cas, les femelles n'ont reçu aucune injection de FSH humaine et n'ont été traitées qu'avec l'anticorps humanisé seul.

Pour cela, des femelles âgées d'au moins 36 mois ont été traitées :
- soit avec 1 seule injection du variant 13VHB à une dose de 20 µg/kg, le premier ou le deuxième jour des règles. Cette dose correspond à une concentration de l'ordre de 0,3 µg/ml de sang si l'on admet un volume de sang de 65 ml/kg (University of Pittsburgh Policy for Regulating the Volume of Experimental Blood Sample Withdrawals in Laboratory Animals) ;
- soit avec 1 injection par jour de 37,5 UI de FSH humaine (Gonal-f^{®}, Merck Serono, France) pendant 12 jours.

Chaque lot expérimental a compris deux guenons.

Des échographies trans-abdominales des ovaires ont été effectuées au cours du traitement de stimulation ovarienne afin de suivre la croissance des follicules ovariens.

L'ovulation naturelle a été inhibée par injection d'un Antagoniste du GnRH (Cétrotide^{®}, Merck Serono, France) lorsque les follicules en croissance ont atteint un diamètre moyen de 3 mm, ou au plus tard au 11^{ème} jour du protocole de stimulation ovarienne. Les femelles ont ensuite reçu une injection de 2600 UI de hCG (Ovitrelle^{®}, Merck Serono, France) 36 heures après la dernière injection de Gonal-f.

La ponction folliculaire par laparotomie a été réalisée 36 heures après l'injection de hCG, selon le mode opératoire décrit ci-dessus dans le protocole Agoniste Long. Le liquide folliculaire ponctionné a été recueilli dans des tubes Falcon 14 ml à fond rond (Dutscher référence 352001, Brumath, France) contenant 20 µl d'héparine (Héparine Choay 25000 UI/5 ml, Cheplapharm Arzneimittel GmbH, Greifswald, Allemagne). Les ovocytes ont été recherchés dans le liquide folliculaire sous loupe binoculaire puis décoronisés par traitement à la hyaluronidase selon le même mode opératoire que ci-dessus puis observés au microscope pour déterminer leur stade de maturation. Une partie des ovocytes a été fixée dans une solution de paraformaldéhyde (PAF) 4% contenant 0,5% de Triton et 1% de BSA pendant 30 min à 37°C, puis transférée dans une solution de PBS/BSA 1% et conservée à 4°C jusqu'à analyse. L'autre partie a été utilisée pour une étude de parthénogénèse.

Les résultats obtenus dans chaque lot expérimental (n=2 guenons) sont présentés dans le tableau 7. Ils indiquent le nombre moyen d'ovocytes totaux et d'ovocytes matures (MII) obtenus par femelle lors de la ponction réalisée à J15 du traitement.

**Tableau 7 : Variation du nombre d'ovocytes totaux et d'ovocytes matures ponctionnés chez les guenons traitées soit avec FSH, soit avec le variant 13VHB injecté seul (moyenne ± SEM) en protocole antagoniste court.**

| Traitement | Nombre d'ovocytes totaux | Nombre d'ovocytes matures |
|---|---|---|
| FSH seule | 46 ± 7,07 | 20 ± 16,97 |
| 13VHB seul | 43,5 ± 17,68 | 18.5 ± 9,19 |

Les résultats démontrent que la réponse ovarienne obtenue suite au traitement avec 13VHB injecté en une seule fois à 20 µg/kg est très proche de celle obtenue avec un traitement comprenant 12 injections de FSH. En effet, 18,5 ovocytes matures ont été obtenus en moyenne dans le lot 13VHB contre 20 dans le lot FSH. Le nombre moyen d'ovocytes totaux a été de 43,5 dans le lot 13VHB contre 46 dans le lot FSH.

Ces résultats sont importants car ils indiquent que le variant 13VHB utilisé en monothérapie, et administré en une seule injection en début de cycle, est capable de potentialiser l'activité de la FSH endogène et d'induire une stimulation de l'ovaire suffisante pour aboutir à la production de plusieurs ovocytes matures de façon identique et aussi efficace qu'un traitement comprenant 12 injections quotidiennes de FSH exogène.

Ceci permet d'envisager la possibilité d'utiliser un variant humanisé selon l'invention, seul, en monothérapie, chez les patients femme ou homme souffrant d'infertilité ou d'hypofertilité et ayant un niveau circulant de FSH suffisant. Dans ce cas, le traitement avec un variant humanisé selon l'invention, e.g. 13VHB, serait utilisé en substitution d'un traitement comportant de multiples injections de FSH exogène.

### Effet du variant 13VHB administré seul, sans FSH exogène, dans le cadre d'un protocole de Traitement avec Agoniste Court

L'effet du variant 13VHB administré seul, sur la FSH endogène, a été évalué également dans un autre protocole utilisé chez la femme en vue d'une FIV. Il s'agit d'un protocole de type Agoniste court (protocole agoniste "flare-up") qui a été adapté pour une utilisation chez la guenon cynomolgus. Dans ce protocole, deux guenons âgées d'au moins 36 mois ont reçu le premier jour des règles une injection d'agoniste du GnRH à action prolongée (Décapeptyl L.P., Ipsen Pharma, France). Puis,
- l'une a reçu 1 injection unique du variant 13VHB à la dose de 20 µg/kg le jour de l'injection de l'agoniste du GnRH ;
- l'autre a reçu 1 injection de FSH humaine (Gonal-f^{®}, Merck Serono, France) à 37,5 UI par jour pendant 9 jours. La première injection de FSH a été réalisée 72 heures après l'injection de l'agoniste du GnRH.

Le traitement avec le variant seul a donc été extrêmement simplifié puisqu'il ne comprend que deux injections le premier jour des règles (1 injection d'anticorps + 1 injection de l'agoniste) comparativement à 10 injections nécessaires dans le traitement avec la FSH (1 injection d'agoniste + 9 injections de FSH).

Les femelles ont ensuite reçu une injection de 2600 UI de hCG (Ovitrelle^{®}, Merck Serono, France) le treizième jour du traitement. La ponction folliculaire par laparotomie a été réalisée 36 heures après l'injection de hCG. Après ouverture de l'abdomen, les ovaires ont été délicatement ramenés à la surface et les follicules ont été ponctionnés à l'aide d'une aiguille 25G et d'une seringue 5 ml, et de milieu de culture G-MOPS PLUS (Vitrolife Référence 10130, Paris, France). Le liquide folliculaire ponctionné a été recueilli dans des tubes Falcon 14 ml à fond rond (Dutscher référence 352001, Brumath, France) contenant 200 µl d'héparine (Héparine Choay 25000 UI/5 ml, Cheplapharm Arzneimittel GmbH, Greifswald, Allemagne), et transporté jusqu'au laboratoire dans une valisette de transport à 37°C (Biotherm INC-RB1 CRYOLOGIC : incubateur transportable pour tubes, Laboratoires JCD, La Mulatière, France).

Tout au long du traitement de stimulation ovarienne, des échographies transabdominales des ovaires et des prises de sang ont été effectuées afin de suivre respectivement la croissance des follicules et la sécrétion des hormones telles que l'œstradiol et la progestérone.

Une fois au laboratoire, les ovocytes ont été recherchés dans le liquide folliculaire sous loupe binoculaire. Pour cela, les tubes Falcon de 14 ml contenant le liquide folliculaire ont été vidés dans des boites de pétri Nunc pour culture cellulaire (référence 055061, Dominique Dutscher, Brumath, France). Les ovocytes ainsi identifiés ont été décoronisés par traitement à la hyaluronidase (BioCare Europe référence 90101-5X1ML, Rome, Italie), puis observés au microscope pour déterminer leur stade de maturation.

Les résultats obtenus dans chaque lot expérimental (n=1 guenon) sont résumés dans le tableau 8. Ils indiquent le nombre d'ovocytes totaux et d'ovocytes matures (MII) obtenus par femelle lors de la ponction folliculaire réalisée à J15.

**Tableau 8: Nombre d'ovocytes totaux et d'ovocytes matures ponctionnés chez les guenons traitées soit avec FSH soit avec le variant 13VHB seul dans un protocole agoniste "flare-up" (n=1 par groupe).**

| Traitement | Nombre d'ovocytes totaux | Nombre d'ovocytes matures |
|---|---|---|
| FSH seule | 42 | 20 |
| 13VHBseul | 94 | 15 |

Le traitement avec le variant 13VHB injecté une seule fois à 20 µg/kg a donné 15 ovocytes matures ce qui représente un quota suffisant pour faire une FIV. Ce résultat est peu différent des 20 matures obtenus chez la guenon ayant reçu 9 injections de FSH. Seul le nombre d'ovocytes totaux est très différent entre les deux femelles.

Les résultats obtenus avec le variant 13VHB seul dans ce deuxième type de protocole indiquent également qu'il est capable de potentialiser l'activité de la FSH endogène et d'induire une stimulation de l'ovaire conduisant à une production significative d'ovocytes matures, suffisante pour envisager de pouvoir faire une FIV.

Ils confirment là encore la possibilité d'envisager l'utilisation d'un variant humanisé selon l'invention, seul, en monothérapie, e.g. 13VHB, chez la femme et chez l'homme ayant des niveaux circulants de FSH suffisants.

La totalité des ovocytes matures obtenus dans les deux protocoles testant l'effet de l'anticorps 13VHB seul, a ensuite été utilisée dans une étude de parthénogénèse.

### Etude de parthénogénèse sur des ovocytes matures ponctionnés chez des guenons traitées avec FSH seule ou avec le variant 13VHB seul.

La capacité des ovocytes matures à se diviser par parthénogénèse a été évaluée avec des ovocytes de stade MII ponctionnés sur des guenons traitées soit avec FSH seule, soit avec 13VHB seul en monothérapie, dans le cas d'un protocole antagoniste ou d'un protocole agoniste court "flare-up".

Pour cela, les ovocytes ont été incubés 2 minutes dans une solution de ionomycine 5 µM (Merck, France), suivi d'un lavage de 5 minutes dans du milieu G-MOPS (Vitrolife Référence 10130, Paris, France). Les ovocytes ont ensuite été incubés pendant 4 heures dans une solution de 6-Dimethylaminopurine (6-DMAP) 2 mM (Merck, France), suivi d'un lavage de 5 minutes dans du milieu G-TL (Vitrolife Référence 10145, Paris, France). Enfin, les ovocytes ont été cultivés pendant 5 jours dans une goutte de 50 µl de milieu G-TL recouverte de 120 µl d'huile minérale, dans un incubateur à 37°C 6% CO₂ 5% O₂ 89% N₂. A l'issue des 5 jours de culture, les ovocytes ont été analysés par microscopie optique afin d'observer la présence ou non de divisions cellulaires.

Les résultats obtenus au bout de 5 jours de culture après le traitement d'induction de la parthénogénèse sont illustrés dans le tableau 9.

**Tableau 9 : Pourcentage d'ovocytes matures divisés par parthénogénèse après 5 jours de culture, dans le cas de guenons traitées avec FSH ou avec 13VHB seul.**

| Traitement | Femelles (n=) | % d'ovocytes divisés après 5 jours de culture |
|---|---|---|
| FSH | 3 | 90% |
| 13VHB-139VL | 3 | 89% |

Les résultats montrent que la plupart des ovocytes traités présentent des figures de parthénogénèse : 89% se sont divisés dans le cas des guenons traitées avec 13VHB et 90% dans le cas des guenons traitées avec FSH. Seuls 11% et 10% des ovocytes dans les deux traitements respectifs ne se sont pas divisés.

Il n'y a donc pas de différence entre le traitement FSH et 13VHB : dans les deux cas, les ovocytes matures collectés ont la même aptitude à se diviser par parthénogénèse, témoignant d'une même qualité ovocytaire avec le traitement 13VHB seul comparativement au traitement standard avec FSH seule.

### EXEMPLE 6 : EFFET POTENTIALISANT IN VIVO DE L'ANTICORPS HUMANISE 13VHB-139VL (SEQ ID NO : 9 et SEQ ID NO : 16) SUR LA SPERMATOGENESE CHEZ LE RAT MALE ADULTE

L'effet potentialisant de l'anticorps humanisé 13VHB-139VL (SEQ ID NO : 9 et SEQ ID NO : 16) a été étudié chez le mâle afin d'évaluer son impact sur la spermatogénèse contrôlée par la FSH et la LH. La FSH est impliquée dans les premiers stades de la spermatogénèse et dans la stimulation des cellules de Sertoli, cellules nourricières des cellules germinales indispensables à leur maturation. La LH est impliquée dans le développement des derniers stades de différenciation de la spermatogénèse (spermatides et spermatozoïdes) et dans la stimulation des cellules de Leydig sécrétant la testostérone.

L'effet potentialisant de l'anticorps 13VHB sur la spermatogénèse a été évalué *in vivo* dans un modèle expérimental de rat mâle adulte prétraité avec un inhibiteur du GnRH afin d'induire un hypogonadisme hypogonadotrope provoquant une inhibition totale de la spermatogénèse.

Pour cela des rats Wistar Han âgés de 12 semaines (Charles River, L'Arbresle, France) ont été prétraités avec un antagoniste du GnRH (Firmagon^{®} LP, Ferring, France), à raison d'une injection de 2 mg/kg le premier jour du traitement puis d'une deuxième injection 6 semaines plus tard.

Le traitement hormonal avec ou sans variant 13VHB a commencé une semaine après la deuxième injection de Firmagon, date à laquelle la spermatogénèse est totalement inhibée. Les animaux ont ainsi été traités pendant huit semaines, durée correspondant à un cycle de spermatogénèse chez le Rat (54 à 56 jours). Les animaux traités avec hormones seules ont reçu soit un mélange de hCG (Ovitrelle^{®}, Merck) à 2,5 Ul/kg injecté 5 fois par semaine et de hMG (Menopur^{®}, Ferring) à la dose de 25 Ul/kg injecté 3 fois par semaine (lot Hormone X1), soit le même mélange avec une double dose d'hormones (hCG 5 Ul/kg injecté 5 fois par semaine et Menopur^{®} 50 Ul/kg injecté 3 fois par semaine) (lot Hormone X2). Menopur^{®} est une hMG (human Menoposal Gonadotropin) composée d'un mélange FSH+LH en quantité équivalente (50% chacune).

Dans un troisième lot, les animaux ont été traités avec le mélange hormone X1 et le variant 13VHB (lot HormoneX1+13VHB). Le variant a été injecté à la dose de 20 µg/kg 3 fois par semaine. Cette dose correspond à une concentration de l'ordre de 0,3 µg/ml de sang si l'on admet un volume de sang de 65ml/kg chez le rat (University of Pittsburgh Policy for Regulating the Volume of Experimental Blood Sample Withdrawals in Laboratory Animals). Le variant 13VHB était formulé dans du PBS stérile.

Un lot contrôle, traité uniquement avec l'antagoniste, n'a reçu que du sérum physiologique, sans aucun traitement hormonal.

L'étude a donc porté sur six lots expérimentaux comprenant chacun quatre animaux :
- un lot contrôle (WT) non prétraité avec le Firmagon et non stimulé ;
- un lot « contrôle d'inhibition » traité avec Firmagon uniquement ;
- un lot traité avec Firmagon puis du sérum physiologique (saline) ;
- un lot traité avec Firmagon puis avec HormonesX1 ;
- un lot traité avec Firmagon puis avec HormonesX1 + 13VHB ;
- un lot traité avec Firmagon puis avec HormonesX2.

A la fin du traitement, les animaux ont été sacrifiés et du sang a été prélevé en intracardiaque sur tubes héparinés, et centrifugés. Les testicules et les épididymes ont été prélevés et pesés.

Les testicules ont ensuite été broyés dans du milieu Ham's F12 complémenté en sodium pyruvate 1% et hepes 1%, puis soniqués. Le nombre de têtes de spermatides a ensuite été compté au microscope optique afin d'évaluer la réserve spermatique. Un testicule pour chacun des lots expérimentaux a été fixé dans une solution de Formaline à 10% et inclus dans de la paraffine pour une analyse histologique. Des coupes fines de 5 µm d'épaisseur ont été réalisées dans l'axe transversal du testicule et colorées à l'acide périodique de Schiff (PAS) et à l'hématoxyline-éosine (H&E). L'examen des lames colorées a été effectué à l'aide du logiciel NDP Viewer. Les coupes de testicules ont été évaluées conformément aux guidelines pour l'évaluation histopathologique de l'OCDE (Partie 2 : Système reproducteur masculin).

Les épididymes ont été découpés en fines sections et incubés à température ambiante au moins 4 heures dans du milieu Ham's F12 complémenté en sodium pyruvate 1% et hepes 1%. Les préparations ont ensuite été récupérées, filtrées et les spermatozoïdes ont été comptés au microscope optique et par cytométrie en flux.

### Effet potentialisant du variant 13VHB sur la reprise de la spermatogénèse.

L'effet potentialisant du variant 13VHB a été évalué après 8 semaines de traitement, en comparant les réponses des animaux ayant reçu un traitement hormonal seul à celles des mâles ayant reçu un traitement hormonal en association avec l'anticorps.

La réponse testiculaire a été évaluée selon trois critères :
- le poids des testicules qui est corrélé à l'intensité de la spermatogénèse ;
- la réserve spermatique définie par le nombre de spermatides dans les testicules ;
- le nombre des spermatozoïdes collectés dans les épididymes.

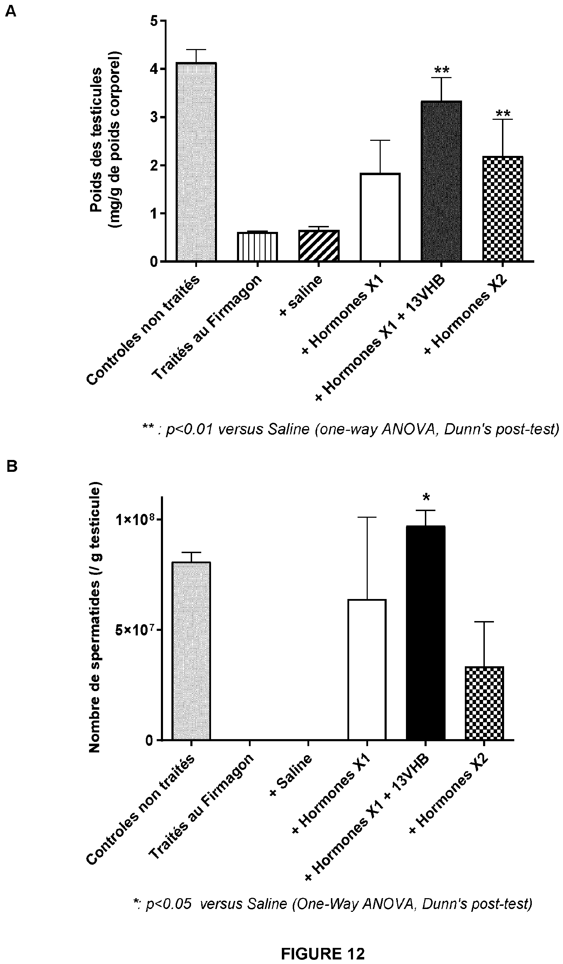

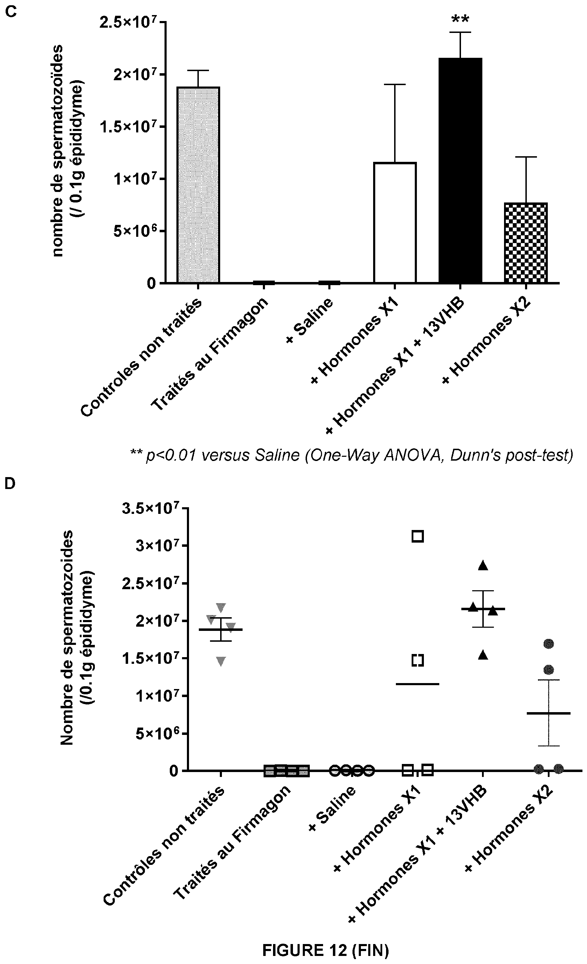

Les résultats obtenus à l'issue des 8 semaines de traitement sont illustrés sur la Figure 12. Ils montrent tout d'abord que l'inhibition induite par le prétraitement avec le Firmagon a été totale avec un poids testiculaire résiduel réduit de 8 fois par rapport au lot contrôle (WT) (Figure 12A), une absence totale de spermatides (Figure 12B) et une absence totale de spermatozoïdes épididymaires (Figure 12C). Les animaux sont totalement azoospermiques. Il en est de même pour le lot traité avec Firmagon + saline dont la spermatogénèse est restée totalement inhibée 8 semaines après le début des traitements de stimulation.

L'effet des différents traitements sur le poids des testicules est illustré Figure 12A. On observe que les animaux ayant reçu le traitement Hormones X1+le variant 13VHB ont un poids testiculaire 1,8 fois plus élevé que ceux ayant reçu le traitement Hormones X1 seul (3,13 mg/g de poids corporel versus 1,83 respectivement). A l'inverse, le fait de doubler les doses d'hormones (Hormones X2) n'a pas amélioré significativement l'augmentation du poids testiculaire (2,19 mg/g poids corporel versus 1,83). L'anticorps 13VHB a donc permis une augmentation beaucoup plus importante du poids testiculaire par rapport à un traitement hormonal seul même avec une double dose d'hormones.

L'effet sur la réserve spermatique est illustré Figure 12B. Les résultats montrent que le nombre de spermatides est de 6,3.10⁷ avec le traitement hormones X1 et de 3,4.10⁷ avec Hormones X2. Là encore, doubler la dose des hormones n'a pas amélioré la reprise de la spermatogénèse par rapport à Hormones X1. A l'inverse, les animaux traités avec le variant 13VHB en association avec Hormones X1 ont un nombre de spermatides 1,6 fois plus élevé que ceux du lot Hormones X1 seules (9,73.10⁷ spermatides/g de testicules contre 6,39.10⁷ respectivement) et 3 fois plus élevé par rapport au lot Hormones X2 (9,73.10⁷ spermatides/g de testicules contre 3,35.10⁷). De plus, en un seul cycle de spermatogénèse, l'effet de 13VHB a permis une restauration complète de la spermatogénèse avec un nombre de spermatides revenu à un niveau normal, égal à celui du lot contrôle WT sans traitement Firmagon (9,7.10⁷ et 8,1.10⁷ spermatides/g de testicules respectivement). Ceci démontre que le traitement associant l'anticorps a été plus efficace et a été le seul à permettre une reprise complète de la spermatogénèse en un seul cycle de spermatogénèse.

Les résultats concernant le nombre de spermatozoïdes épididymaires obtenu à l'issue des 8 semaines des différents traitements sont illustrés Figure 12C. On observe que le nombre de spermatozoïdes épididymaires est deux fois plus élevé dans le lot 13VHB+Hormones X1 comparativement au lot Hormones X1 : 1,15.10⁷ et 2,16.10⁷ spermatozoïdes par 0,1 g d'épididyme respectivement. Dès 8 semaines (un seul cycle de spermatogénèse) le traitement associant 13VHB + Hormones X1 a donc permis un retour à un niveau normal de spermatozoïdes épididymaires identique à celui des animaux contrôles non traités (WT) : 2,15.10⁷ *versus* 1,8.10⁷ spermatozoïdes par 0,1 g d'épididyme respectivement. A l'inverse, le doublement des doses d'hormones (HormonesX2) n'a eu aucun effet améliorateur sur l'augmentation du nombre de spermatozoïdes par rapport au traitement Hormones X1, démontrant que le traitement associant le variant 13VHB apporte une plus grande efficacité qu'un traitement hormonal standard, même à double dose.

La Figure 12D est une représentation en dot-plot où chaque point représente le nombre de spermatozoïdes comptés par animal dans chaque lot. On observe que tous les animaux du lot associant le variant 13VHB ont répondu au traitement et présentent tous une reprise totale de la spermatogénèse avec une valeur médiane égale à celle du lot contrôle WT non prétraité au Firmagon. A l'inverse, les lots traités avec hormones seules X1 ou X2 ont présenté une forte variabilité individuelle de réponse avec une valeur médiane très inférieure à celle du lot associant 13VHB.

En conclusion, les trois paramètres physiologiques analysés démontrent que l'effet potentialisant du variant 13VHB associé au traitement hormonal (Hormone X1) a permis une reprise et une stimulation de la spermatogénèse beaucoup plus intense qu'un traitement hormonal seul, se traduisant par le retour à un niveau de spermatogénèse normal au bout de huit semaines de traitement, c'est-à-dire d'un seul cycle de spermatogénèse. Chez des animaux azoospermiques, en un cycle de spermatogénèse, il a permis le retour à un niveau normal du nombre de spermatides et de spermatozoïdes. Ce nouveau traitement apporte une meilleure efficacité et permettrait par conséquent un temps de traitement plus court chez l'homme.

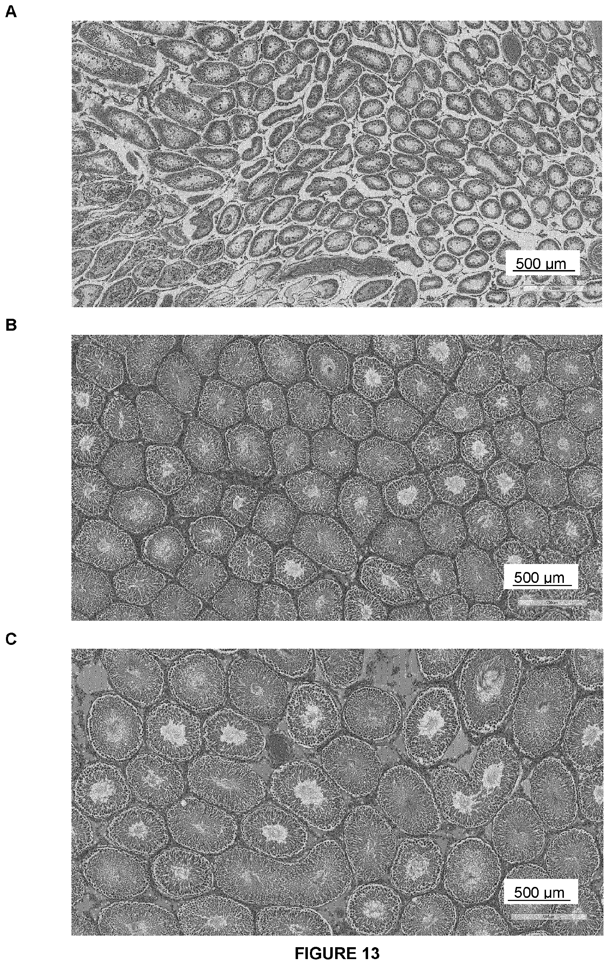

Cette étude a été complétée par une analyse histologique des coupes de testicules provenant de chacun des lots expérimentaux. Trois exemples représentatifs sont illustrés Figure 13.

Le testicule issu du lot contrôle Firmagon (Figure 13A), est nettement atrophique avec des tubes séminifères de petite taille et un tissu interstitiel réduit et déstructuré. Les tubes séminifères sont pauvres en cellules germinales et dépourvus de toute spermatide allongée reflétant une spermatogénèse totalement défectueuse.

L'histologie des testicules issus du lot traité avec hormones X1 (Figure 13B) et du lot traité avec hormones X1+13VHB (Figure 13C) montre dans les deux cas une structure plus dense. On remarque que la taille des tubes séminifères est plus importante (diamètre et surface) dans le testicule du lot hormones X1+13VHB attestant d'une spermatogénèse plus intense par rapport au lot hormones seules X1. Ceci est corrélé avec l'obtention d'un poids moyen des testicules plus élevé dans le lot hormones X1 + 13VHB. De même la présence de stades précoces et tardifs de spermatogenèse à proportion normale a été observée dans les deux testicules mais l'abondance des spermatides allongées est supérieure dans le testicule du lot « hormones+13VHB » traduisant là encore une plus forte reprise de la spermatogénèse.

L'étude des coupes histologiques des testicules a été complétée par une analyse d'images afin de mesurer le diamètre moyen des tubes séminifères (en µm) et l'aire moyenne des tubes séminifères (en µm²) dans chaque groupe. Sur chaque coupe histologique, 100 sections circulaires de tubes séminifères ont ainsi été analysées. Les résultats sont illustrés dans le tableau 10 ci-dessous.

**Tableau 10 : Valeurs moyennes des diamètres et des aires des sections de tubes séminifères selon les traitements (moyenne ± écart-type). Comparaison par rapport à Hormones X1 : ***p<0.001, ****p<0.0001 (test de Kruskal-Wallis**

| Traitements | Diamètre moyen des tubes séminifères (µm) (moyenne ± SD) | Aire moyenne des tubes séminifères (µm2) (moyenne ± SD) |
|---|---|---|
| Traités au Firmagon | 164 ± 12 (****) | 20270 ± 3657 (****) |
| + saline | 157 ± 9 (****) | 17860 ± 1802 (****) |
| + hormones X1 | 325 ± 26 | 77620 ± 10045 |
| + hormones X1 + 13VHB | 373 ± 28 (***) | 103540 ± 12590 (***) |

On observe un diamètre moyen des tubes séminifères significativement supérieur dans le lot [hormones X1 + 13VHB] comparativement au lot [hormones X1] (***, p<0.001). De même, l'aire moyenne des tubes est significativement supérieure dans le lot [hormones X1 + 13VHB] comparativement au lot [hormones X1] (p<0.001). Les groupes contrôles [traités au Firmagon] et [+saline] sont très significativement inférieurs aux groupes traités avec hormones seules ou en association avec 13VHB (****, p<0.0001).

L'ensemble de ces résultats démontrent que le traitement hormonal en combinaison avec 13VHB est significativement plus efficace que le traitement avec hormones seules avec une reprise et une stimulation de la spermatogénèse significativement plus importante. Après 8 semaines de traitement hormones X1 + 13VHB (un seul cycle de spermatogénèse), le niveau de production des spermatozoïdes est redevenu identique à celui des animaux WT contrôles non traités au Firmagon. Chez l'homme, cette perspective permettrait de raccourcir significativement les temps de traitement nécessaires pour relancer et stimuler la spermatogénèse chez un individu oligozoospermique par exemple.

### EXEMPLE 7 : EFFET POTENTIALISANT IN VIVO DE L'ANTICORPS HUMANISE 13VHB-139VL (SEQ ID NO : 9 et SEQ ID NO : 16) SUR LA SPERMATOGENESE CHEZ LA SOURIS HYPOGONADIQUE hpg MALE ADULTE

L'effet de l'anticorps 13VHB sur la stimulation de la spermatogénèse a été évalué également dans un modèle d'hypogonadisme hypogonadotrope congénital chez la souris mâle adulte *hpg.* Ces souris sont porteuses d'une mutation *(hpg)* du gène du GnRH entraînant une absence de sécrétion d'hormones gonadotropes (LH et FSH) endogènes [20]. A l'état homozygote pour la mutation *hpg* (*hpg -*/*-*), les mâles sont dépourvus de toute spermatogenèse. Ils constituent en cela un modèle « naturel » d'hypogonadisme hypogonadotrope et d'azoospermie chez le mâle.

Ces souris ont été obtenues suite à la revitalisation d'embryons provenant du laboratoire Jackson (The Jackson Laboratory, USA) et la lignée a été entretenue par les laboratoires Charles River (France).

Pour chaque expérimentation, des souris mâles homozygotes adultes, âgés de 8 semaines, ont reçu un traitement hormonal, seul ou en association avec le variant 13VHB, pendant 7 semaines au minimum. Cette durée correspond à un cycle de spermatogénèse qui est de 35 jours chez la souris. Pour les groupes d'animaux traités avec le variant 13VHB, l'anticorps a été injecté à la dose de 20 µg/kg deux ou trois fois par semaine. Cette dose correspond à une concentration de l'ordre de 0,26 µg d'anticorps par ml de sang si l'on admet un volume de sang de 75 ml/kg chez la souris (University of Pittsburgh Policy for Regulating the Volume of Experimental Blood Sample Withdrawals in Laboratory Animals). L'anticorps 13VHB était préparé dans du PBS stérile.

A la fin de chaque traitement, les animaux ont été sacrifiés et du sang a été prélevé en intracardiaque sur tubes héparinés, et centrifugé. Les plasmas ainsi ont été congelés à -20°C. Les testicules et les épididymes ont été prélevés et pesés.

Les testicules ont ensuite été broyés dans du milieu Ham's F12 complémenté en sodium pyruvate 1% et Hepes 1%, puis soniqué. Le nombre de têtes de spermatides a ensuite été compté au microscope optique afin d'évaluer la réserve spermatique. Un testicule pour chacun des lots expérimentaux a été fixé dans une solution de Formaline à 10% et inclus dans de la paraffine pour une analyse histologique. Des coupes fines de 5 µm d'épaisseur ont été réalisées dans l'axe transversal du testicule et colorées à l'acide périodique de Schiff (PAS) et à l'hématoxyline-éosine (H&E). L'examen des lames colorées a été effectué à l'aide du logiciel NDP Viewer. Les coupes de testicules ont été évaluées conformément aux guidelines pour l'évaluation histopathologique de l'OCDE (Partie 2 : Système reproducteur masculin). Parallèlement, une analyse des préparations testiculaires par cytométrie en flux a été réalisée afin de mesurer le nombre de cellules haploïdes (Spermatides), diploïdes (Spermatogonies, Spermatocytes II) et tétraploïdes (Spermatocytes I) et suivre ainsi l'évolution de la reprise de la méïose selon les traitements reçus par les animaux.

Les épididymes ont été découpés en fines sections et incubés au moins 4 heures à température ambiante dans du milieu Ham's F12 complémenté en sodium pyruvate 1% et Hepes 1%. Les préparations ont ensuite été récupérées, filtrées et les spermatozoïdes ont été comptés au microscope optique.

Trois protocoles différents de traitements hormonaux ont été utilisés dans ces essais. Ces protocoles sont utilisés en médecine humaine chez les hommes souffrant d'infertilité d'origine hormonale. C'est le cas par exemple des patients azoospermiques, OligoAsthenoTeratoSpermiques (OATS) ou Oligozoospermiques.

### Effet du variant 13VHB dans un protocole de stimulation utilisant de la FSH et de la hCG.

Deux lots de souris mâles *hpg* -/-, ont été traités pendant sept semaines :
- 6 souris ont reçu un mélange FSH 1 UI (Gonal^{®}, Merck) + hCG 1 UI (Ovitrelle^{®}, Merck) injecté 5 fois par semaine ;
- 7 souris ont reçu un mélange FSH 1 UI + hCG 1 UI injecté 5 fois par semaine et le variant 13VHB injecté à 20 µg/kg 3 fois par semaine.

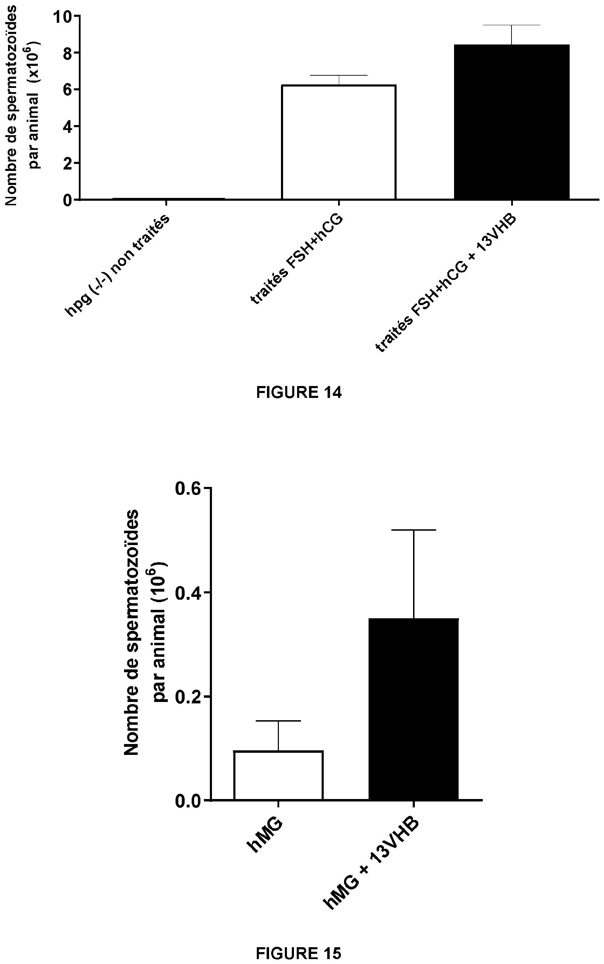

Après 7 semaines de traitement, les animaux ont été sacrifiés, les épididymes prélevés et les préparations de spermatozoïdes analysées. Les résultats sont exprimés en nombre total de spermatozoïdes (millions) par animal. Ils sont illustrés Figure 14 et permettent de comparer la reprise de la spermatogénèse dans les deux traitements. Chez les homozygotes contrôles, non traités, aucun spermatozoïde épididymaire n'a été observé attestant que ces mâles étaient totalement azoospermiques. Chez les homozygotes traités avec le mélange FSH + hCG en association avec le variant 13VHB le nombre moyen de spermatozoïdes par animal était 1,4 fois supérieur à celui obtenu chez les mâles traités avec le mélange FSH + hCG seul : 8,5.10⁶ ± 0,5.10⁶ spermatozoïdes contre 6,2.10⁶ ± 0,5.10⁶ respectivement. Bien que non significative cette tendance traduit une reprise plus importante de la spermatogénèse avec le traitement associant le variant 13VHB.

### Effet du variant 13VHB dans un protocole de stimulation utilisant de la hMG.

L'effet du variant 13VHB a été évalué en association avec un traitement hormonal utilisant de la hMG (Menopur^{®}, Ferring) à la dose de 25 UI/kg. La hMG est constituée d'un mélange équimolaire de FSH et de LH.

Deux lots de souris mâles *hpg* -/-, ont été traités pendant sept semaines :
- 8 souris ont reçu 25 Ul/kg Menopur (hMG) injectée 5 jours par semaine ;
- 9 souris ont reçu 25 Ul/kg Menopur (hMG) 5 jours par semaine et le variant 13VHB injecté à 20 µg/kg 2 jours par semaine. Le variant 13VHB était préparé dans du PBS stérile.

Après 7 semaines de traitement, les animaux ont été sacrifiés, les épididymes prélevés et les préparations de spermatozoïdes analysées. Les résultats illustrés Figure 15 montrent que la valeur moyenne du nombre de spermatozoïdes chez les mâles traités avec hMG en association avec le variant 13VHB était 3,5 fois supérieure à celle obtenue chez les mâles traités avec le mélange hMG seule : 0,35.10⁶ ± 0,18.10⁶ spermatozoïdes par animal contre 0,1.10⁶ ± 0,05.10⁶ respectivement. Bien que non significative cette tendance traduit là encore une reprise plus importante de la spermatogénèse avec le traitement hMG associant le variant 13VHB.

### Effet du variant 13VHB dans un protocole de stimulation associant un traitement avec FSH suivi d'un traitement avec hMG.

L'effet du variant 13VHB a été évalué en association avec un troisième type de protocole comprenant :
- 7 semaines de traitement avec 1 UI FSH (Gonal-f^{®}, Merck) injectée 5 jours par semaine ;
- suivies de 7 semaines de traitement avec 25 Ul/kg hMG (Menopur^{®}, Ferring) injectée 5 jours par semaine.

Deux lots de souris mâles *hpg* -/-, ont été traités :
- un lot de 8 souris a reçu le traitement hormonal seul ;
- un lot de 9 souris a reçu le traitement hormonal en association avec le variant 13VHB injecté à la dose de 20 µg/kg 2 jours par semaine. Le variant 13VHB a été préparé dans du PBS stérile.

Après les 14 semaines de traitement, les animaux ont été sacrifiés, les testicules et les épididymes ont été prélevés, pesés et les préparations de spermatides et de spermatozoïdes analysées.

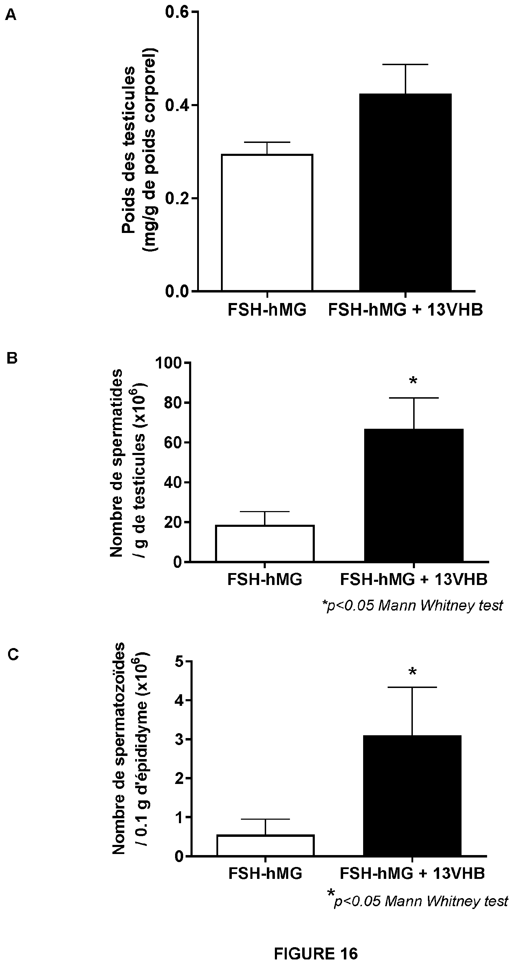

Les résultats obtenus sont illustrés Figure 16. Ils montrent que les animaux traités avec le variant 13VHB associé au traitement hormonal ont un poids testiculaire (Figure 16A), un nombre de spermatides dans le testicule (Figure 16B) et un nombre de spermatozoïdes épididymaires (Figure 16C) plus élevés que chez les animaux ayant été traités avec hormones seules.

Le poids des testicules est 1,45 fois plus élevé chez les animaux ayant reçu le traitement hormonal en association avec le variant 13VHB par rapport aux animaux ayant reçu le traitement hormonal seul : 0,42 mg/g de poids corporel contre 0,29 mg/g de poids corporel respectivement. Cette différence n'est pas significative (Figure 16A).

On constate que le lot ayant reçu le traitement hormonal en association avec le variant 13VH a un nombre moyen de spermatides 3,5 fois supérieur à celui du lot ayant reçu le traitement hormonal seul : 67.10⁶ ± 13.10⁶ spermatides/g de testicule contre 19.10⁶ ± 6,1.10⁶ spermatides/g de testicule respectivement (Figure 16B). Cette différence est significative (* : *p<0.05,* Mann Whitney test).

De même, le lot ayant reçu le traitement hormonal en association avec le variant 13VH a un nombre moyen de spermatozoïdes épididymaires 6 fois supérieur à celui du lot ayant reçu le traitement hormonal seul : 3,1.10⁶ ± 1,14.10⁶ spermatozoïdes / 0,1 g d'épididyme contre 0,55.10⁶ ± 0,4.10⁶ respectivement (Figure 16C). Cette différence est significative (* : *p<0.05,* Mann Whitney test).

### Analyse histologique des testicules

Une analyse histologique des coupes de testicules provenant de chacun des lots expérimentaux a été réalisée et a montré une structure très atrophique du testicule issu d'un mâle homozygote avec des tubes séminifères vides, de petite taille et un tissu interstitiel réduit et déstructuré. L'histologie des testicules issus du lot traité avec hormones seules et hormones + 13VHB a montré dans les deux cas une structure plus dense avec une taille des tubes séminifères plus importante (diamètre et surface) dans le testicule du lot hormones + 13VHB.

Plus précisément, le diamètre moyen des tubes séminifères (µm) et l'aire moyenne des tubes séminifères (µm²) ont été mesurés par analyse d'images sur 50 sections circulaires de tubes séminifères de chaque coupe histologique. Les résultats sont illustrés dans le tableau 11.

**Tableau 11 : Valeurs moyennes des diamètres et des aires des sections de tubes séminifères selon les traitements (moyenne ± écart-type). Comparaison par rapport au traitement hormones : **p<0.01, *p<0.05 (test de Kruskal-Wallis).**

| Traitement | Diamètre moyen des tubes séminifères (µm) (moyenne ± SD) | Aire moyenne des tubes séminifères (µm²) (moyenne ± SD) |
|---|---|---|
| + hormones | 398,4 ± 38,7 | 12010 ± 2500 |
| + hormones + 13VHB | 470,2 ± 43,7 (*) | 16640 ± 3100 (**) |

On observe un diamètre moyen des tubes séminifères significativement supérieur dans le lot [hormones + 13VHB] d'un facteur 1,2 comparativement au lot [hormones] (*, p<0,05). De même, l'aire moyenne des tubes est significativement supérieure dans le lot [hormones + 13VHB] d'un facteur 1,4 comparativement au lot [hormones] (**, p<0,01). Le traitement associant 13VHB a donc augmenté le développement des tubules séminifères avec un diamètre moyen et une surface moyenne significativement supérieurs attestant d'une reprise et d'une stimulation de la spermatogenèse significativement plus importante par rapport au traitement hormonal seul.

### Analyse des populations cellulaires du testicule par cytométrie en flux

Une analyse des différentes populations de cellules testiculaires a été réalisée par cytométrie en flux. Pour cela, un volume de 20 µl de suspension cellulaire issue du broyat de chaque testicule a été analysé en cytométrie de flux après filtration sur une grille de 100 µm et marquage de l'ADN des cellules au Hoechst 33342. Les cellules à compter ont été sélectionnées en déterminant la zone de la fluorescence bleue émise par le Hoechst 33342 sur le biparamétrique associant FSC et SSC. A partir de cette sélection préalable, un nouvel histogramme biparamétrique associant l'auto-fluorescence verte et la fluorescence bleue du Hoescht 33342 est alors utilisé, sur lequel sont définies une population cellulaire à 1C ADN (Spermatides, Spermatozoïdes), une population cellulaire à 2C ADN (Spermatogonies, Spermatocytes II, cellules somatiques) et une population cellulaire à 4C ADN (Spermatocytes I).

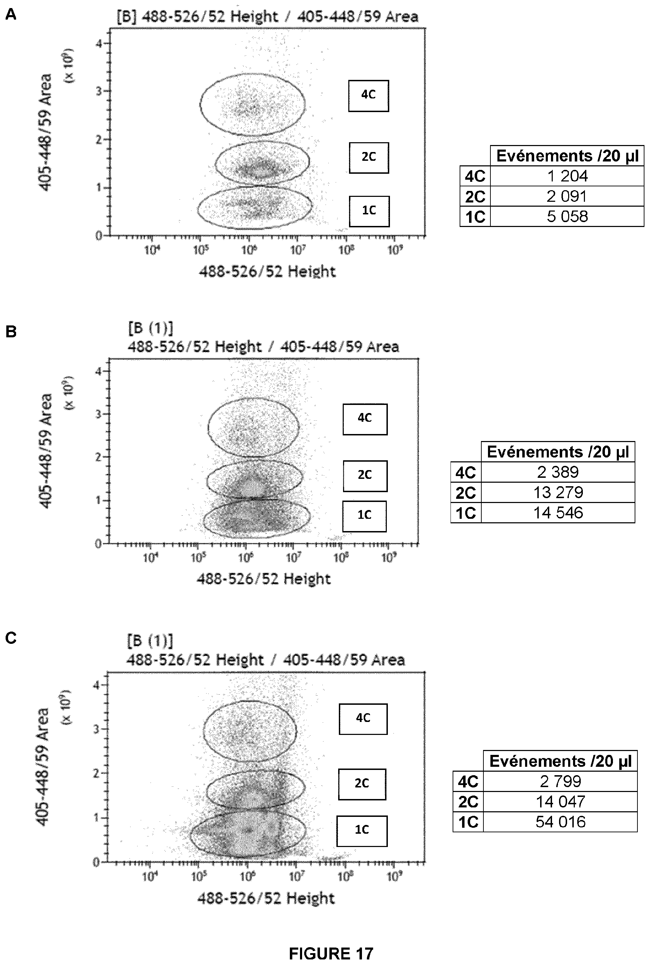

Des résultats individuels représentatifs de chaque lot sont illustrés Figure 17. La Figure 17A illustre le cas d'un mâle n'ayant pas répondu au traitement. La Figure 17B illustre le cas d'un mâle ayant répondu au traitement hormonal seul et la Figure 17C celui d'un mâle ayant répondu au traitement Hormones+13VHB. On constate que le nombre d'évènements à 4C et 2C (cellules à 4 et à 2 chromatides) sont proches entre les deux traitements. A l'inverse, le nombre de cellules haploïdes (évènements à 1C) chez l'animal traité avec Hormones + 13VHB est 3,7 fois supérieur à celui traité avec Hormones seules: 54 016 évènements versus 14 546 respectivement. Ce résultat traduit la mise en place d'une spermatogénèse plus intense avec une production de spermatides et spermatozoïdes supérieure chez l'animal traité avec hormones + 13VHB.

Les valeurs moyennes obtenues avec l'ensemble des animaux du lot traité avec hormones seules et ceux du lot traité avec hormones + 13VHB sont illustrées dans le tableau 11 suivant.

**Tableau 11**

| Population cellulaire par testicule | Traitement 1 (T1) avec hormones seules | % | Traitement 2 (T2) avec hormones + 13VHB | % | Différence T2 - T1 |
|---|---|---|---|---|---|
| 1C | 904 870 | 63 | 1 250 425 | 75 | + 345 555 |
| 2C | 429 422 | 30 | 359 748 | 21 | - 69 674 |
| 4C | 98 118 | 7 | 65 198 | 4 | - 32 920 |
| Total | 1 432 410 | | 1 675 372 | | + 242 962 |

Les résultats montrent qu'après un traitement hormonal associant 13VHB, le nombre moyen de cellules haploïdes (spermatides) a augmenté de 12% par rapport au lot traité avec hormones seules. Inversement, le nombre moyen de cellules à 4C (spermatocytes I) et 2C est numériquement et proportionnellement un peu plus faible avec le traitement hormonal associant 13VHB : -9% et -3% respectivement. Ceci démontre qu'une spermatogénèse plus intense s'est mise en place chez les mâles ayant reçu 13VHB aboutissant à un nombre de spermatides formés (1C) plus élevé, après une même durée de traitement. Cette reprise plus importante de la spermatogénèse a pour conséquence également un nombre de cellules 4C en baisse, reflétant une levée plus efficace du blocage de la spermatogénèse au stade Spermatocytes 1, caractéristique des males homozygotes hpg (-/-).

L'impact de ces résultats précliniques est très important car ils démontrent très clairement un effet potentialisant significatif d'un anticorps variant humanisé selon l'invention, i.e. 13VHB, permettant une restauration plus rapide, et une stimulation plus intense et plus efficace de la spermatogénèse chez des mâles ayant une azoospermie congénitale comparativement à un traitement hormonal seul standard.

### EXEMPLE 8 : CONSTRUCTION, PRODUCTION ET CARACTERISATION FONCTIONNELLE D'UN FRAGMENT MONOVALENT DU VARIANT 13VHB-139VL (SEQ ID NO: 18)

Un fragment monovalent a été développé à partir du variant humanisé 13VHB-139VL sous un format scFv. Ses propriétés de liaison et de potentialisation *in vitro* de la FSH et de la LH/CG ont été évaluées tel que décrit pour la sélection des variants, et, comparées au variant entier IgG4.

### Construction du fragment scFv du variant 13VHB

Le gène de synthèse codant pour le fragment scFv 13VHB dérivé du variant 13VHB-139VL a été synthétisé par ATG:Biosynthetics GmbH (Allemagne). Le scFv 13VHB a été constitué par la fusion de la séquence de la chaîne lourde CF12-13-VHB (SEQ ID NO : 9) et de la séquence de la chaîne légère CF12-VL139 (SEQ ID NO : 16) par l'intermédiaire d'une séquence codant pour un linker [GGGGS]₃ et l'ajout en 3' d'une séquence codant pour 6x Histidine. Le gène de synthèse a été conçu par la fusion de la séquence débutant au site enzymatique Hindlll et la fin de la séquence codant pour le peptide signal d'adressage au cytoplasme PelB décrite dans Ward et al. [21] avec le gène du scFv 13VHB terminé par une séquence de site enzymatique Xhol. La séquence décrite par Ward et al. [21] a été modifiée en substituant la thymine (T) en position 23 par une cytosine (C). Le gène de synthèse a été inséré dans le plasmide pUC19-PelB construit tel que décrit par Ward et al. [21] entre les sites enzymatiques Hindlll et Xhol. Après contrôle par séquençage de la qualité de la construction, le plasmide pUC19-scFv 13VHB a été transformé par choc thermique en bactéries HB2151 (T53040, Interchim, France) rendues compétentes [22].

**Tableau 12 : Séquence peptidique du scFv 13VHB**

| scFv 13VHB | |
|---|---|
| Séquence peptidique | QVQLVQSGAEVKKPGASVKVSCKTSGFTFSSSYISWLRQAPGQRL EWIAWIYAGTGGTSYSQKFQGRVQLTVDTSASTAYMEFSSLRSED TAVYYCARHGSYFDYWGQGTLVTVSSGGGGSGGGGSGGGGSDI QLTQSPSSLSASVGDRVTITCRASQSVDYDGDSYMNWYQQKPGK APKLLIYAASSLESGVPSRFSGSGSGTDFTLTISSLQPEDFATYYCQ QSNEDPYTFGQGTKLEIKHHHHHH* |
| SEQ ID NO : 17 | |

### Production du fragment d'anticorps recombinant

### 1/ Culture bactérienne

Une pré-culture a été réalisée dans 5 ml de milieu 2xYT contenant 100 µg/ml d'ampicilline toute la nuit à 37°C 200 RPM. Le lendemain, 500 µl de cette préculture ont été ensemencés dans 500 ml de milieu 2xYT contenant 50 µg/ml d'ampicilline et mis à croître à 37°C à 180 RPM jusqu'à obtenir une DO₆₀₀ₙₘ de 1,5. La synthèse du scFv a été induite par l'ajout de 0,1 mM d'IPTG pendant 16 h à 16°C et 150 RPM.

### 2/ Extraction

La suspension bactérienne a été centrifugée 30 min à 4000 g à 4°C. La suite de la préparation a été réalisée à 4°C. Pour extraire le périplasme bactérien, le culot a été remis en suspension et incubé dans 10 ml de tampon TES (Tris 0,2 M pH8, EDTA 0,5 mM, saccharose 0,5 M) pendant 30 min auxquels ont alors été ajoutés 15 ml de tampon TES dilué au ¼ pour être de nouveau incubé 30 min. L'extrait bactérien a alors été centrifugé 45 min à 10 000g. Le surnageant a été mis à dialyser contre un tampon phosphate de sodium 20 mM, NaCl 300 mM pH 7,4 pendant une nuit. Le surnageant dialysé a ensuite été traité immédiatement pour purifier le scFv ou conservé à -20°C jusqu'à utilisation.

### 3/ Purification

Le périplasme a été centrifugé pendant 20 min à 4000 g à 4°C. Une solution d'imidazole concentrée a été ajoutée au surnageant pour ajuster la concentration à 10 mM. Le surnageant a alors été incubé avec de la résine Ni-NTA HisPur^{™} (88221, ThermoFisher Scientific, MA, USA) sous agitation pendant 1h à 4°C. La résine a ensuite été lavée avec un tampon phosphate de sodium 20 mM, NaCl 300 mM pH 7,4 additionné de 20 mM d'imidazole jusqu'à obtenir une DO₂₈₀ₙₘ proche de 0. Le scFv 13VHB a alors été élué avec un tampon phosphate de sodium 20 mM, NaCl 300 mM, imidazole 250 mM pH 7,4. L'éluat a été dialysé contre du tampon phosphate de potassium 10 mM, NaCl 150 mM pH 7,4 et conservé à -80°C.

### 4/ Contrôle qualité

Afin de vérifier sa pureté, la fraction purifiée du scFv 13VHB a été analysée par électrophorèse sur gel de polyacrylamide à 15% après coloration au bleu de Coomassie et par Western Blot avec un anticorps anti-His tag (R931-25, ThermoFisher Scientific, MA, USA). Les résultats ont montré la présence d'une seule bande homogène traduisant la présence d'une seule forme moléculaire du produit, sans aucune forme dégradée.

### Activité de liaison du scFv 13VHB sur la hFSH et la LH/hCG

L'activité de liaison du scFv 13VHB sur les hormones recombinantes humaines FSH (Gonal-f ^{®}, Merck) et hCG (Ovitrelle ^{®}, Merck) ainsi que sur la hMG extraite (Menopur ^{®}, Ferring) a été étudiée par technique ELISA. Chaque hormone évaluée a été préparée à la concentration de 10 µg/ml dans du tampon PBS et distribuée à raison de 100 µl par puits sur une plaque ELISA Immulon 2HB (Réf. 3455 Thermo Fisher, USA). Le temps d'adsorption a été de 18 heures à +4°C. Après cinq lavages, les puits ont été traités avec 100 µl de PBS additionné de BSA 1% pendant 1 heure à 37°C, puis le scFv 13VHB préparé à différentes concentrations a été distribué à raison de 100 µl/puits et incubé 2 heures à 37°C. La gamme de concentrations du scFv préparée dans du PBS additionné de 0,2% de BSA allait de 3,5 à 600 µg/ml.

Après cinq lavages, un anticorps secondaire anti-His Tag couplé à la peroxydase (HRP) (Réf. R93125 Life technologies, France) dilué au 1/1000^{ème} dans une tampon PBS-BSA 0,2% a été distribué à raison de 100 µl/puits et incubé 1 heure à 37°C. Après cinq lavages, l'activité enzymatique a été révélée avec du TMB (Réf 5120-0074 SeraCare Life Sciences, USA) distribué à raison de 100 µl/puits et incubé 10 minutes à température ambiante. Après ajout de H₂SO₄ 1 M (50 µl/puits) l'intensité de la réaction colorée (Densité Optique) a été mesurée à l'aide d'un spectrophotomètre pour plaques ELISA.

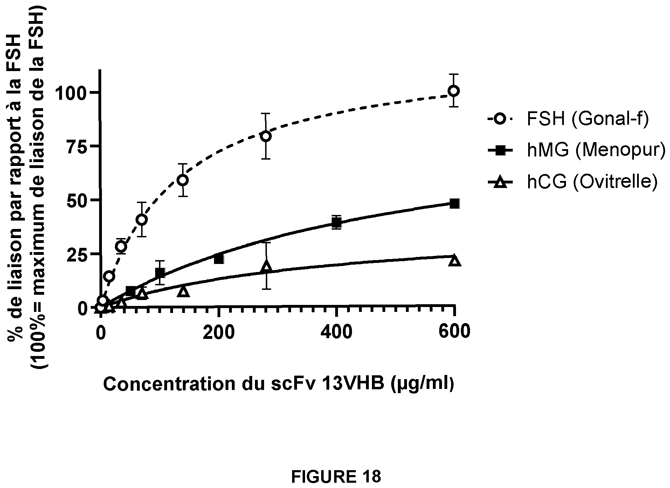

Les résultats ont mis en évidence une activité de liaison du scFv sur les trois hormones. La Figure 18 représente les courbes de liaison exprimées en pourcentage par rapport au 100% donné par le signal obtenu avec le scFv à 600 µg/ml sur la FSH. Le signal basal a été fixé à 0%. Une liaison du scFV 13VHB a été observée sur les trois hormones. Comparativement à la FSH (100%), le maximum de liaison obtenu sur hMG a été de 47,7% et le maximum de liaison sur hCG a été de 21,2%. Le niveau de binding est différent selon les hormones attestant d'une spécificité de liaison du scFv semblable à celle du variant 13VHB IgG4, à savoir une meilleure affinité sur hFSH puis par ordre décroissant sur hMG et hCG.

### Effet potentialisant du scFv 13VHB in vitro sur la bioactivité de la FSH

L'effet potentialisant du scFv 13VHB sur l'activité de la FSH a été évalué *in vitro* sur des cellules HEK 293 (HiTSeeker HEK293 cells) exprimant le récepteur humain de la FSH (Innovative Technologies in Biological Systems, référence P30117, Derio Bizkaia, Espagne).

Les cellules ont été distribuées à raison de 80 000 cellules par puits, dans des boites 96 puits à contours et fond blancs (Greiner Bio One référence 655083, Dominique Dutscher, Brumath, France), dans du milieu DMEM (Sigma-Aldrich référence D6429, Merck Millipore, Saint Quentin Fallavier, France) supplémenté avec 10% de sérum de veau foetal (GIBCO, référence A47668-01, Thermofisher, USA) décomplémenté au préalable, 10 µg/ml de puromycine (Invivogen référence ant-pr, Toulouse, France) et incubées la nuit dans un incubateur à 37°C 5°C CO₂ sous atmosphère humide. Le lendemain, le milieu des cellules a été aspiré et remplacé par 30 µl de tampon phosphate (PBS) contenant 1% d'hepes (Lonza référence BE17-737E). Après 2 heures d'incubation à 37°C, sous atmosphère humide et 5% CO₂, les cellules ont été stimulées pendant 1h30 avec 15 µl d'une gamme de concentrations de FSH recombinante (Gonal-f^{®}, Merck) allant de 10⁻¹² M à 5.10⁻⁹ M ou de hMG extraite (Menopur^{®}, Ferring) allant de 10⁻¹¹ M à 5.10⁻⁸ M, préparée seule ou en combinaison avec le scFv ou le variant entier à la concentration fixe de 100 µg/ml (3,5 µM). Le mélange hormone + scFv ou variant a été préincubé 20 minutes à 37°C avant dépôt. Après stimulation, l'AMPc a été mesurée à l'aide d'un kit HitHunter^{®} cAMP Assay for biologicals (Eurofins Discoverx Products LLC référence 90-0075LM25, Fremont, USA). La luminescence émise a été quantifiée à l'aide d'un lecteur de plaques 96 puits LUMIstar Oméga (BMG Labtech, Champigny sur Marne, France). Les valeurs de l'EC50 ont été calculées par le logiciel Prism (GraphPAd Prism Software Inc., San Diego, CA, USA, version 10.0.1).

Les résultats obtenus avec Gonal-f sont illustrés dans le tableau 11. Ils montrent un effet potentialisant du scFv 13VHB se traduisant par une augmentation de la puissance par rapport à la réponse en AMPc comparativement à celle obtenue avec la FSH seule.

**Tableau 11 : Valeurs des EC50 des courbes dose réponse obtenues après stimulation avec Gonal-f, Gonal-f + scFv 13VHB et Gonal-f + variant 13VHB entier.**

| | Gonal-f | Gonal-f + scFv 13VHB | Gonal-f + variant 13VHB |
|---|---|---|---|
| EC50 (M) | 1,25.10⁻¹⁰ | 4,83.10⁻¹¹ | 3,02.10⁻¹¹ |

On observe que l'EC50 obtenue avec le complexe FSH + scFv 13VHB est augmentée de 2,6 fois par rapport à l'EC50 de la FSH seule. Comparativement, l'EC50 est augmentée d'un facteur 4 avec le complexe FSH +variant 13VHB entier.

Les résultats obtenus avec Menopur^{®} ont montré un effet potentialisant du scFv identique à celui du variant 13VHB entier comme l'illustre le tableau 12.

**Tableau 12 : Valeurs des EC50 des courbes dose réponse obtenues après stimulation avec Menopur, Menopur + scFv 13VHB et Menopur + variant 13VHB.**

| | Menopur | Menopur °+ scFv 13VHB | Menopur + variant 13VHB |
|---|---|---|---|
| EC50 (M) | 3,83.10⁻⁹ | 1,42.10⁻⁹ | 1,37.10⁻⁹ |

Le complexe hMG + scFv 13VHB induit une augmentation de la puissance de la réponse en AMPc identique à celle du complexe hMG + variant entier : la valeur de l'EC50 avec le mélange FSH + scFv est augmentée de 2,7 fois par rapport à l'EC50 de la hMG seule, et de 2,8 fois avec le variant 13VHB entier.

En conclusion, le fragment monovalent scFv 13VHB exerce un effet potentialisant sur l'activité de la FSH *in vitro.* Cet effet est très proche de celui du variant 13VHB entier dans le cas de Gonal-f et identique à celui du variant 13VHB entier dans le cas de Menopur^{®}.

Pour compléter cette étude, une dose réponse a été réalisée en stimulant les cellules avec une gamme croissante du ligand (13VHB variant entier ou son scFv) associée à une dose fixe d'hormone FSH (Gonal-f, Merck) ou de hMG (Menopur^{®}, Ferring).

Pour cela, le même protocole expérimental a été utilisé. Les cellules ont été stimulées pendant 1h30 soit avec 15 µl d'une gamme de concentrations de scFv allant de 5.10⁻⁹ M à 10⁻⁵ M soit avec une gamme de concentrations du variant 13VH entier allant de 5.10⁻¹⁰ M à 10⁻⁶ M. Dans les deux cas, chaque point de concentrations du scFv ou de l'anticorps entier a été préparé en combinaison avec de la FSH (Gonal-f) à la concentration fixe de 50 pM ou avec de la hMG (Menopur) à la concentration fixe de 1 nM. Le mélange scFv ou anticorps entier + hormone a été pré-incubé 20 minutes à 37°C avant dépôt sur les cellules.

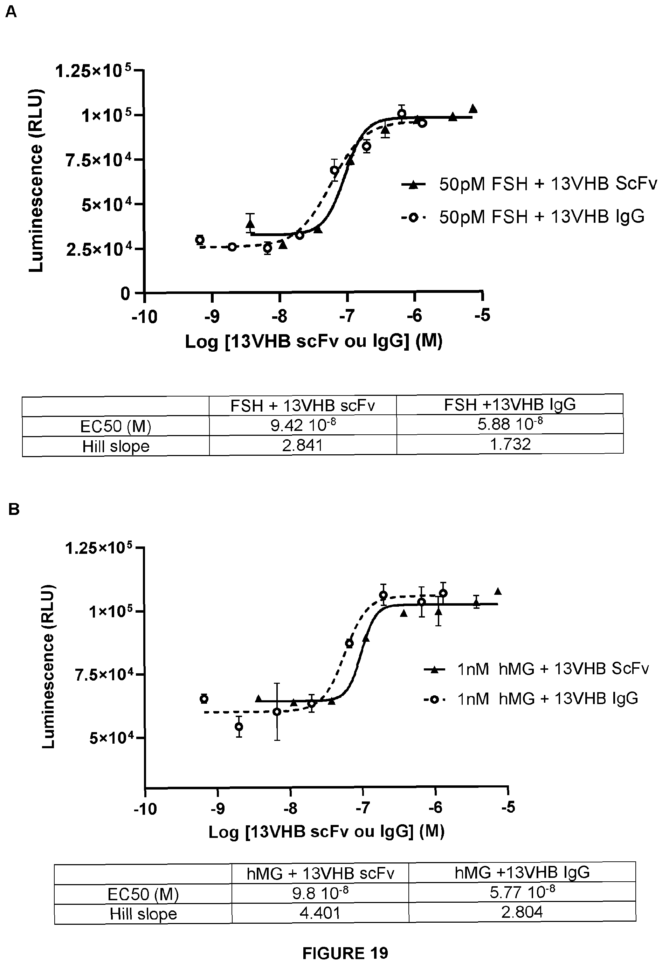

Les courbes dose réponse (D/R) scFv ou anticorps entier sont illustrées Figure 19. La Figure 19A montre que les courbes obtenues avec 50 pM de FSH (Gonal-f) sont relativement similaires : dans les deux cas, l'EC50 est de l'ordre de 10⁻⁸ M avec un facteur de 1,6 en faveur de l'anticorps entier. La pente de Hill est 1,64 fois plus forte avec le scFv que l'anticorps entier. La Figure 19B montre également que les courbes obtenues avec 1 nM de hMG (Menopur) sont relativement similaires entre le scFv et l'anticorps entier : l'EC50 est de l'ordre de 10⁻⁸ M avec une puissance 1,7 fois supérieure dans le cas de 13VHB IgG. La pente de Hill est également 1,57 fois plus forte dans le cas du scFv. Il est à noter que les valeurs de l'EC50 et de la pente de Hill sont très reproductibles d'une hormone à l'autre : par exemple l'EC50 du scFv est 9,42.10⁻⁸ M avec FSH et 9,8.10⁻⁸ M avec hMG. De même l'EC50 du variant entier est de 5,88.10⁻⁸ M avec FSH et 5,77.10⁻⁸ M avec hMG. La valeur de la pente de Hill obtenue avec le scFv est supérieure de 1,6 fois en moyenne dans le cas des deux hormones.

Ces résultats démontrent que l'effet potentialisant sur l'activité de la FSH n'est pas dépendant de la bivalence de l'anticorps13VHB, le fragment monovalent scFV exerçant également le même effet.

### Effet potentialisant du scFv 13VHB in vitro sur la bioactivité de la LH/CG

L'effet potentialisant du scFv 13VHB sur l'activité de la LH/CG a été évalué *in vitro* sur des cellules HEK 293 exprimant le récepteur humain de la LH (Innovative Technologies in Biological Systems, référence P30177, Derio Bizkaia, Espagne).

Les cellules ont été distribuées à raison de 80 000 cellules par puits, dans des boites 96 puits à contours et fond blancs (Greiner Bio One référence 655083, Dominique Dutscher, Brumath, France), dans du milieu DMEM (Sigma-Aldrich référence D6429, Merck Millipore, Saint Quentin Fallavier, France) supplémenté avec 10% de sérum de veau foetal (GIBCO, référence A47668-01, Thermofisher, USA) décomplémenté au préalable, 10 µg/ml de puromycine (Invivogen référence ant-pr, Toulouse, France) et incubées la nuit dans un incubateur à 37°C 5% CO₂ sous atmosphère humide. Le lendemain, le milieu des cellules a été aspiré et remplacé par 30 µl de tampon phosphate (PBS) contenant 1% d'hepes (Lonza référence BE17-737E). Après 2 heures d'incubation à 37°C, sous atmosphère humide et 5% CO₂, les cellules ont été stimulées pendant 1h30 avec 15 µl d'une gamme de concentrations de hCG (Ovitrelle^{®}, Merck) allant de 10⁻¹¹ M à 5.10⁻⁹ M, préparée seule ou en combinaison avec le scFv à la concentration fixe de 100 µg/ml (3,5 µM). Le mélange hCG + scFv a été préincubé 20 minutes à 37°C avant dépôt.

Après stimulation, l'AMPc a été mesurée à l'aide d'un kit HitHunter^{®} cAMP Assay for biologicals (Eurofins Discoverx Products LLC référence 90-0075LM25, Fremont, USA). La luminescence émise a été quantifiée à l'aide d'un lecteur de plaques 96 puits LUMIstar Oméga (BMG Labtech, Champigny sur Marne, France).

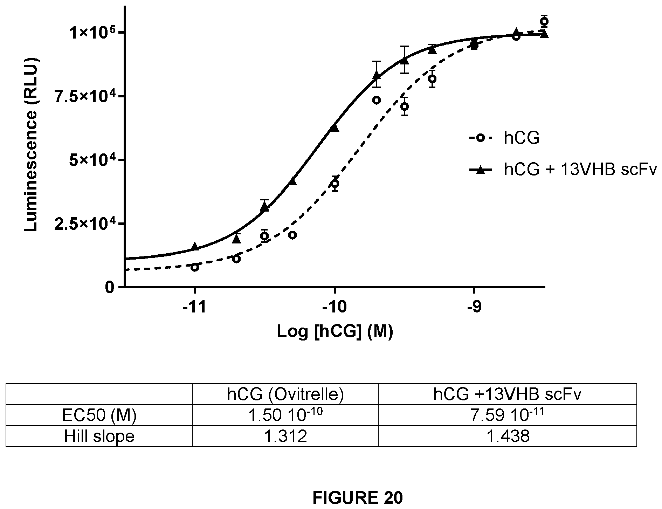

L'effet potentialisant a été évalué en comparant les courbes dose-réponse obtenues avec l'hCG seule et avec le complexe hCG + scFv 13VHB. Les résultats sont illustrés Figure 20 et montrent un effet potentialisant du scFv sur l'activité de la hCG avec une meilleure puissance de la réponse en AMPc. La valeur de l'EC50 obtenue avec le complexe hCG + scFv 13VHB est augmentée d'un facteur 2 par rapport à celle obtenue avec la hCG seule : elle est de 0,75.10⁻¹⁰ M avec scFv 13VHB versus 1,5.10⁻¹⁰ M avec hCG seule. La valeur de la pente de la courbe dose/réponse (Hill slope) est également augmentée dans le cas de la stimulation avec hCG + scFv 13VHB : elle est de 1,438 avec scFv + hCG versus 1,312 avec hCG seule.

Outre son effet potentialisant sur l'activité FSH, ces résultats démontrent que la forme monovalente d'un anticorps variant humanisé, *i.e*. scFv 13VHB, est également capable de potentialiser l'activité LH comme l'anticorps variant humanisé bivalent dont il dérive, *i.e*. le variant 13VHB entier bivalent.

### Liste de références

[1] Hervé et al., Endocrinology, 145 : 294-303, 2004
[2] Brevet EP 1518863
[3] Demande Internationale WO 2012/066519
[4] Demande internationale WO 2016/038308
[5] Jones et al., Nature, 321 : 522-525, 1986
[6] Verhoeyen et al., Science, 239 : 1534-1536, 1988
[7] Foote J., Winter G., J. Mol. Biol., 224 : 487-499, 1992
[8] Makabe. et al., J. Biol. Chem., 283 : 1156-1166, 2008
[9] Ramos, Methods Mol. Biol., 907 : 39-55, 2012
[10] Lefranc et al., Nucleic Acids Res., 29 : 207-209, 2001
[11] Kabat et al ., Sequences of Proteins of Immunological Interest, 4th edn., US Department of Health and Human Services, N1H, Washington, DC. 1991
[12] Chothia et al., J. Mol. Biol., 196(4) : 901-917, 1987
[13] Chothia et al., Nature, 342(6252) : 877-883, 1989
[14] Chothia et al., J. Mol. Biol., 278(2) : 457-479, 1998
[15] Abhinandan et Martin, Protein Eng Des Sel 23 : 689-697, 2010
[16] Van der Spoel et al., J. Comput. Chem., 26 : 1701-1718, 2005
[17] Scott et al., Drugs 58, 305-311, 1999
[18] Steelman SL, Pohley FM. Endocrinology, 53 :604-616, 1953
[19] Scobey et al. Reprod. Biol. Endocr. 3 :61, 2005
[20] Cattanah et al., Nature, 269 (5626) : 338-340, 1977
[21] Ward et al., Nature, 341 : 544-546, 1989
[22] Li et al., Afr. J. Biotechnol., 9(50) : 8549-8554, 2010

## Revendications

1. Anticorps variant humanisé ou fragment de celui-ci potentialisant la bioactivité de l'hormone folliculo-stimulante (FSH), de l'hormone lutéo-stimulante (LH) et de la Chorio-Gonadotropine humaine (hCG), **caractérisé en ce que** :
le domaine variable de la chaine lourde dudit anticorps variant humanisé ou fragment de celui-ci contient la séquence en acides animés QX²QLVQSGAEVKKPGASVKVSCKX²⁴SGFTFSSSYIX³⁵WX³⁷RQAPGQRLEWX⁴⁸X⁴⁹ WIYAGTGGTSYX⁶¹QKFX⁶⁵GX⁶⁷X⁶⁸X⁶⁹X⁷⁰TX⁷²DTSASTAYMEX⁸³SSLRSEDTAVYYC ARHGSYFDYWGQGTLVTVSS où X² est V ou G, X²⁴ est A ou T, X³⁵ est H ou S, X³⁷ est V ou L, X⁴⁸ est I ou M, X⁴⁹ est A ou G, X⁶¹ est S ou N, X⁶⁵ est T ou Q, X⁶⁷ est R ou K, X⁶⁸ est A ou V, X⁶⁹ est T ou Q, X⁷⁰ est I ou L, X⁷² est V ou R, X⁸³ est L ou F (SEQ ID NO : 1), ou la séquence en acides aminés EVQLVESGGGLVQPGGSLRLSCAX²⁴SGFTFSSSYISWLRQAPGKGLEWX⁴⁸AWIYA GTGGTSYAQX⁶³VKGRFX⁶⁹X⁷⁰SVDTSKNTAYLQMNSLRAEDTAVYYCARHGSYFDY WGQGTLVTVSS où X²⁴ est A ou T, X⁴⁸ est I ou V, X⁶³ est S ou K, X⁶⁹ est Q ou T, X⁷⁰ est L ou I (SEQ ID NO: 2), ou la séquence en acides aminés QMQLVQSGPEVKKPGTSVKVSCKX²⁴SGFTFSSSYISWLRQARGQRLEWIAWIYAG TGGTSYAQKFQERVX⁶⁹X⁷⁰TVDMSTSTAYMEFSSLRSEDTAVYYCARHGSYFDYW GQGTLVTVSS où X²⁴ est T ou A, X⁶⁹ est Q ou T, X⁷⁰ est L ou I (SEQ ID NO : 3) ;
et
le domaine variable de la chaine légère dudit anticorps variant humanisé ou fragment de celui-ci contient la séquence en acides aminés DIX³X⁴TQSPX⁹SLX¹²X¹³SX¹⁵GX¹⁷RX¹⁹TIX²²CX²⁴X²⁵SQSVDYDGDSYMX³⁸WYQQKP GX⁴⁶X⁴⁷PKLLIYAASX⁵⁷X⁵⁸ESGVPX⁶⁴RFSGSGSGTDFTLTISSLQX⁸⁴EDX⁸⁷AX⁸⁹YYC QQSNEDPYTFGQGTKLEIK où X³ est Q ou V, X⁴ est M ou L, X⁹ est D ou S, X¹² est A ou S, X¹³ est A ou V, X¹⁵ est L ou V, X¹⁷ est E ou D, X¹⁹ est A ou V, X²² est N ou T, X²⁴ est R ou K, X²⁵ est A ou S, X³⁸ est A ou N, X⁴⁶ est Q ou K, X⁴⁷ est P ou A, X⁵⁷ est N ou S, X⁵⁸ est R ou L, X⁶⁴ est D ou S, X⁸⁴ est A ou P, X⁸⁷ est V ou F, X⁸⁹ est V ou T (SEQ ID NO : 4).

2. Anticorps variant humanisé ou fragment de celui-ci selon la revendication 1, **caractérisé en ce que** :
le domaine variable de la chaine lourde dudit anticorps variant humanisé ou fragment de celui-ci contient la séquence en acides aminés QVQLVQSGAEVKKPGASVKVSCKASGFTFSSSYIHVWRQAPGQRLEWMGWIYAG TGGTSYSQKFQGRVTITRDTSASTAYMELSSLRSEDTAVYYCARHGSYFDYWGQG TLVTVSS (SEQ ID NO: 5), ou la séquence en acides aminés QVQLVQSGAEVKKPGASVKVSCKASGFTFSSSYISWVRQAPGQRLEWMAWIYAGT GGTSYNQKFTGKVTITRDTSASTAYMELSSLRSEDTAVYYCARHGSYFDYWGQGT LVTVSS (SEQ ID NO : 6), ou la séquence en acides aminés QGQLVQSGAEVKKPGASVKVSCKASGFTFSSSYISWLRQAPGQRLEWIAWIYAGT GGTSYNQKFTGKVTITRDTSASTAYMELSSLRSEDTAVYYCARHGSYFDYWGQGT LVTVSS (SEQ ID NO : 7), ou la séquence en acides aminés QGQLVQSGAEVKKPGASVKVSCKASGFTFSSSYISWLRQAPGQRLEWIAWIYAGT GGTSYNQKFTGKATLTVDTSASTAYMELSSLRSEDTAVYYCARHGSYFDYWGQGT LVTVSS (SEQ ID NO : 8), ou la séquence en acides aminés QVQLVQSGAEVKKPGASVKVSCKTSGFTFSSSYISWLRQAPGQRLEWIAWIYAGTG GTSYSQKFQGRVQLTVDTSASTAYMEFSSLRSEDTAVYYCARHGSYFDYWGQGTL VTVSS (SEQ ID NO : 9), ou la séquence en acides aminés QVQLVQSGAEVKKPGASVKVSCKASGFTFSSSYISWLRQAPGQRLEWMAWIYAGT GGTSYSQKFQGRVTITVDTSASTAYMEFSSLRSEDTAVYYCARHGSYFDYWGQGT LVTVSS (SEQ ID NO: 10), ou la séquence en acides aminés EVQLVESGGGLVQPGGSLRLSCATSGFTFSSSYISWLRQAPGKGLEWIAWIYAGTG GTSYAQKVKGRFQLSVDTSKNTAYLQMNSLRAEDTAVYYCARHGSYFDYWGQGT LVTVSS (SEQ ID NO: 11), ou la séquence en acides aminés EVQLVESGGGLVQPGGSLRLSCAASGFTFSSSYISWLRQAPGKGLEWVAWIYAGT GGTSYAQSVKGRFTISVDTSKNTAYLQMNSLRAEDTAVYYCARHGSYFDYWGQGT LVTVSS (SEQ ID NO: 12), ou la séquence en acides aminés QMQLVQSGPEVKKPGTSVKVSCKTSGFTFSSSYISWLRQARGQRLEWIAWIYAGT GGTSYAQKFQERVQLTVDMSTSTAYMEFSSLRSEDTAVYYCARHGSYFDYWGQG TLVTVSS (SEQ ID NO: 13), ou la séquence en acides aminés QMQLVQSGPEVKKPGTSVKVSCKASGFTFSSSYISWLRQARGQRLEWIAWIYAGT GGTSYAQKFQERVTITVDMSTSTAYMEFSSLRSEDTAVYYCARHGSYFDYWGQGT LVTVSS (SEQ ID NO : 14) ;
et
le domaine variable de la chaine légère dudit anticorps variant humanisé ou fragment de celui-ci contient la séquence en acides aminés DIVMTQSPDSLAVSLGERATINCKSSQSVDYDGDSYMAWYQQKPGQPPKLLIYAAS NRESGVPDRFSGSGSGTDFTLTISSLQAEDVAVYYCQQSNEDPYTFGQGTKLEIK (SEQ ID NO: 15), ou la séquence en acides aminés DIQLTQSPSSLSASVGDRVTITCRASQSVDYDGDSYMNWYQQKPGKAPKLLIYAAS SLESGVPSRFSGSGSGTDFTLTISSLQPEDFATYYCQQSNEDPYTFGQGTKLEIK (SEQ ID NO : 16).

3. Anticorps variant humanisé ou fragment de celui-ci selon la revendication 2, **caractérisé en ce que** :
le domaine variable de la chaine lourde dudit anticorps variant humanisé ou fragment de celui-ci contient la séquence SEQ ID NO : 5, ou SEQ ID NO : 6, ou SEQ ID NO : 7 ou SEQ ID NO : 8, et le domaine variable de la chaine légère dudit anticorps variant humanisé ou fragment de celui-ci contient la séquence SEQ ID NO : 15 ;
ou
le domaine variable de la chaine lourde dudit anticorps variant humanisé ou fragment de celui-ci contient la séquence SEQ ID NO : 9, ou SEQ ID NO : 10, et le domaine variable de la chaine légère dudit anticorps variant humanisé ou fragment de celui-ci contient la séquence SEQ ID NO : 16 ;
ou
le domaine variable de la chaine lourde dudit anticorps variant humanisé ou fragment de celui-ci contient la séquence SEQ ID NO : 11 ou SEQ ID NO : 12, et le domaine variable de la chaine légère dudit anticorps variant humanisé ou fragment de celui-ci contient la séquence SEQ ID NO : 16 ;
ou
le domaine variable de la chaine lourde dudit anticorps variant humanisé ou fragment de celui-ci contient la séquence SEQ ID NO : 13 ou SEQ ID NO : 14, et le domaine variable de la chaine légère dudit anticorps variant humanisé ou fragment de celui-ci contient la séquence SEQ ID NO : 16.

4. Anticorps variant humanisé ou fragment de celui-ci selon la revendication 3, **caractérisé en ce qu'**il comprend le domaine variable de la chaine lourde et le domaine variable de la chaine légère de séquences respectives suivantes : SEQ ID NO : 9 et SEQ ID NO : 16 ; SEQ ID NO : 10 et SEQ ID NO : 16 ; SEQ ID NO : 11 et SEQ ID NO : 16 ; SEQ ID NO : 12 et SEQ ID NO : 16 ; SEQ ID NO : 5 et SEQ ID NO : 15 ; SEQ ID NO : 6 et SEQ ID NO : 15 ; SEQ ID NO : 7 et SEQ ID NO : 15 ; SEQ ID NO : 8 et SEQ ID NO : 15 ; SEQ ID NO : 13 et SEQ ID NO : 16 ; SEQ ID NO : 14 et SEQ ID NO : 16.

5. Anticorps variant humanisé ou fragment de celui-ci selon la revendication 4, **caractérisé en ce qu'**il comprend le domaine variable de la chaine lourde et le domaine variable de la chaine légère de séquences respectives suivantes : SEQ ID NO : 6 et SEQ ID NO : 15 ; SEQ ID NO : 8 et SEQ ID NO : 15 ; SEQ ID NO : 12 et SEQ ID NO : 16 ; SEQ ID NO : 9 et SEQ ID NO : 16 ; SEQ ID NO : 11 et SEQ ID NO : 16 ; SEQ ID NO : 14 et SEQ ID NO : 16.

6. Anticorps variant humanisé ou fragment de celui-ci selon la revendication 5, **caractérisé en ce qu'**il comprend le domaine variable de la chaine lourde contenant la séquence SEQ ID NO : 8 ou SEQ ID NO : 9, et le domaine variable de la chaine légère contenant la séquence SEQ ID NO : 15 ou SEQ ID NO : 16, respectivement.

7. Anticorps variant humanisé ou fragment de celui-ci selon la revendication 6, **caractérisé en ce qu'**il comprend le domaine variable de la chaine lourde contenant la séquence SEQ ID NO : 9, et le domaine variable de la chaine légère contenant la séquence SEQ ID NO : 16.

8. Anticorps variant humanisé ou fragment de celui-ci selon la revendication 7, **caractérisé en ce qu'**il comprend la chaine lourde de séquence SEQ ID NO : 20 et la chaine légère de séquence SEQ ID NO : 21.

9. Anticorps variant humanisé ou fragment de celui-ci selon l'une quelconque des revendications 1 à 8, où le fragment est un scFv, de préférence un scFv de séquence SEQ ID NO : 17.

10. Composition pharmaceutique, **caractérisée en ce qu'**elle comprend un anticorps variant humanisé ou fragment de celui-ci selon l'une quelconque des revendications 1 à 9, et un véhicule pharmaceutiquement acceptable.

11. Composition pharmaceutique selon la revendication 10, **caractérisé en ce qu'**elle comprend en outre de la FSH, ou de la LH, ou un mélange FSH et LH, ou de la hMG (human Menopausal Gonadotropin), ou un mélange FSH et hMG, ou un mélange LH et hMG, ou de la hCG, ou un mélange hCG et FSH, ou un mélange hCG et hMG.

12. Anticorps variant humanisé ou fragment de celui-ci selon l'une quelconque des revendications 1 à 9 ou composition pharmaceutique selon l'une quelconque des revendications 10 ou 11, pour une utilisation comme médicament.

13. Anticorps variant humanisé ou fragment de celui-ci selon l'une quelconque des revendications 1 à 9 ou composition pharmaceutique selon l'une quelconque des revendications 10 ou 11, pour une utilisation selon la revendication 12, où le médicament est destiné à induire l'ovulation ou la polyovulation chez un mammifère femelle.

14. Anticorps variant humanisé ou fragment de celui-ci selon l'une quelconque des revendications 1 à 9 ou composition pharmaceutique selon l'une quelconque des revendications 10 ou 11, pour une utilisation selon la revendication 12, où le médicament est destiné à induire ou stimuler la spermatogénèse chez un mammifère mâle.

15. Anticorps variant humanisé ou fragment de celui-ci selon l'une quelconque des revendications 1 à 9 ou composition pharmaceutique selon l'une quelconque des revendications 10 ou 11, pour une utilisation selon la revendication 12, où le médicament est destiné à augmenter la stéroïdogenèse chez un mammifère mâle ou femelle.

16. Anticorps variant humanisé ou fragment de celui-ci selon l'une quelconque des revendications 1 à 9 ou composition pharmaceutique selon l'une quelconque des revendications 10 ou 11, pour une utilisation dans la prévention ou le traitement de l'infertilité ou de l'hypofertilité chez un mammifère mâle ou femelle.

17. Anticorps variant humanisé ou fragment de celui-ci selon l'une quelconque des revendications 1 à 9 ou composition pharmaceutique selon l'une quelconque des revendications 10 ou 11, pour une utilisation pour stimuler la procréation chez un mammifère femelle.
